(19) Europäisches Patentamt
European Patent Office
Office européen des brevets

(11) EP 1 964 831 A1

(12) # EUROPEAN PATENT APPLICATION
published in accordance with Art. 158(3) EPC

(43) Date of publication:
**03.09.2008 Bulletin 2008/36**

(21) Application number: **06834472.0**

(22) Date of filing: **12.12.2006**

(51) Int Cl.:
***C07C 68/06*** *(2006.01)* ***C07C 68/08*** *(2006.01)*
***C07C 69/96*** *(2006.01)* ***C08G 64/04*** *(2006.01)*

(86) International application number:
**PCT/JP2006/324717**

(87) International publication number:
**WO 2007/072705 (28.06.2007 Gazette 2007/26)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **19.12.2005 JP 2005364511**

(71) Applicant: **Asahi Kasei Chemicals Corporation**
**Tokyo 100-8440 (JP)**

(72) Inventors:
- **FUKUOKA, Shinsuke**
  **Chiyoda-ku, Tokyo 1008440 (JP)**
- **MIYAJI, Hironori**
  **Chiyoda-ku, Tokyo 1008440 (JP)**
- **HACHIYA, Hiroshi**
  **Chiyoda-ku, Tokyo 1008440 (JP)**
- **MATSUZAKI, Kazuhiko**
  **Chiyoda-ku, Tokyo 1008440 (JP)**

(74) Representative: **Strehl Schübel-Hopf & Partner**
**Maximilianstrasse 54**
**80538 München (DE)**

(54) **PROCESS FOR PRODUCING HIGH-PURITY DIPHENYL CARBONATE ON AN INDUSTRIAL SCALE**

(57) It is an object of the present invention to provide a specific process that enables a high-purity diphenyl carbonate required for producing a high-quality high-performance aromatic polycarbonate to be produced industrially in a large amount (e.g. not less than 1 ton / hr) stably for a prolonged period of time (e.g. not less than 1000 hours, preferably not less than 3000 hours, more preferably not less than 5000 hours) from a cyclic carbonate and a phenol. When producing, from a cyclic carbonate and a phenol, a high-purity diphenyl carbonate required for producing a high-quality high-performance aromatic polycarbonate, the above object can be attained by carrying out a process according to the present invention which comprises steps of: (I) producing a dialkyl carbonate and a diol using a reactive distillation column having a specified structure; (II) producing a diphenyl carbonate using two reactive distillation columns each having a specified structure, and (III) obtaining a high-purity diphenyl carbonate from the diphenyl carbonate using a high boiling point material separating column A and a diphenyl carbonate purifying column B.

**FIG.1**

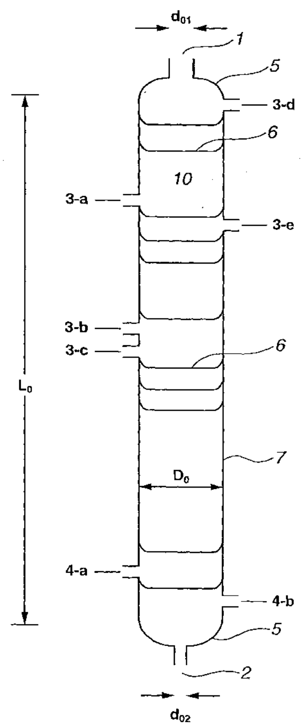


EP 1 964 831 A1

## Description

### Technical Field

The present invention relates to an industrial process for the production of a high-purity diphenyl carbonate. More particularly, the present invention relates to a process for producing, from a cyclic carbonate and a phenol, stably for a prolonged period of time on an industrial scale, a high-purity diphenyl carbonate required for producing a high-quality high-performance aromatic polycarbonate.

### Background Art

In order to produce an aromatic polycarbonate on an industrial scale through transesterification, a high-purity diphenyl carbonate must be procured in a large amount on an industrial scale. Aromatic dihydroxy compounds such as high-purity bisphenol A are mass-produced industrially, and can easily be procured, but a high-purity diphenyl carbonate cannot be procured in a large amount on an industrial scale. It is thus necessary to produce the high-purity diphenyl carbonate.

As a process for producing the diphenyl carbonate, a process of reacting a phenol with phosgene has been known from long ago, and has also been the subject of a variety of studies in recent years. However, this process has the problem of using phosgene, and in addition chlorinated impurities that are difficult to separate out are present in the diphenyl carbonate produced using this process, and hence the diphenyl carbonate cannot be used as a starting material for the production of an aromatic polycarbonate as is. The reason for this is that such chlorinated impurities markedly inhibit the polymerization reaction in the transesterification method for producing the aromatic polycarbonate which is carried out in the presence of an extremely small amount of a basic catalyst; for example, even if such chlorinated impurities are present in an amount of only 1 ppm, the polymerization hardly proceeds at all. To make the diphenyl carbonate capable of being used as the starting material of a transesterification method aromatic polycarbonate, a troublesome multi-stage separation / purification process involving thorough washing with a dilute aqueous alkaline solution and hot water, oil / water separation, distillation and so on is thus required. Furthermore, the yield decreases due to hydrolysis loss and distillation loss during this separation / purification process. There are thus many problems in carrying out this process economically on an industrial scale.

On the other hand, a process for producing aromatic carbonates through transesterification reactions between a dialkyl carbonate and an aromatic monohydroxy compound is also known. However, such transesterification reactions are all equilibrium reactions. The equilibrium is biased extremely toward the original system and the reaction rate is slow, and hence there have been many difficulties in producing aromatic carbonates industrially in large amounts using such a process.

Several proposals have been made to improve on the above difficulties, but most of these have related to development of a catalyst to increase the reaction rate. Many metal compounds have been proposed as catalysts for this type of transesterification reaction. However, the problem of the disadvantageous equilibrium cannot be resolved merely by developing a catalyst, and hence there are very many issues to be resolved including the reaction system in order to provide a process for industrial production aiming for mass production.

Attempts have also been made to devise a reaction system so as to shift the equilibrium toward the product system as much as possible, and thus improve the aromatic carbonate yield. For example, for the reaction between dimethyl carbonate and phenol, there have been proposed a method in which methanol produced as a by-product is distilled off by azeotropy together with an azeotrope-forming agent (see Patent Document 1: Japanese Patent Application Laid-Open No. 54-48732 (corresponding to West German Patent Application Laid-Open No. 736063, and U.S. Patent No. 4252737)), and a method in which the methanol produced as a by-product is removed by being adsorbed onto a molecular sieve (see Patent Document 2: Japanese Patent Application Laid-Open No. 58-185536 (corresponding to U.S. Patent No. 410464)). Moreover, a method has also been proposed in which, using an apparatus in which a distillation column is provided on top of a reactor, an alcohol produced as a by-product in the reaction is separated off from the reaction mixture, and at the same time unreacted starting material that evaporates is separated off by distillation (see Patent Document 3: Japanese Patent Application Laid-Open No. 56-123948 (corresponding to U.S. Patent No. 4182726)).

However, these reaction systems are basically batch system or switchover system. This is because there is a limitation on how much the reaction rate can be improved through catalyst development for such a transesterification reaction, and hence the reaction rate is still slow, and thus it has been thought that a batch system is preferable to a continuous system. Of these, a continuous stirred tank reactor (CSTR) system in which a distillation column is provided on top of a reactor has been proposed as a continuous system, but there are problems such as the reaction rate being slow, and the gas-liquid interface in the reactor being small based on the volume of the liquid. It is thus not possible to make the conversion high. Accordingly, it is difficult to attain the object of producing an aromatic carbonate continuously

in large amounts stably for a prolonged period of time by means of the above methods, and many issues remain to be resolved before economical industrial implementation is possible.

The present inventors have developed reactive distillation methods in which such a transesterification reaction is carried out in a continuous multi-stage distillation column simultaneously with separation by distillation, and have been the first in the world to disclose that such a reactive distillation system is useful for such a transesterification reaction, for example a reactive distillation method in which a dialkyl carbonate and an aromatic hydroxy compound are continuously fed into a multi-stage distillation column, and reaction is carried out continuously inside the column in which a catalyst is present, while continuously withdrawing by distillation a low boiling point component containing an alcohol produced as a by-product, and continuously withdrawing a component containing a produced alkyl aryl carbonate from a lower portion of the column (see Patent Document 4: Japanese Patent Application Laid-Open No. 3-291257), a reactive distillation method in which an alkyl aryl carbonate is continuously fed into a multi-stage distillation column, and reaction is carried out continuously inside the column in which a catalyst is present, while continuously withdrawing by distillation a low boiling point component containing a dialkyl carbonate produced as a by-product, and continuously withdrawing a component containing a produced diaryl carbonate from a lower portion of the column (see Patent Document 5: Japanese Patent Application Laid-Open No. 4-9358), a reactive distillation method in which these reactions are carried out using two continuous multi-stage distillation columns, and hence a diaryl carbonate is produced continuously while efficiently recycling a dialkyl carbonate produced as a by-product (see Patent Document 6: Japanese Patent Application Laid-Open No. 4-211038 (corresponding to WO 91/09832, European Patent No. 0461274, and U.S. Patent No. 5210268)), and a reactive distillation method in which a dialkyl carbonate and an aromatic hydroxy compound or the like are continuously fed into a multi-stage distillation column, and a liquid that flows down through the column is withdrawn from a side outlet provided at an intermediate stage and / or a lowermost stage of the distillation column, and is introduced into a reactor provided outside the distillation column so as to bring about reaction, and is then introduced back in through a circulating inlet provided at a stage above the stage where the outlet is provided, whereby reaction is carried out in both the reactor and the distillation column (see Patent Document 7: Japanese Patent Application Laid-Open No. 4-235951).

These reactive distillation methods proposed by the present inventors are the first to enable aromatic carbonates to be produced continuously and efficiently, and processes in which a diaryl carbonate is produced from a dialkyl carbonate using two continuous multi-stage distillation columns based on the above disclosures have been proposed thereafter (see, for example, Patent Document 8: Japanese Patent Application Laid-Open No. 6-157424 (corresponding to European Patent No. 0582931, and U.S. Patent No. 5334742), Patent Document 9: Japanese Patent Application Laid-Open No. 6-184058 (corresponding to European Patent No. 0582930, and U.S. Patent No. 5344954), Patent Document 10: Japanese Patent Application Laid-Open No. 9-40616, Patent Document 11: Japanese Patent Application Laid-Open No. 9-59225, Patent Document 12: Japanese Patent Application Laid-Open No. 9-176094, Patent Document 13: WO 00118720 (corresponding to U.S. Patent No. 6093842), Patent Document 14: Japanese Patent Application Laid-Open No. 2001-64235).

Among reactive distillation systems, the present applicants have further proposed, as a method that enables highly pure aromatic carbonates to be produced stably for a prolonged period of time without a large amount of a catalyst being required, a method in which high boiling point material containing a catalyst component is reacted with an active substance and then separated off, and the catalyst component is recycled (see Patent Document 15: WO 97/11049 (corresponding to European Patent No. 0855384, and U.S. Patent No. 5872275)), and a method carried out while keeping the weight ratio of a polyhydric aromatic hydroxy compound in the reaction system to a catalyst metal at not more than 2.0 (see Patent Document 16: Japanese Patent Application Laid-Open No. 11-92429 (corresponding to European Patent No. 1016648, and U.S. Patent No. 6262210)). Furthermore, the present inventors have proposed a method in which 70 to 99 % by weight of phenol produced as a by-product in a polymerization process is used as a starting material, and diphenyl carbonate is produced using a reactive distillation method; this diphenyl carbonate can be used as a starting material for polymerization to produce an aromatic polycarbonate (see Patent Document 17: Japanese Patent Application Laid-Open No. 9-255772 (corresponding to European Patent No. 0892001, and U. S. Patent No. 5747609)).

However, in all of these prior art documents in which the production of aromatic carbonates using a reactive distillation method is proposed, there is no disclosure whatsoever of a specific process or apparatus enabling mass production on an industrial scale (e.g. 1 ton / hr), nor is there any description suggesting such a process or apparatus. For example, the descriptions regarding heights ($H_1$ and $H_2$: cm), diameters ($D_1$ and $D_2$: cm), numbers of stages ($n_1$ and $n_2$), and starting material feeding rates ($Q_1$ and $Q_2$: kg / hr) for two reactive distillation columns disclosed for producing mainly diphenyl carbonate (DPC) from dimethyl carbonate and phenol are as summarized in Table 1.

Table 1

TABLE 1

| $H_1$ | $D_1$ | $n_1$ | $Q_1$ | $H_2$ | $D_2$ | $n_2$ | $Q_2$ | PATENT DOCUMENTS |
|---|---|---|---|---|---|---|---|---|
| 600 | 25 | 20 | 66 | 600 | 25 | 20 | 23 | 6 |
| 350 | 2.8 | - | 0.2 | 305 | 5~10 | 15+ PACKINGS | 0.6 | 9 |
| 500 | 5 | 50 | 0.6 | 400 | 8 | 50 | 0.6 | 10 |
| 100 | 4 | - | 1.4 | 200 | 4 | - | 0.8 | 11 |
| 300 | 5 | 40 | 1.5 | - | 5 | 25 | 0.7 | 12 |
| 1200 | 20 | 40 | 86 | 600 | 25 | 20 | 31 | 15<br>16 |
| 600 | - | 20 | 66 | 600 | - | 20 | 22 | 17 |

In other words, the biggest pairs of continuous multi-stage distillation columns used when carrying out this reaction using a reactive distillation system are those disclosed by the present applicants in Patent Document 15 (WO 97/11049 (corresponding to European Patent No. 0855384, and U.S. Patent No. 5872275)) and Patent Document 16 (Japanese Patent Application Laid-Open No. 11-92429 (corresponding to European Patent No. 1016648, and U.S. Patent No. 6262210)). As can be seen from Table 1, the maximum values of the various conditions for the continuous multi-stage distillation columns disclosed for the above reaction are $H_1$ = 1200 cm, $H_2$ = 600 cm, $D_1$ = 20 cm, $D_2$ = 25 cm, $n_1 = n_2$ = 50 (the only conditions: Patent Document 10 (Japanese Patent Application Laid-Open No. 9-40616)), $Q_1$ = 86 kg / hr, and $Q_2$ = 31 kg / hr, and the amount of diphenyl carbonate produced has not exceeded approximately 6.7 kg / hr, which is not an amount produced on an industrial scale.

The dialkyl carbonate used in a step (II) of the present invention must be produced on an industrial scale, and furthermore must not contain a halogen. The only process in which such a dialkyl carbonate is industrially produced in a large amount as a starting material for an aromatic polycarbonate is an oxidative carbonylation process in which methanol is reacted with carbon monoxide and oxygen to produce dimethyl carbonate and water. However, with this oxidative carbonylation process (see, for example, Patent Document 18: WO 03/016257), the reaction must be carried out in a slurry state using a large amount of CuCl-HCl as a catalyst, and hence there is a problem of the corrosivity being very high in the reaction system and a separation/purification system. Moreover, in this process, the carbon monoxide is prone to being oxidized into carbon dioxide, and hence there is a problem that the selectivity based on the carbon monoxide is low at approximately 80%.

On the other hand, several processes for the production of a dialkyl carbonate and a diol from a reaction between a cyclic carbonate and an aliphatic monohydric alcohol have been proposed. With this reaction, a dialkyl carbonate can be produced without using a halogen, and hence this is a preferable process. As reaction systems, four systems have been proposed. These four reaction systems are used in a process for the production of dimethyl carbonate and ethylene glycol from ethylene carbonate and methanol, which is the most typical reaction example, and are (1) a completely batch reaction system, (2) a batch reaction system using a reaction vessel having a distillation column provided on top thereof, (3) a flowing liquid reaction system using a tubular reactor, and (4) a reactive distillation system first disclosed by the present inventors (see, for example, Patent Document 19: Japanese Patent Application Laid-Open No. 4-198141, Patent Document 20: Japanese Patent Application Laid-Open No. 9-194435, Patent Document 21: WO99/64382 (corresponding to European Patent No. 1086940, and U.S. Patent No. 6346638), Patent Document 22: WO00/51954 (corresponding to European Patent No. 1174406, and U.S. Patent No. 6479689), Patent Document 23: Japanese Patent Application Laid-Open No. 5-213830 (corresponding to European Patent No. 0530615, and U.S. Patent No. 5231212), Patent Document 24: Japanese Patent Application Laid-Open No. 6-9507 (corresponding to European Patent No. 0569812, and U.S. Patent No. 5359118), Patent Document 25: Japanese Patent Application Laid-Open No. 2003-119168 (corresponding to WO03/006418), Patent Document 26: Japanese Patent Application Laid-Open No. 2003-300936, Patent Document 27: Japanese Patent Application Laid-Open No. 2003-342209). However, there have been problems with these systems as follows.

In the case of (1) and (3), the upper limit of the cyclic carbonate conversion is determined by the composition put in and the temperature, and hence the reaction cannot be carried out to completion, and thus the conversion is low. Moreover, in the case of (2), to make the cyclic carbonate conversion high, the produced dialkyl carbonate must be distilled off using a very large amount of the aliphatic monohydric alcohol, and a long reaction time is required. In the case of (4), the reaction can be made to proceed with a higher conversion than with (1), (2) or (3). However, processes of (4) proposed hitherto have related to producing the dialkyl carbonate and the diol either in small amounts or for a short period of time, and have not related to carrying out the production on an industrial scale stably for a prolonged

period of time. That is, these processes have not attained the object of producing a dialkyl carbonate continuously in a large amount (e.g. not less than 2 ton / hr) stably for a prolonged period of time (e.g. not less than 1000 hours, preferably not less than 3000 hours, more preferably not less than 5000 hours). For example, the maximum values of the height (H: cm), diameter (D: cm), and number of stages (n) of the reactive distillation column, the amount produced P (kg/hr) of dimethyl carbonate, and the continuous production time T (hr) in examples disclosed for the production of dimethyl carbonate (DMC) and ethylene glycol (EG) from ethylene carbonate and methanol are as in Table 2.

Table 2

**TABLE 2**

| PATENT DOCUMENT | H : cm | D : cm | NO. STAGES : n | P : kg/hr | T : hr |
|---|---|---|---|---|---|
| 19 | 100 | 2 | 30 | 0.106 | 400 |
| 20 | 160 | 5 | 40 | 0.743 | NOTE 5 |
| 21 | 200 | 4 | PACKING COLUMN (Dixon) | 0.932 | NOTE 5 |
| 22 | NOTE 1 | 5 | 60 | 0.275 | NOTE 5 |
| 23 | 250 | 3 | PACKING COLUMN (Raschig) | 0.392 | NOTE 5 |
| 24 | NOTE 2 | NOTE 2 | NOTE 2 | 0.532 | NOTE 5 |
| 25 | NOTE 3 | NOTE 3 | 42 | NOTE 4 | NOTE 5 |
| 26 | NOTE 3 | NOTE 3 | 30 | 3750 | NOTE 5 |
| 27 | 200 | 15 | PACKING COLUMN (BX) | 0.313 | NOTE 5 |

NOTE 1 : OLDERSHAW DISTILLATION COLUMN.
NOTE 2 : NO DESCRIPTION WHATSOEVER DEFINING DISTILLATION COLUMN.
NOTE 3 : ONLY DESCRIPTION DEFINING DISTILLATION COLUMN IS NUMBER OF STAGES.
NOTE 4 : NO DESCRIPTION WHATSOEVER OF PRODUCED AMOUNT.
NOTE 5 : NO DESCRIPTION WHATSOEVER REGARDING STABLE PRODUCTION FOR PROLONGED PERIOD OF TIME.

In Patent Document 26 (Japanese Patent Application Laid-Open No. 2003-300936), it is stated at paragraph that "The present example uses the same process flow as for the preferred mode shown in FIG. 1 described above, and was carried out with the object of operating a commercial scale apparatus for producing dimethyl carbonate and ethylene glycol through transesterification by a catalytic conversion reaction between ethylene carbonate and methanol. Note that the following numerical values in the present example can be adequately used in the operation of an actual apparatus", and as that example it is stated that 3750 kg / hr of dimethyl carbonate was specifically produced. The scale described in that example corresponds to an annual production of over 30,000 tons, and hence this implies that operation of the world's largest scale commercial plant using this process had been carried out at the time of the filing of the patent application for Patent Document 26 (Japanese Patent Application Laid-Open No. 2003-300936) (April 9, 2002),. However, even at the time of filing the present application, there is not the above fact at all. Moreover, in the example of Patent Document 26 (Japanese Patent Application Laid-Open No. 2003-300936), exactly the same value as the theoretically calculated value is stated for the amount of dimethyl carbonate produced, but the yield for ethylene glycol is approximately 85.6%, and the selectivity is approximately 88.4%, and hence it cannot really be said that a high yield and high selectivity have been attained. In particular, the low selectivity indicates that this process has a fatal drawback as an industrial production process. Note also that Patent Document 26 (Japanese Patent Application Laid-Open No. 2003-300936) was deemed to have been withdrawn on July 26, 2005 due to examination not having been requested.

With the reactive distillation method, there are very many causes of fluctuation such as composition variation due to reaction and composition variation due to distillation in the distillation column, and temperature variation and pressure variation in the column, and hence continuing stable operation for a prolonged period of time is accompanied by many difficulties, and in particular these difficulties are further increased in the case of handling large amounts. To continue mass production of a dialkyl carbonate and a diol using the reactive distillation method stably for a prolonged period of time while maintaining high yields and high selectivities for the dialkyl carbonate and the diol, the reactive distillation apparatus must be cleverly devised. However, the only description of continuous stable production for a

prolonged period of time with the reactive distillation method proposed hitherto has been the 200 to 400 hours in Patent Document 19 (Japanese Patent Application Laid-Open No. 4-198141).

Meanwhile, as processes for separating the diphenyl carbonate from a reaction mixture containing a diphenyl carbonate that has been produced through transesterification or the like with a dialkyl carbonate and a phenol as a starting material, and purifying the diphenyl carbonate, crystallization, distillation and so on have been proposed. With regard to distillation, three methods have been proposed.

One method is a method in which the diphenyl carbonate is obtained as a column top component from a distillation column; for example, there are:

(1) a method in which the reaction mixture containing the catalyst is distilled as is in a batch process distillation column, and the diphenyl carbonate is obtained as the column top component (see example in Patent Document 1: Japanese Patent Application Laid-Open No. 54-48732 (corresponding to West German Patent Application Laid-Open No. 736063, and U.S. Patent No. 4252737), example 2 in Patent Document 28: Japanese Patent Application Laid-Open No. 6-9506 (corresponding to European Patent No. 0560159, and U.S. Patent No. 5282965));

(2) a method in which the reaction mixture containing the catalyst is subjected to flash distillation in an evaporator or the like, and thus separated into high boiling point material containing most of the catalyst and low boiling point material, and then the low boiling point material is distilled in a distillation column for starting material recovery, and catalyst-containing diphenyl carbonate is obtained as column bottom material, and then this column bottom material is distilled in a purifying column, whereby the diphenyl carbonate is obtained as the column top component (see example 1 in Patent Document 32: Japanese Patent Application Laid-Open No. 4-100824, Patent Document 33: Japanese Patent Application Laid-Open No. 9-169704); and

(3) a method in which the reaction mixture containing the catalyst is distilled in a distillation column (or evaporator), and thus separated into high boiling point material containing most of the catalyst and low boiling point material, and then the low boiling point material is subjected to continuous sequential distillation using a distillation apparatus comprising three columns, i.e. a light fraction separating column, a methyl phenyl carbonate separating column, and a diphenyl carbonate separating column, whereby diphenyl carbonate is obtained as the column top component (see Patent Document 29: Japanese Patent Application Laid-Open No. 9-110805).

Another method is a method in which the diphenyl carbonate is obtained as a column bottom component from a distillation column; for example, there is:

(4) a method in which the reaction mixture containing the catalyst is distilled in a distillation column, and thus separated into high boiling point material containing most of the catalyst and low boiling point material, and then the low boiling point material is distilled in an other distillation column, and the diphenyl carbonate is obtained as said other column bottom component (see Patent Document 14: Japanese Patent Application Laid-Open No. 2001-64235, Patent Document 30: Japanese Patent Application Laid-Open No. 2001-64234).

Still another method is a method in which the diphenyl carbonate is obtained as a side cut component from a distillation column; for example, there are:

(5) a method in which the reaction mixture containing the catalyst is introduced as is into a third reactive distillation column, and further reaction and distillation are carried out, whereby the diphenyl carbonate is obtained as a side cut component from the reactive distillation column (see Patent Document 8: Japanese Patent Application Laid-Open No. 6-157424 (corresponding to European Patent No. 0582931, and U.S. Patent No. 5334742), Patent Document 9: Japanese Patent Application Laid-Open No. 6-184058 (corresponding to European Patent No. 0582930, and U.S. Patent No. 5344954));

(6) a method in which the reaction mixture containing the catalyst is subjected to flash distillation in an evaporator or the like, and thus separated into high boiling point material containing most of the catalyst and low boiling point material, and then the low boiling point material is introduced into a distillation column and distillation is carried out, whereby the diphenyl carbonate is obtained as a side cut component from the distillation column (see Patent Document 16: Japanese Patent Application Laid-Open No. 11-92429 (corresponding to European Patent No. 1016648, and U.S. Patent No. 6262210), Patent Document 17: Japanese Patent Application Laid-Open No. 9-255772 (corresponding to European Patent No. 0892001, and U.S. Patent No. 5747609), Patent Document 34: WO 92/18458 (corresponding to U.S. Patent No. 5426207));

(7) a method in which the reaction mixture containing the catalyst is distilled in a first purifying column, and thus separated into high boiling point material containing the catalyst and low boiling point material, and then the low boiling point material is introduced into a second purifying column and distillation is carried out, whereby the diphenyl carbonate is obtained as a side cut component from the second purifying column (see Patent Document 35: Japanese Patent Application Laid-Open No. 11-49727); and

(8) a method in which diphenyl carbonate containing phenyl salicylate is introduced into a distillation column having 5 to 15 theoretical stages, and distillation is carried out at a column bottom temperature of not less than 150°C, whereby the diphenyl carbonate is obtained as a side cut component from the distillation column (see Patent Document 31: Japanese Patent Application Laid-Open No. 9-194437 (corresponding to European Patent No. 0784048)).

However, it has become clear that various problems still remain with such diphenyl carbonate separation/purification methods using distillation. Specifically:

(1) The purity of the diphenyl carbonate obtained through the process of Patent Document 1 (Japanese Patent Application Laid-Open No. 54-48732 (corresponding to West German Patent Application Laid-Open No. 736063, and U.S. Patent No. 4252737)) in the above (1) is low, and moreover this is a batch process and hence is not suitable for mass production on an industrial scale.
(2) Regarding the above (2), the process of example 1 in Patent Document 32 (Japanese Patent Application Laid-Open No. 4-100824) is a batch process, and the diphenyl carbonate obtained through the process of Patent Document 33 (Japanese Patent Application Laid-Open No. 9-169704) contains a titanium catalyst, albeit in an amount of not more than 1 ppm, and hence is not suitable as a raw material for the production of a high-purity uncolored polycarbonate.
(3) With the process of Patent Document 29 (Japanese Patent Application Laid-Open No. 9-110805) in the above (3), the diphenyl carbonate is heated to a high temperature in the bottom of each of the light fraction separating column and the methyl phenyl carbonate separating column, and is then subjected to a high temperature in the diphenyl carbonate separating column; the diphenyl carbonate is thus altered, bringing about a decrease in the purity and a decrease in the yield.
(4) The process of Patent Document 14 (Japanese Patent Application Laid-Open No. 2001-64235) and Patent Document 30 (Japanese Patent Application Laid-Open No. 2001-64234) in the above (4) in which the diphenyl carbonate is obtained from the bottom of the column is unsuitable since the purity is low and hence a desired polycarbonate cannot be produced.
(5) With the process of Patent Document 8 (Japanese Patent Application Laid-Open No. 6-157424 (corresponding to European Patent No. 0582931, and U.S. Patent No. 5334742)) and Patent Document 9 (Japanese Patent Application Laid-Open No. 6-184058 (corresponding to European Patent No. 0582930, and U.S. Patent No. 5344954)) in the above (5), a reaction mixture containing each of the catalyst, unreacted starting material and impurities obtained from the bottom of the second reactive distillation column is introduced into the third reactive distillation column from an upper portion thereof, and the diphenyl carbonate is withdrawn from the side of the third reactive distillation column; vapor or mist of the starting material, the impurities, the catalyst or the like may thus be entrained, and hence the purity of the diphenyl carbonate obtained is low.
(6) With the process of the above (6), the amount of the diphenyl carbonate produced is 6.7 kg / hr (example 3 in Patent Document 16: Japanese Patent Application Laid-Open No. 11-92429 (corresponding to European Patent No. 1016648, and U.S. Patent No. 6262210)) or 3.9 kg / hr (example 1 in Patent Document 17: Japanese Patent Application Laid-Open No. 9-255772 (corresponding to European Patent No. 0892001, and U.S. Patent No. 5747609)), which is not on an industrial scale.
(7) The process of the above (7) is a preferable process, but the amount of the diphenyl carbonate produced is 2 kg / hr (example 8 in Patent Document 35: Japanese Patent Application Laid-Open No. 11-49727), which is a small amount not on an industrial scale. Moreover, the process is carried out with the column top pressure in the first purifying column at a high vacuum of 200 Pa, and hence if one tried to carry out the process industrially, then a very large distillation column enabling such a high vacuum to be maintained would be required, which would be problematic.
(8) With the process of the above (8), although it is stated that the content of phenyl salicylate is reduced from 3000 ppm to 50 ppm (example 2 in Patent Document 31: Japanese Patent Application Laid-Open No. 9-194437 (corresponding to European Patent No. 0784048)), nothing is mentioned whatsoever for other impurities. For example, even though the diphenyl carbonate is produced using the phosgene method in this example, and hence this is definitely a purification process for diphenyl carbonate containing chlorinated impurities, nothing is mentioned whatsoever with regard to the chlorinated impurities (which have an adverse effect on the polymerization to produce a polycarbonate and the properties of the polycarbonate even in an extremely small amount of only a few tens of ppb). With this process, such chlorinated impurities will not be separated out sufficiently, and hence it will not be possible to use the diphenyl carbonate as a raw material for a polycarbonate. This is as described in comparative example 1 (in which the alkali column is not used) of the purification process (in which after washing twice with alkaline hot water, washing with hot water is carried out, and then the diphenyl carbonate is dehydrated through distillation and then passed through a column packed with a solid alkali, before being subjected to reduced pressure distillation in a multi-stage distillation column) of Patent Document 35 (Japanese Patent Application Laid-Open No. 11-49727), which was filed more than one year after Patent Document 31 (Japanese Patent Application Laid-Open No. 9-194437 (corresponding to European Patent No. 0784048)).

Furthermore, in Patent Document 31 (Japanese Patent Application Laid-Open No. 9-194437 (corresponding to European Patent No. 0784048)), the temperature and time at which phenol starts to be distilled off in the case that reaction is carried out with bisphenol A are given as a method of evaluating the purity of the diphenyl carbonate obtained through the distillation, but evaluation of whether the diphenyl carbonate is suitable for polymerization cannot be carried out using this test method. This is because even for diphenyl carbonate of low purity such that a polycarbonate of the required degree of polymerization cannot be produced, the initial reaction in which phenol is eliminated occurs sufficiently. Moreover, with this evaluation method, a large amount of 2.3 ppm based on the bisphenol A of NaOH is used as a catalyst, and hence even for diphenyl carbonate containing, for example, 1 ppm of chlorinated impurities, an incorrect evaluation that the diphenyl carbonate is of high purity and is suitable as a raw material for a polycarbonate would be obtained. As stated earlier, diphenyl carbonate containing 1 ppm of chlorinated impurities cannot be used as a raw material for a polycarbonate at all. In ordinary polymerization, such a large amount of an alkaline catalyst is not used, and hence this evaluation method is not suitable for evaluating the purity of diphenyl carbonate to be used for producing a polycarbonate. Moreover, in Patent Document 31 (Japanese Patent Application Laid-Open No. 9-194437 (corresponding to European Patent No. 0784048)), there is no specific description whatsoever of purification of diphenyl carbonate that has been obtained using the transesterification method. The types and contents of impurities differ between diphenyl carbonate obtained using the phosgene method and diphenyl carbonate obtained using the transesterification method, and hence it cannot be said that diphenyl carbonate of the same purity will be obtained using the same purification process. It thus cannot be said at all that diphenyl carbonate having the required purity for a raw material of a polycarbonate would be obtained through the purification process of Patent Document 31 (Japanese Patent Application Laid-Open No. 9-194437 (corresponding to European Patent No. 0784048)). Moreover, the amount of diphenyl carbonate purified in Patent Document 31 (Japanese Patent Application Laid-Open No. 9-194437 (corresponding to European Patent No. 0784048)) is stated to be 0.57 kg / hr, which is not on an industrial scale.

A reaction mixture obtained through transesterification with a dialkyl carbonate and a phenol as a starting material in the presence of a homogeneous catalyst generally contains various reaction by-products. In particular, if a diphenyl carbonate for which the amounts of high boiling point by-products having a higher boiling point than the diphenyl carbonate such as phenyl salicylate, xanthone, phenyl methoxybenzoate and 1-phenoxycarbonyl-2-phenoxycarboxy-phenylene have not been reduced down to a sufficient level is used as the raw material of a transesterification method polycarbonate, then these high boiling point by-products will cause discoloration and a deterioration in properties. It is thus preferable to reduce the amounts of such impurities as much as possible. However, such high boiling point by-products are difficult to separate out, and with processes proposed hitherto, it has not been possible to reduce the amounts of such high boiling point by-products down to a sufficient level. In particular, there has been no proposal whatsoever of a process for the production on an industrial scale of not less than 1 ton / hr of a diphenyl carbonate of the high purity required for a raw material of a high-quality high-performance polycarbonate.

## Disclosure of Invention

### Problems to be Solved by the Invention

It is an object of the present invention to provide a specific process that enables a high-purity diphenyl carbonate required for producing a high-quality and high-performance aromatic polycarbonate to be produced industrially in a large amount (e.g. not less than 1 ton / hr) stably for a prolonged period of time (e.g. not less than 1000 hours, preferably not less than 3000 hours, more preferably not less than 5000 hours) from a cyclic carbonate and a phenol.

### Means for Solving the Problems

The present inventors have carried out studies aimed at discovering a specific process enabling the above object to be attained, and as a result have reached to the present invention.

That is, according to the first aspect of the present invention, there are provides:

1. a process for producing a high-purity diphenyl carbonate on an industrial scale in which the high-purity diphenyl carbonate is continuously produced from a cyclic carbonate and a phenol, the process comprising the steps of:

   (I) continuously producing a dialkyl carbonate and a diol through a reactive distillation system of continuously feeding the cyclic carbonate and an aliphatic monohydric alcohol into a continuous multi-stage distillation column To in which a catalyst is present, carrying out reaction and distillation simultaneously in said column, continuously withdrawing a low boiling point reaction mixture containing the produced dialkyl carbonate from an upper portion of the column in a gaseous form, and continuously withdrawing a high boiling point reaction mixture containing the diol from a lower portion of the column in a liquid form;

(II) continuously producing a diphenyl carbonate by taking the dialkyl carbonate and a phenol as a starting material, continuously feeding the starting material into a first continuous multi-stage distillation column in which a homogeneous catalyst is present, carrying out reaction and distillation simultaneously in said first column, continuously withdrawing a first column low boiling point reaction mixture containing a produced alcohol from an upper portion of said first column in a gaseous form, continuously withdrawing a first column high boiling point reaction mixture containing a produced alkyl phenyl carbonate from a lower portion of said first column in a liquid form, continuously feeding the first column high boiling point reaction mixture into a second continuous multi-stage distillation column in which a catalyst is present, carrying out reaction and distillation simultaneously in said second column, continuously withdrawing a second column low boiling point reaction mixture containing a produced dialkyl carbonate from an upper portion of said second column in a gaseous form; continuously withdrawing a second column high boiling point reaction mixture containing the produced diphenyl carbonate from a lower portion of said second column in a liquid form, and continuously feeding the second column low boiling point reaction mixture containing the dialkyl carbonate into the first continuous multi-stage distillation column; and
(III) purifying said diphenyl carbonate by continuously introducing the second column high boiling point reaction mixture containing said diphenyl carbonate into a high boiling point material separating column A, and continuously carrying out separation by distillation into a column top component $A_T$ containing the diphenyl carbonate and a column bottom component $A_B$ containing the catalyst, and then continuously introducing the column top component $A_T$ into a diphenyl carbonate purifying column B having a side cut outlet, and continuously carrying out separation by distillation into three components being a column top component $B_T$, a side cut component $B_S$ and a column bottom component $B_B$, so as to obtain the high-purity diphenyl carbonate as the side cut component;
wherein:

(a) said continuous multi-stage distillation column To comprises a structure having a cylindrical trunk portion having a length $L_0$ (cm) and an inside diameter $D_0$ (cm) and having an internal with a number of stages no thereinside, and further having a gas outlet having an inside diameter $d_{01}$ (cm) at a top of the column or in an upper portion of the column near to the top, a liquid outlet having an inside diameter $d_{02}$ (cm) at a bottom of the column or in a lower portion of the column near to the bottom, at least one first inlet provided in the upper portion and / or a middle portion of the column below the gas outlet, and at least one second inlet provided in the middle portion and / or the lower portion of the column above the liquid outlet, wherein $L_0$, $D_0$, $L_0 / D_0$, $n_0$, $D_0 / d_{01}$, and $D_0 / d_{02}$ respectively satisfy the following formulae (1) to (6);

$$2100 \leq L_0 \leq 8000 \quad (1),$$

$$180 \leq D_0 \leq 2000 \quad (2),$$

$$4 \leq L_0 / D_0 \leq 40 \quad (3),$$

$$10 \leq n_0 \leq 120 \quad (4),$$

$$3 \leq D_0 / d_{01} \leq 20 \quad (5),$$

and

$$5 \leq D_0 / d_{02} \leq 30 \quad (6);$$

(b) said first continuous multi-stage distillation column comprises a structure having a cylindrical trunk portion having a length $L_1$ (cm) and an inside diameter $D_1$ (cm), and having and internal with a number of stages $n_1$ thereinside, and further having a gas outlet having an inside diameter $d_{11}$ (cm) at a top of the column or in an upper portion of the column near to the top, a liquid outlet having an inside diameter $d_{12}$ (cm) at a bottom of the column or in a lower portion of the column near to the bottom, at least one third inlet provided in the upper portion and / or a middle portion of the column below the gas outlet, and at least one fourth inlet provided in the middle portion and / or the lower portion of the column above the liquid outlet, wherein $L_1$, $D_1$, $L_1$ / $D_1$, $n_1$, $D_1$ / $d_{11}$, and $D_1$ / $d_{12}$ respectively satisfy the following formulae (7) to (12);

$$1500 \leq L_1 \leq 8000 \quad (7),$$

$$100 \leq D_1 \leq 2000 \quad (8),$$

$$2 \leq L_1 / D_1 \leq 40 \quad (9),$$

$$20 \leq n_1 \leq 120 \quad (10),$$

$$5 \leq D_1 / d_{11} \leq 30 \quad (11),$$

and

$$3 \leq D_1 / d_{12} \leq 20 \quad (12);$$

(c) said second continuous multi-stage distillation column comprises a structure having a cylindrical trunk portion having a length $L_2$ (cm) and an inside diameter $D_2$ (cm), and having an internal with a number of stages $n_2$ thereinside, and further having a gas outlet having an inside diameter $d_{21}$ (cm) at a top of the column or in an upper portion of the column near to the top, a liquid outlet having an inside diameter $d_{22}$ (cm) at a bottom of the column or in a lower portion of the column near to the bottom, at least one fifth inlet provided in the upper portion and / or a middle portion of the column below the gas outlet, and at least one sixth inlet provided in the middle portion and / or the lower portion of the column above the liquid outlet, wherein $L_2$, $D_2$, $L_2$ / $D_2$, $n_2$, $D_2$ / $d_{21}$, and $D_2$ / $d_{22}$ respectively satisfy the following formulae (13) to (18);

$$1500 \leq L_2 \leq 8000 \quad (13),$$

$$100 \leq D_2 \leq 2000 \quad (14),$$

$$2 \leq L_2 / D_2 \leq 40 \quad (15),$$

$$10 \leq n_2 \leq 80 \quad (16),$$

$$2 \leq D_2 / d_{21} \leq 15 \quad (17),$$

and

$$5 \leq D_2 / d_{22} \leq 30 \quad (18);$$

(d) said high boiling point material separating column A comprises a continuous multi-stage distillation column having a length $L_A$ (cm) and an inside diameter $D_A$ (cm), and having an internal with a number of stages $n_A$ thereinside, wherein $L_A$, $D_A$, and $n_A$ satisfy following the following formulae (19) to (21);

$$800 \leq L_A \leq 3000 \quad (19),$$

$$100 \leq D_A \leq 1000 \quad (20),$$

and

$$20 \leq n_A \leq 100 \quad (21);$$

(e) said diphenyl carbonate purifying column B comprises a continuous multi-stage distillation column having a length $L_B$ (cm) and an inside diameter $D_B$ (cm), having an internals thereinside, having an inlet B1 at a middle portion of the column, and a side cut outlet B2 between the inlet B1 and the column bottom, and having a number of stages $n_{B1}$ of the internal above the inlet B1, a number of stages $n_{B2}$ of the internal between the inlet B1 and the side cut outlet B2, a number of stages $n_{B3}$ of the internals below the side cut outlet B2, and a total number of stages $n_B$ (= $n_{B1}$ + $n_{B2}$ + $n_{B3}$), wherein $L_B$, $D_B$, $n_{B1}$, $n_{B2}$, $n_{B3}$, and $n_B$ satisfy following formulae (22) to (27);

$$1000 \leq L_B \leq 5000 \quad (22),$$

$$100 \leq D_B \leq 1000 \quad (23),$$

$$5 \leq n_{B1} \leq 20 \quad (24),$$

$$12 \leq n_{B2} \leq 40 \quad (25),$$

$$3 \leq n_{B3} \leq 15 \qquad (26),$$

and

$$20 \leq n_B \leq 70 \qquad (27),$$

2. the process according to item 1, wherein not less than 1 ton / hr of the high-purity diphenyl carbonate is produced,
3. the process according to item 1 or 2, wherein said $d_{01}$ and said $d_{02}$ for said continuous multi-stage distillation column To used in step (I) satisfy the formula (28);

$$1 \leq d_{01} / d_{02} \leq 5 \qquad (28),$$

4. the process according to any one of items 1 to 3, wherein $L_0$, $D_0$, $L_0$ / $D_0$, $n_0$, $D_0$ / $d_{01}$, and $D_0$ / $d_{02}$ for said continuous multi-stage distillation column To satisfy respectively $2300 \leq L_0 \leq 6000$, $200 \leq D_0 \leq 1000$, $5 \leq L_0 / D_0 \leq 30$, $30 \leq n_0 \leq 100$, $4 \leq D_0 / d_{01} \leq 15$, and $7 \leq D_0 / d_{02} \leq 25$,
5. the process according to any one of items 1 to 4, wherein $L_0$, $D_0$, $L_0$ / $D_0$, $n_0$, $D_0$ / $d_{01}$, and $D_0$ / $d_{02}$ for said continuous multi-stage distillation column To satisfy respectively $2500 \leq L_0 \leq 5000$, $210 \leq D_0 \leq 800$, $7 \leq L_0 / D_0 \leq 20$, $40 \leq n_0 \leq 90$, $5 \leq D_0 / d_{01} \leq 13$, and $9 \leq D_0 / d_{02} \leq 20$,
6. the process according to any one of items 1 to 5, wherein said continuous multi-stage distillation column $T_0$ is a distillation column having a tray and / or a packing as said internal,
7. the process according to item 6, wherein said continuous multi-stage distillation column $T_0$ is a plate type distillation column having the tray as said internal,
8. the process according to item 6 or 7, wherein said tray in said continuous multi-stage distillation column $T_0$ is a sieve tray having a sieve portion and a downcomer portion,
9. the process according to item 8, wherein said sieve tray in said continuous multi-stage distillation column To has 100 to 1000 holes / $m^2$ in said sieve portion thereof,
10. the process according to item 8 or 9, wherein a cross-sectional area per hole of said sieve tray in said continuous multi-stage distillation column $T_0$ is in a range of from 0.5 to 5 $cm^2$,
11. the process according to any one of items 8 to 10, wherein an aperture ratio of said sieve tray in said continuous multi-stage distillation column $T_0$ is in a range of from 1.5 to 15%,
12. the process according to any one of items 1 to 11, wherein said $d_{11}$ and said $d_{12}$ for said first continuous multi-stage distillation column used in step (II) satisfy the following formula (29), and said $d_{21}$ and said $d_{22}$ for said second continuous multi-stage distillation column used in step (II) satisfy the following formula (30);

$$1 \leq d_{12} / d_{11} \leq 5 \qquad (29),$$

and

$$1 \leq d_{21} / d_{22} \leq 6 \qquad (30),$$

13. the process according to any one of items 1 to 12, wherein $L_1$, $D_1$, $L_1$ I $D_1$, $n_1$, $D_1$ / $d_{11}$, and $D_1$ / $d_{12}$ for said first continuous multi-stage distillation column used in step (II) satisfy respectively $2000 \leq L_1 \leq 6000$, $150 \leq D_1 \leq 1000$, $3 \leq L_1 / D_1 \leq 30$, $30 \leq n_1 \leq 100$, $8 \leq D_1 / d_{11} \leq 25$, and $5 \leq D_1 / d_{12} \leq 18$, and $L_2$, $D_2$, $L_2$ / $D_2$, $n_2$, $D_2$ / $d_{21}$, and $D_2$ / $d_{22}$ for said second continuous multi-stage distillation column used in step (II) satisfy respectively $2000 \leq L_2 \leq 6000$, $150 \leq D_2 \leq 1000$, $3 \leq L_2 / D_2 \leq 30$, $15 \leq n_2 \leq 60$, $2.5 \leq D_2 / d_{21} \leq 12$, and $7 \leq D_2 / d_{22} \leq 25$,
14. the process according to any one of items 1 to 13, wherein $L_1$, $D_1$, $L_1$ / $D_1$, $n_1$, $D_1$ / $d_{11}$, and $D_1$ /$d_{12}$ for said first continuous multi-stage distillation column satisfy respectively $2500 \leq L_1 \leq 5000$, $200 \leq D_1 \leq 800$, $5 \leq L_1 / D_1 \leq 15$, $40 \leq n_1 \leq 90$, $10 \leq D_1 / d_{11} \leq 25$, and $7 \leq D_1 / d_{12} \leq 15$, and $L_2$, $D_2$, $L_2$ / $D_2$, $n_2$, $D_2$ / $d_{21}$, and $D_2$ / $d_{22}$ for said second

continuous multi-stage distillation column satisfy respectively $2500 \le L_2 \le 5000$, $200 \le D_2 \le 800$, $5 \le L_2 / D_2 \le 15$, $20 \le n_2 \le 50$, $3 \le D_2 / d_{21} \le 10$, and $9 \le D_2 / d_{22} \le 20$,

15. the process according to any one of items 1 to 14, wherein each of said first continuous multi-stage distillation column and said second continuous multi-stage distillation column is a distillation column having a tray and / or a packing as said internal,

16. the process according to item 15, wherein said first continuous multi-stage distillation column is a plate type distillation column having the tray as said internal, and said second continuous multi-stage distillation column is a distillation column having both the packing and the tray as said internal,

17. the process according to item 15 or 16, wherein each of said trays in said first continuous multi-stage distillation column and said second continuous multi-stage distillation column is a sieve tray having a sieve portion and a downcomer portion,

18. the process according to item 17, wherein each of said sieve trays in the first continuous multi-stage distillation column and the second continuous multi-stage distillation column has 100 to 1000 holes / $m^2$ in said sieve portion,

19. the process according to item 17 or 18, wherein the cross-sectional area per hole of each of said sieve trays in said first continuous multi-stage distillation column and said second continuous multi-stage distillation column is in a range of from 0.5 to 5 $cm^2$,

20. the process according to item 15 or 16, wherein said second continuous multi-stage distillation column is a distillation column having, as said internal, the packing in the upper portion of the column, and the tray in the lower portion of the column,

21. the process according to any one of items 15 to 20, wherein said packing of said internal in said second continuous multi-stage distillation column is one or a plurality of sets of structured packings,

22. the process according to item 21, wherein said structured packing in said second continuous multi-stage distillation column is of at least one type selected from the group consisting of Mellapak, Gempak, Techno-pack, Flexipac, a Sulzer packing, a Goodroll packing, and Glitschgrid,

23. the process according to any one of items 1 to 22, wherein each of said high boiling point material separating column A and said diphenyl carbonate purifying column B is a distillation column having a tray and / or a packing as said internal,

24. the process according to item 23, wherein each of said internals of said high boiling point material separating column A and said diphenyl carbonate purifying column B is the packing,

25. the process according to item 24, wherein said packing is a structured packing of at least one type selected fromt the group consisting of Mellapak, Gempak, Techno-pack, Flexipac, a Sulzer packing, a Goodroll packing, and Glitschgrid.

In addition, according to the second aspect of the present invention, there are provided:

26. a high-purity diphenyl carbonate produced by the process according to any one of Claims 1 to 25 in an amount of not less than 1 ton / hr,

27. the high-purity diphenyl carbonate according to item 26, which has a halogen content of not more than 0.1 ppm,

28. the high-purity diphenyl carbonate according to item 26, which has a halogen content of not more than 1 ppb,

29. the high-purity diphenyl carbonate according to any one of items 26 to 28, which has a content of by-products having a higher boiling point than that of the diphenyl carbonate of not more than 100 ppm,

30. the high-purity diphenyl carbonate according to item 29, which has a halogen content of not more than 10 ppb, and a content of each of phenyl salicylate, xanthone, phenyl methoxybenzoate, and 1-phenoxycarbonyl-2-phenoxycarboxy-phenylene, which are the by-products having a higher boiling point than that of the diphenyl carbonate, of not more than 30 ppm,

31. the high-purity diphenyl carbonate according to item 30, which has a content of the by-products having a higher boiling point than that of the diphenyl carbonate of not more than 50 ppm,

32. the high-purity diphenyl carbonate according to item 31, which has a halogen content of not more than 1 ppb, and a content of the by-products having a higher boiling point than that of the diphenyl carbonate of not more than 10 ppm.

Advantageous Effects of the Invention

It has been discovered that by implementing the process according to the present invention, a high-purity diphenyl carbonate required for producing a high-quality high-performance aromatic polycarbonate can be produced on an industrial scale of not less than 1 ton / hr, preferably not less than 2 ton / hr, more preferably not less than 3 ton / hr, from a cyclic carbonate and a phenol. Moreover, it has been discovered that the high-purity diphenyl carbonate can be produced stably for a prolonged period of time of, for example, not less than 2000 hours, preferably not less than 3000 hours, more preferably not less than 5000 hours, with a halogen content of not more than 0.1 ppm, preferably not more than 10 ppb, more preferably not more than 1 ppb and an impurity having a high boiling point than that of the diphenyl

carbonate, a content of which being not more than 100 ppm, preferably not more than 50 ppm, more preferably not more than 10 ppm. The present invention thus provides a process that achieves excellent effects as an industrial process for the production of the high-purity diphenyl carbonate.

Brief Description of Drawings

FIG. 1 is an example of schematic drawing of a continuous reactive distillation column To preferable for carrying out the present invention, the distillation column having internals consisting of sieve trays provided inside a trunk portion thereof;

FIG. 2 is an example of schematic drawing of a first continuous reactive distillation column preferable for carrying out the present invention, the distillation column having internals provided in a trunk portion thereof;

FIG. 3 is an example of schematic drawing of a second continuous reactive distillation column preferable for carrying out the present invention, the distillation column having provided, in a trunk portion thereof, internals consisting of structured packings in an upper portion and sieve trays in a lower portion;

FIG. 4 is an example of schematic view of an apparatus comprising the first continuous reactive distillation column and the second continuous reactive distillation column linked together preferable for carrying out the present invention; and

FIG. 5 is an example of schematic view of an apparatus comprising a high boiling point material separating column A and a diphenyl carbonate purifying column B linked together preferable for carrying out the present invention.

Description of Reference Numerals

(In Fig. 1)

1: gas outlet, 2: liquid outlet, 3-a to 3-e: inlet, 4-a to 4-b: inlet, 5: end plate, 6: internal, 7: trunk portion, 10: continuous multi-stage distillation column, $L_0$: length (cm) of trunk portion, $D_0$: inside diameter (cm) of trunk portion, $d_{01}$: inside diameter of gas outlet, $d_{02}$: inside diameter (cm) of liquid outlet

(In Figs 2, 3 and 4)

1: gas outlet, 2: liquid outlet, 3: inlet, 4: inlet, 5: end plate, $L_1$ and $L_2$: length (cm) of trunk portion, $D_1$ and $D_2$: inside diameter (cm) of trunk portion, $d_{11}$ and $d_{21}$: inside diameter (cm) of gas outlet (cm), $d_{12}$ and $d_{22}$: inside diameter (cm) of liquid outlet, 101: first continuous reactive distillation column, 201: second continuous reactive distillation column, 11, 12 and 21: inlet, 13 and 23: column top gas outlet, 14, 24, 18 and 28: heat exchanger, 17 and 27: column bottom liquid outlet, 16 and 26: column top component outlet, 31: column bottom component outlet of second continuous multi-stage distillation column

(In Fig. 5)

A1 and B1: inlet, B2: outlet, 11: column bottom component outlet of high boiling point material separating column A, 13 and 23: column top gas outlet, 14, 24, 18, 28, and 38: heat exchanger, 15 and 25: reflux liquid inlet, 16: column top component outlet of high boiling point material separating column A, 17 and 27: column bottom liquid outlet, 26: column top component outlet of diphenyl carbonate purifying column B, 31: column bottom component outlet of diphenyl carbonate purifying column B, 33: side cut component outlet of diphenyl carbonate purifying column B

Best Mode for Carrying Out the Invention

Following is a detailed description of the present invention.

In the present invention, first, a step (I) of continuously producing a dialkyl carbonate and a diol on an industrial scale from a cyclic carbonate and an aliphatic monohydric alcohol is carried out. The reaction of step (I) is a reversible transesterification reaction represented by following formula:

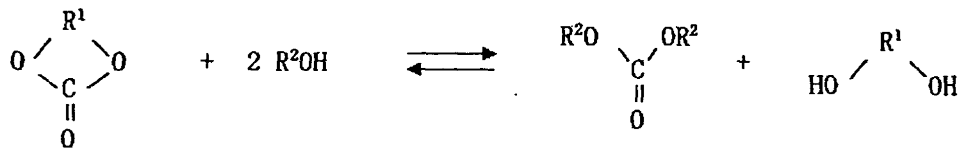

wherein $R^1$ represents a bivalent group $-(CH_2)_m-$ (m is an integer from 2 to 6), one or more of the hydrogens thereof being optionally substituted with an alkyl group or aryl group having 1 to 10 carbon atoms. Moreover, $R^2$ represents a monovalent aliphatic group having 1 to 12 carbon atoms, one or more of the hydrogens thereof being optionally substituted with an alkyl group or aryl group having 1 to 10 carbon atoms.

As the cyclic carbonate, for example, an alkylene carbonate such as ethylene carbonate or propylene carbonate, or 1,3-dioxacyclohexa-2-one, 1,3-dioxacyclohepta-2-one, or the like can be preferably used, ethylene carbonate or propylene carbonate being more preferably used due to ease of procurement and so on, and ethylene carbonate being particularly preferably used.

Moreover, as the aliphatic monohydric alcohol, one having a lower boiling point than the diol produced is used. Although possibly varying depending on the type of the cyclic carbonate used, examples of the monohydric alcohol include methanol, ethanol, propanol (isomers), allyl alcohol, butanol (isomers), 3-buten-1-ol, amyl alcohol (isomers), hexyl alcohol (isomers), heptyl alcohol (isomers), octyl alcohol (isomers), nonyl alcohol (isomers), decyl alcohol (isomers), undecyl alcohol (isomers), dodecyl alcohol (isomers), cyclopentanol, cyclohexanol, cycloheptanol, cyclooctanol, methylcyclopentanol (isomers), ethylcyclopentanol (isomers), methylcyclohexanol (isomers), ethylcyclohexanol (isomers), dimethylcyclohexanol (isomers), diethylcyclohexanol (isomers), phenylcyclohexanol (isomers), benzyl alcohol, phenethyl alcohol (isomers), phenylpropanol (isomers), and so on. Furthermore, these aliphatic monohydric alcohols may be substituted with substituents such as halogens, lower alkoxy groups, cyano groups, alkoxycarbonyl groups, aryloxycarbonyl groups, acyloxy groups, and nitro groups.

Of such aliphatic monohydric alcohols, ones preferably used are alcohols having 1 to 6 carbon atoms, more preferably alcohols having 1 to 4 carbon atoms, i.e. methanol, ethanol, propanol (isomers), and butanol (isomers). In the case of using ethylene carbonate or propylene carbonate as the cyclic carbonate, preferable aliphatic monohydric alcohols are methanol and ethanol, methanol being particularly preferable.

When carrying out the reactive distillation of step (I), the method of making a catalyst be present in the reactive distillation column may be any method, but in the case, for example, of a homogeneous catalyst that dissolves in the reaction liquid under the reaction conditions, the catalyst can be made to be present in a liquid phase in the reactive distillation column by feeding the catalyst into the reactive distillation column continuously, or in the case of a heterogeneous catalyst that does not dissolve in the reaction liquid under the reaction conditions, the catalyst can be made to be present in the reaction system by disposing the catalyst as a solid in the reactive distillation column; these methods may also be used in combination.

In the case that a homogeneous catalyst is continuously fed into the reactive distillation column, the homogeneous catalyst may be fed in together with the cyclic carbonate and/or the aliphatic monohydric alcohol, or may be fed in at a different position to the starting materials. The reaction actually proceeds in the distillation column in a region below the position at which the catalyst is fed in, and hence it is preferable to feed the catalyst into a region between the top of the column and a position slightly below the position(s) at which the starting materials are fed in. The catalyst must be present in at least 5 stages, preferably at least 7 stages, more preferably at least 10 stages.

Moreover, in the case of using a heterogeneous solid catalyst, the catalyst must be present in at least 5 stages, preferably at least 7 stages, more preferably at least 10 stages. A solid catalyst that also has an effect as a packing in the distillation column may be used.

Examples of the catalyst used in step (I) include:

alkali metals and alkaline earth metals such as lithium, sodium, potassium, rubidium, cesium, magnesium, calcium, strontium, and barium;

basic compounds of alkali metals and alkaline earth metals such as hydrides, hydroxides, alkoxides, aryloxides, and amides;

basic compounds of alkali metals and alkaline earth metals such as carbonates, bicarbonates, and organic acid salts;

tertiary amines such as triethylamine, tributylamine, trihexylamine, and benzyldiethylamine;

nitrogen-containing heteroaromatic compounds such as N-alkylpyrroles, N-alkylindoles, oxazoles, N-alkylimidazoles, N-alkylpyrazoles, oxadiazoles, pyridine, alkylpyridines, quinoline, alkylquinolines, isoquinoline, alkylisoquinolines, acridine, alkylacridines, phenanthroline, alkylphenanthrolines, pyrimidine, alkylpyrimidines, pyrazine, alkylpyrazines, triazines, and alkyltriazines;

cyclic amidines such as diazobicycloundecene (DBU) and diazobicyclononene (DBN);

thallium compounds such as thallium oxide, thallium halides, thallium hydroxide, thallium carbonate, thallium nitrate, thallium sulfate, and thallium organic acid salts;
tin compounds such as tributylmethoxytin, tributylethoxytin, dibutyldimethoxytin, diethyldiethoxytin, dibutyldiethoxytin, dibutylphenoxytin, diphenylmethoxytin, dibutyltin acetate, tributyltin chloride, and tin 2-ethylhexanoate;
zinc compounds such as dimethoxyzinc, diethoxyzinc, ethylenedioxyzinc, and dibutoxyzinc;
aluminum compounds such as aluminum trimethoxide, aluminum triisopropoxide, and aluminum tributoxide;
titanium compounds such as tetramethoxytitanium, tetraethoxytitanium, tetrabutoxytitanium, dichlorodimethoxytitanium, tetraisopropoxytitanium, titanium acetate, and titanium acetylacetonate;
phosphorus compounds such as trimethylphosphine, triethylphosphine, tributylphosphine, triphenylphosphine, tributylmethylphosphonium halides, trioctylbutylphosphonium halides, and triphenylmethylphosphonium halides;
zirconium compounds such as zirconium halides, zirconium acetylacetonate, zirconium alkoxides, and zirconium acetate; and
lead and lead-containing compounds, for example lead oxides such as PbO, $PbO_2$, and $Pb_3O_4$;
lead sulfides such as PbS, $Pb_2S_3$, and $PbS_2$;
lead hydroxides such as $Pb(OH)_2$, $Pb_3O_2(OH)_2$, $Pb_2[PbO_2(OH)_2]$, and $Pb_2O(OH)_2$;
plumbites such as $Na_2PbO_2$, $K_2PbO_2$, $NaHPbO_2$, and $KHPbO_2$;
plumbates such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$;
lead carbonates and basic salts thereof such as $PbCO_3$ and $2PbCO_3$-$Pb(OH)_2$;
alkoxylead compounds and aryloxylead compounds such as $Pb(OCH_3)_2$, $(CH_3O)Pb(OPh)$, and $Pb(OPh)_2$;
lead salts of organic acids, and carbonates and basic salts thereof, such as $Pb(OCOCH_3)_2$, $Pb(OCOCH_3)_4$, and $Pb(OCOCH_3)_2 \cdot PbO \cdot 3H_2O$;
organolead compounds such as $Bu_4Pb$, $Ph_4Pb$, $Bu_3PbCl$, $Ph_3PbBr$, $Ph_3Pb$ (or $Ph_6Pb_2$), $Bu_3PbOH$, and $Ph_2PbO$ (wherein Bu represents a butyl group, and Ph represents a phenyl group);
lead alloys such as Pb-Na, Pb-Ca, Pb-Ba, Pb-Sn, and Pb-Sb; lead minerals such as galena and zinc blende; and hydrates of such lead compounds.

In the case that the compound used dissolves in a starting material of the reaction, the reaction mixture, a reaction by-product or the like, the compound can be used as a homogeneous catalyst, whereas in the case that the compound does not dissolve, the compound can be used as a solid catalyst. Furthermore, it is also preferable to use, as a homogeneous catalyst, a mixture obtained by dissolving a compound as above in a starting material of the reaction, the reaction mixture, a reaction by-product or the like in advance, or by reacting to bring about dissolution.

Furthermore, ion exchangers such as anion exchange resins having tertiary amino groups, ion exchange resins having amide groups, ion exchange resins having at least one type of exchange groups selected from sulfonate groups, carboxylate groups and phosphate groups, and solid strongly basic anion exchangers having quaternary ammonium groups as exchange groups; solid inorganic compounds such as silica, silica-alumina, silica-magnesia, aluminosilicates, gallium silicate, various zeolites, various metal-exchanged zeolites, and ammonium-exchanged zeolites, and so on can also be used as the catalyst.

As a solid catalyst, a particularly preferably used one is a solid strongly basic anion exchanger having quaternary ammonium groups as exchange groups, examples thereof including a strongly basic anion exchange resin having quaternary ammonium groups as exchange groups, a cellulose strongly basic anion exchanger having quaternary ammonium groups as exchange groups, and an inorganic carrier supported type strongly basic anion exchanger having quaternary ammonium groups as exchange groups. As a strongly basic anion exchange resin having quaternary ammonium groups as exchange groups, for example a styrene type strongly basic anion exchange resin or the like can be preferably used. A styrene type strongly basic anion exchange resin is a strongly basic anion exchange resin having a copolymer of styrene and divinylbenzene as a parent material, and having quaternary ammonium groups (type I or type II) as exchange groups, and can be schematically represented, for example, by the following formula:

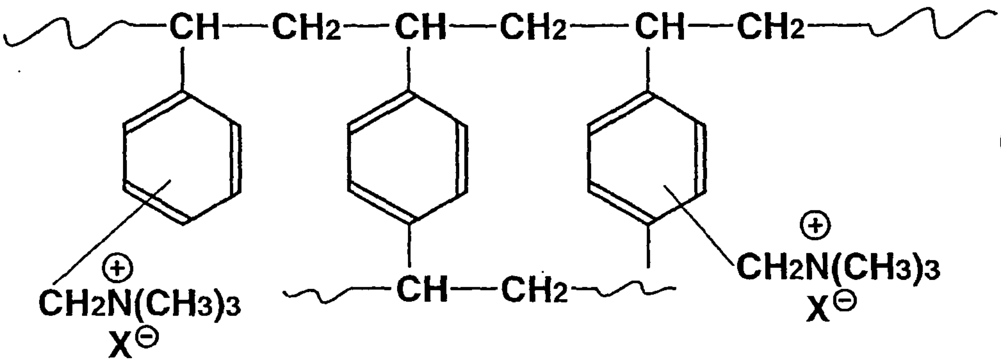

(type I)

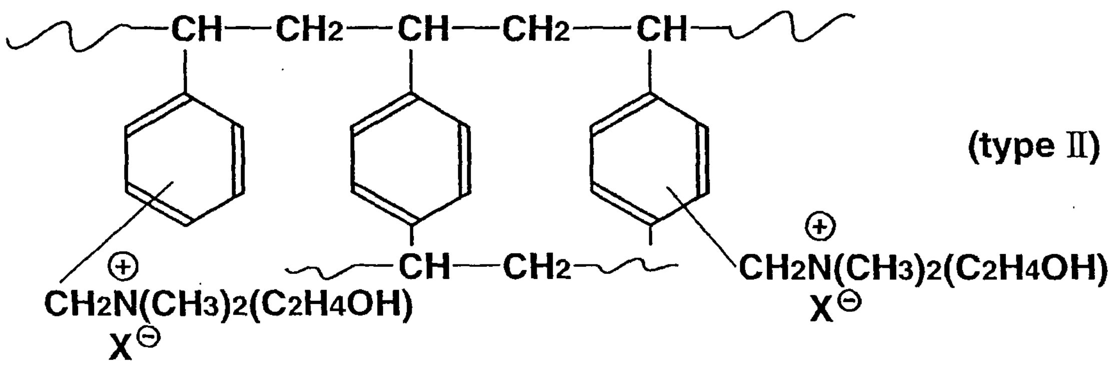

(type II)

wherein X represents an anion; as X, generally at least one type of anion selected from $F^-$, $Cl^-$, $Br^-$, $I^-$, $HCO_3^-$, $CO_3^{2-}$, $CH_3CO_2^-$, $HCO_2^-$, $IO_3^-$, $BrO_3^-$, and $ClO_3^-$ is used, preferably at least one type of anion selected from the group consisting of $Cl^-$, $Br^-$, $HCO_3^-$, and $CO_3^{2-}$. Moreover, as the structure of the resin parent material, either a gel type one or a macroreticular (MR) type one can be used, the MR type being particularly preferable due to the organic solvent resistance being high.

An example of a cellulose strongly basic anion exchanger having quaternary ammonium groups as exchange groups is cellulose having $-OCH_2CH_2NR_3X$ exchange groups obtained by converting some or all of the -OH groups in the cellulose into trialkylaminoethyl groups. Here, R represents an alkyl group; methyl, ethyl, propyl, butyl or the like is generally used, preferably methyl or ethyl. Moreover, X represents an anion as above.

An inorganic carrier supported type strongly basic anion exchanger having quaternary ammonium groups as exchange groups means an inorganic carrier that has had $-O(CH_2)_nNR_3X$ quaternary ammonium groups introduced thereto by modifying some or all of the -OH surface hydroxyl groups of the inorganic carrier. Here, R and X are defined as above. n is generally an integer from 1 to 6, preferably n = 2. As the inorganic carrier, silica, alumina, silica-alumina, titania, a zeolite, or the like can be used, it being preferable to use silica, alumina, or silica-alumina, particularly preferably silica. Any method can be used as the method of modifying the surface hydroxyl groups of the inorganic carrier.

As the solid strongly basic anion exchanger having quaternary ammonium groups as exchange groups, a commercially available one may be used. In this case, the anion exchanger may also be used as the transesterification catalyst after being subjected to ion exchange with a desired anionic species in advance as pretreatment.

Moreover, a solid catalyst comprising a macroreticular or gel-type organic polymer having bonded thereto heterocyclic groups each containing at least one nitrogen atom, or an inorganic carrier having bonded thereto heterocyclic groups each containing at least one nitrogen atom can also be preferably used as the transesterification catalyst. Furthermore, a solid catalyst in which some or all of these nitrogen-containing heterocyclic groups have been converted into a quaternary salt can be similarly used. Note that a solid catalyst such as an ion exchanger may also act as a packing.

The amount of the catalyst used in step (I) varies depending on the type of the catalyst used, but in the case of continuously feeding in a homogeneous catalyst that dissolves in the reaction liquid under the reaction conditions, the amount used is generally in a range of from 0.0001 to 50 % by weight, preferably from 0.005 to 20 % by weight, more preferably from 0.01 to 10 % by weight, as a proportion of the total weight of the cyclic carbonate and the aliphatic monohydric alcohol fed in as the starting materials. Moreover, in the case of using a solid catalyst installed in the distillation

column, the catalyst is preferably used in an amount in a range of from 0.01 to 75 vol%, more preferably 0.05 to 60 vol%, yet more preferably 0.1 to 60 vol%, based on the empty column volume of the distillation column.

There are no particular limitations on the method of continuously feeding the starting material cyclic carbonate and aliphatic monohydric alcohol into a continuous multi-stage distillation column To constituting the reactive distillation column used in step (I); any feeding method may be used so long as the cyclic carbonate and the aliphatic monohydric alcohol can be made to contact the catalyst in a region of at least 5 stages, preferably at least 7 stages, more preferably at least 10 stages, of the distillation column. That is, the cyclic carbonate and the aliphatic monohydric alcohol can be continuously fed in from a required number of inlets in stages of the continuous multi-stage distillation column satisfying the conditions described earlier. Moreover, the cyclic carbonate and the aliphatic monohydric alcohol may be introduced into the same stage of the distillation column, or may be introduced into different stages to one another.

The starting material cyclic carbonate and aliphatic monohydric alcohol are fed continuously into the continuous multi-stage distillation column To in a liquid form, in a gaseous form, or as a mixture of a liquid and a gas. Other than feeding the starting materials into the distillation column in this way, it is also preferable to additionally feed in a gaseous starting material intermittently or continuously from a lower portion of the distillation column. Moreover, another preferable method is one in which the cyclic carbonate is continuously fed in a liquid form or a gas / liquid mixed form into a stage of the distillation column above the stages in which the catalyst is present, and the aliphatic monohydric alcohol is continuously fed in a gaseous form and / or a liquid form into the lower portion of the distillation column. In this case, the cyclic carbonate may of course contain the aliphatic monohydric alcohol.

In step (I), the starting materials fed in may contain the product dialkyl carbonate and / or diol. The content thereof is, for the dialkyl carbonate, generally in a range of from 0 to 40 % by weight, preferably from 0 to 30 % by weight, more preferably from 0 to 20 % by weight, in terms of the percentage by weight of the dialkyl carbonate in the aliphatic monohydric alcohol / dialkyl carbonate mixture, and is, for the diol, generally in a range of from 0 to 10 % by weight, preferably from 0 to 7 % by weight, more preferably from 0 to 5 % by weight, in terms of the percentage by weight of the diol in the cyclic carbonate / diol mixture.

When carrying out the reaction of step (I) industrially, besides fresh cyclic carbonate and / or aliphatic monohydric alcohol newly introduced into the reaction system, material having the cyclic carbonate and / or the aliphatic monohydric alcohol as a main component thereof recovered from this process and / or another process can also be preferably used for the starting materials. It is an excellent characteristic feature of the present invention that this is possible. An example of another process is step (II) in which a diphenyl carbonate is produced from the dialkyl carbonate and a phenol, the aliphatic monohydric alcohol being by-produced in step (II) and recovered. The recovered by-produced aliphatic monohydric alcohol generally often contains the dialkyl carbonate, the aromatic monohydroxy compound, an alkyl aryl ether and so on, and may also contain small amounts of an alkyl aryl carbonate, the diphenyl carbonate and so on. The by-produced aliphatic monohydric alcohol may be used as is as a starting material in step (I), or may be used as a starting material in step (I) after the amount of contained material having a higher boiling point than that of the aliphatic monohydric alcohol has been reduced through distillation or the like.

A cyclic carbonate preferably used in step (I) is one produced through reaction between, for example, an alkylene oxide such as ethylene oxide, propylene oxide or styrene oxide and carbon dioxide; a cyclic carbonate containing small amounts of such compounds or the like may be used as a starting material in step (I).

In step (I), a ratio between the amounts of the cyclic carbonate and the aliphatic monohydric alcohol fed into the reactive distillation column varies according to the type and amount of the transesterification catalyst and the reaction conditions, but a molar ratio of the aliphatic monohydric alcohol to the cyclic carbonate fed in is generally in a range of from 0.01 to 1000 times. To increase the cyclic carbonate conversion, it is preferable to feed in the aliphatic monohydric alcohol in an excess of at least 2 times the number of mols of the cyclic carbonate, but if the amount of the aliphatic monohydric alcohol used is too great, then it is necessary to make the apparatus larger. For such reasons, the molar ratio of the aliphatic monohydric alcohol to the cyclic carbonate is preferably in a range of from 2 to 20, more preferably from 3 to 15, yet more preferably from 5 to 12. Furthermore, if much unreacted cyclic carbonate remains, then the unreacted cyclic carbonate may react with the product diol to by-produce oligomers such as a dimer or a trimer, and hence in industrial implementation, it is preferable to reduce the amount of unreacted cyclic carbonate remaining as much as possible. In the process of the present invention, even if the above molar ratio is not more than 10, the cyclic carbonate conversion can be made to be not less than 97%, preferably not less than 98%, more preferably not less than 99%. This is another characteristic feature of the present invention.

In step (I), preferably not less than approximately 0.4 ton / hr of the dialkyl carbonate is continuously produced; the minimum amount of the cyclic carbonate continuously fed in to achieve this is generally 0.44 P ton / hr, preferably 0.42 P ton / hr, more preferably 0.4 P ton / hr, based on the amount P (ton / hr) of the dialkyl carbonate to be produced. In a yet more preferable case, this amount can be made to be less than 0.39 P ton / hr.

The continuous multi-stage distillation column To used in step (I) comprises a structure having a cylindrical trunk portion having a length $L_0$ (cm) and an inside diameter $D_0$ (cm) and having internals with a number of stages no thereinside, and further having a gas outlet having an inside diameter $d_{01}$ (cm) at a top of the column or in an upper

portion of the column near to the top, a liquid outlet having an inside diameter $d_{02}$ (cm) at a bottom of the column or in a lower portion of the column near to the bottom, at least one first inlet provided in the upper portion and/or a middle portion of the column below the gas outlet, and at least one second inlet provided in the middle portion and / or the lower portion of the column above the liquid outlet, wherein $L_0$, $D_0$, $L_0$ / $D_0$, $n_0$, $D_0$ / $d_{01}$, and $D_0$ / $d_{02}$ must satisfy respectively the following formulae (1) to (6);

$$2100 \leq L_0 \leq 8000 \quad (1)$$

$$180 \leq D_0 \leq 2000 \quad (2)$$

$$4 \leq L_0 / D_0 \leq 40 \quad (3)$$

$$10 \leq n_0 \leq 120 \quad (4)$$

$$3 \leq D_0 / d_{01} \leq 20 \quad (5)$$

and

$$5 \leq D_0 / d_{02} \leq 30 \quad (6).$$

Note that the term "the top of the column or the upper portion of the column near to the top" used in the present invention means the portion from the top of the column downward as far as approximately 0.25 $L_0$, and the term "the bottom of the column or the lower portion of the column near to the bottom" means the portion from the bottom of the column upward as far as approximately 0.25 $L_0$. (Likewise 0.25 $L_1$ and 0.25 $L_2$ respectively for the first and second continuous multi-stage distillation columns, and 0.25 $L_A$ and 0.25 $L_B$ respectively for the high boiling point material separating column A and the diphenyl carbonate purifying column B.)

It has been discovered that by using the continuous multi-stage distillation column $T_0$ that simultaneously satisfies the formulae (1), (2), (3), (4), (5) and (6), the dialkyl carbonate and the diol can be produced on an industrial scale of preferably not less than 0.4 ton / hr of the dialkyl carbonate and / or preferably not less than 0.26 ton / hr of the diol with a high conversion, high selectivity, and high productivity stably for a prolonged period of time of, for example, not less than 1000 hours, preferably not less than 3000 hours, more preferably not less than 5000 hours, from the cyclic carbonate and the aliphatic monohydric alcohol. The reason why it has become possible to produce the dialkyl carbonate and the diol on an industrial scale with such excellent effects by implementing step (I) as described above is not clear, but this is supposed to be due to a composite effect brought about when the conditions of formulae (1) to (6) are combined. Preferable ranges for the respective factors are described below.

If $L_0$ (cm) is less than 2100, then the conversion decreases and hence it is not possible to attain the desired production amount. Moreover, to keep down the equipment cost while securing the conversion enabling the desired production amount to be attained, $L_0$ must be made to be not more than 8000. A more preferable range for $L_0$ (cm) is $2300 \leq L_0 \leq 6000$, with $2500 \leq L_0 \leq 5000$ being yet more preferable.

If $D_0$ (cm) is less than 180, then it is not possible to attain the desired production amount. Moreover, to keep down the equipment cost while attaining the desired production amount, $D_0$ must be made to be not more than 2000. A more preferable range for $D_0$ (cm) is $200 \leq D_0 \leq 1000$, with $210 \leq D_0 \leq 800$ being yet more preferable.

If $L_0$ / $D_0$ is less than 4 or greater than 40, then stable operation becomes difficult. In particular, if $L_0$ / $D_0$ is greater than 40, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. A

more preferable range for $L_0$ / $D_0$ is $5 \leq L_0 / D_0 \leq 30$, with $7 \leq L_0 / D_0 \leq 20$ being yet more preferable.

If no is less than 10, then the conversion decreases and hence it is not possible to attain the desired production amount. Moreover, to keep down the equipment cost while securing the conversion enabling the desired production amount to be attained, no must be made to be not more than 120. Furthermore, if no is greater than 120, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. A more preferable range for no is 30 ≤ no ≤ 100, with 40 ≤ no ≤ 90 being yet more preferable.

If $D_0$ / $d_{01}$ is less than 3, then the equipment cost becomes high. Moreover, a large amount of a gaseous component is readily released to the outside of the system, and hence stable operation becomes difficult. If $D_0$ / $d_{01}$ is greater than 20, then the gaseous component withdrawal amount becomes relatively low, and hence stable operation becomes difficult, and moreover a decrease in the conversion is brought about. A more preferable range for $D_0$ / $d_{01}$ is $4 \leq D_0 / d_{01} \leq 15$, with $5 \leq D_0 / d_{01} \leq 13$ being yet more preferable.

If $D_0$ / $d_{02}$ is less than 5, then the equipment cost becomes high. Moreover, the liquid withdrawal amount becomes relatively high, and hence stable operation becomes difficult. If $D_0$ / $d_{02}$ is greater than 30, then the flow rate through the liquid outlet and piping becomes excessively fast, and hence erosion becomes liable to occur, bringing about corrosion of the apparatus. A more preferable range for $D_0$ / $d_{02}$ is $7 \leq D_0 / d_{02} \leq 25$, with $9 \leq D_0 / d_{02} \leq 20$ being yet more preferable.

Furthermore, it has been found that it is further preferable for $d_{01}$ and $d_{02}$ for the continuous multi-stage distillation column To used in step (I) to satisfy the formula (28);

$$1 \leq d_{01} / d_{02} \leq 5 \qquad (28).$$

The term "prolonged stable operation" referred to in step (I) means that operation can be carried out continuously in a steady state based on the operating conditions with no flooding, clogging of piping, or erosion for not less than 1000 hours, preferably not less than 3000 hours, more preferably not less than 5000 hours, and predetermined amounts of the dialkyl carbonate and the diol can be produced while maintaining a high conversion, high selectivity, and high productivity.

The "selectivity" for each of the dialkyl carbonate and the diol in step (I) is based on the cyclic carbonate reacted. In the present invention, a high selectivity of not less than 95% can generally be attained, preferably not less than 97%, more preferably not less than 99%. Moreover, the "conversion" in step (I) generally indicates the cyclic carbonate conversion, in the present invention it being possible to make the cyclic carbonate conversion be not less than 95%, preferably not less than 97%, more preferably not less than 99%, yet more preferably not less than 99.5%, still more preferably not less than 99.9%. It is one of the excellent characteristic features of step (I) that the high conversion can be maintained while maintaining high selectivity in this way.

The continuous multi-stage distillation column To used in step (I) is preferably a distillation column having trays and/or packings as the internal. The term "internal" used in the present invention means the part in the distillation column where gas and liquid are actually brought into contact with one another. Examples of the trays include a bubble-cap tray, a sieve tray, a valve tray, a counterflow tray, a Superfrac tray, a Maxfrac tray, or the like. Examples of the packings include an irregular packing such as a Raschig ring, a Lessing ring, a Pall ring, a Berl saddle, an Intalox saddle, a Dixon packing, a McMahon packing or Heli-Pak, or a structured packing such as Mellapak, Gempak, Techno-pack, Flexipac, a Sulzer packing, a Goodroll packing or Glitschgrid. A multi-stage distillation column having both a tray portion and a portion packed with the packings can also be used. Furthermore, the term "number of stages of the internal" used in the present invention means the number of trays in the case of the trays, and the theoretical number of stages in the case of the packings. In the case of a multi-stage distillation column having both a tray portion and a portion packed with the packings, the number of stages is thus the sum of the number of tray and the theoretical number of stage.

In step (I) in which the cyclic carbonate and the aliphatic monohydric alcohol are reacted together, it has been discovered that a high conversion, high selectivity, and high productivity can be attained even if a plate type continuous multi-stage distillation column in which the internals comprise the trays and / or the packings having a predetermined number of stages and / or a packed column type continuous multi-stage distillation column is used, but a plate type distillation column in which the internal is the trays is preferable. Furthermore, it has been discovered that sieve trays each having a sieve portion and a downcomer portion are particularly good as the tray in terms of the relationship between performance and equipment cost. It has also been discovered that each sieve tray preferably has 100 to 1000 holes / $m^2$ in the sieve portion. A more preferable number of holes is 120 to 900 holes / $m^2$, yet more preferably 150 to 800 holes /$m^2$. Moreover, it has been discovered that the cross-sectional area per hole of each sieve tray is preferably in a range of from 0.5 to 5 $cm^2$. A more preferable cross-sectional area per hole is from 0.7 to 4 $cm^2$, yet more preferably

from 0.9 to 3 $cm^2$. Furthermore, it has been discovered that it is particularly preferable if each sieve tray has 100 to 1000 holes / $m^2$ in the sieve portion, and the cross-sectional area per hole is in a range of from 0.5 to 5 $cm^2$.

Furthermore, it has been discovered that an aperture ratio of each of the sieve trays is preferably in a range of from 1.5 to 15%. A more preferable aperture ratio is in a range of from 1.7 to 13%, yet more preferably from 1.9 to 11 %. Here, the "aperture ratio" of the sieve tray indicates the ratio of the cross-sectional area of all of the holes (the total cross-sectional area of the holes) present in the sieve tray to the area (including the total cross-sectional area of the holes) of the sieve portion. The area of the sieve portion and / or the total cross-sectional area of the holes may differ between the sieve trays, in which case the aperture ratio of each of the sieve trays is preferably within the above range. Furthermore, the number of holes in the sieve portion may be the same for all of the sieve trays, or may differ. It has been shown that by adding the above conditions to the continuous multi-stage distillation column To, the object for step (I) can be attained more easily.

When carrying out step (I), a dialkyl carbonate and a diol are continuously produced by continuously feeding a cyclic carbonate and an aliphatic monohydric alcohol as starting materials into a continuous multi-stage distillation column in which a catalyst is present, carrying out reaction and distillation simultaneously in the column, continuously withdrawing a low boiling point reaction mixture containing the produced dialkyl carbonate from an upper portion of the column in a gaseous form, and continuously withdrawing a high boiling point reaction mixture containing the diol from a lower portion of the column in a liquid form.

Moreover, in step (I), as the continuous feeding of the starting material cyclic carbonate and aliphatic monohydric alcohol into the continuous multi-stage distillation column To, the cyclic carbonate and the aliphatic monohydric alcohol may be fed in as a starting material mixture or separately, in a liquid form and / or a gaseous form, from inlet(s) provided in one place or a plurality of places in the upper portion or the middle portion of the column below the gas outlet in the upper portion of the distillation column. A method in which the cyclic carbonate or a starting material containing a large amount of the cyclic carbonate is fed into the distillation column in a liquid form from inlet(s) in the upper portion or the middle portion of the distillation column, and the aliphatic monohydric alcohol or a starting material containing a large amount of the aliphatic monohydric alcohol is fed into the distillation column in a gaseous form from inlet(s) provided in the middle portion or the lower portion of the column above the liquid outlet in the lower portion of the distillation column is also preferable.

The reaction time for the transesterification reaction carried out in step (I) is considered to equate to the average residence time of the reaction liquid in the continuous multi-stage distillation column $T_0$. The reaction time varies depending on the form of the internals in the distillation column and the number of stages, the amounts of the starting materials fed in, the type and amount of the catalyst, the reaction conditions, and so on. The reaction time is generally in a range of from 0.1 to 20 hours, preferably from 0.5 to 15 hours, more preferably from 1 to 10 hours.

The reaction temperature in step (I) varies depending on the type of the starting material compound used, and the type and amount of the catalyst. The reaction temperature is generally in a range of from 30 to 300 °C. It is preferable to increase the reaction temperature so as to increase the reaction rate. However, if the reaction temperature is too high, then side reactions become liable to occur. The reaction temperature is thus preferably in a range of from 40 to 250 °C, more preferably from 50 to 200 °C, yet more preferably from 60 to 150 °C. In the present invention, the reactive distillation can be carried out with the column bottom temperature set to not more than 150 °C, preferably not more than 130 °C, more preferably not more than 110 °C, yet more preferably not more than 100 °C. An excellent characteristic feature of step (I) is that the high conversion, high selectivity, and high productivity can be attained even with such a low column bottom temperature. Moreover, the reaction pressure varies depending on the type of the starting material compounds used and the composition therebetween, the reaction temperature, and so on. The reaction pressure may be any of a reduced pressure, normal pressure, or an applied pressure, and is generally in a range of from 1 to $2x10^7$ Pa, preferably from $10^3$ to $10^7$ Pa, more preferably from $10^4$ to $5x10^6$ Pa.

The reflux ratio used for the continuous multi-stage distillation column To in step (I) is generally in a range of from 0 to 10, preferably from 0.01 to 5, more preferably from 0.05 to 3.

The material constituting the continuous multi-stage distillation column To used in step (I) is generally a metallic material such as carbon steel or stainless steel. In terms of the quality of the dialkyl carbonate and diol to be produced, stainless steel is preferable.

In the present invention, next, a step (II) of continuously producing a diphenyl carbonate on an industrial scale from the dialkyl carbonate produced in step (I) and a phenol is carried out.

The dialkyl carbonate used in step (II) is a compound represented by the following formula as shown earlier in the following formula:

$$R^2OCOOR^2$$

wherein $R^2$ is defined as before.

Examples of dialkyl carbonates having such an $R^2$ include dimethyl carbonate, diethyl carbonate, dipropyl

carbonate (isomers), diallyl carbonate, dibutenyl carbonate (isomers), dibutyl carbonate (isomers), dipentyl carbonate (isomers), dihexyl carbonate (isomers), diheptyl carbonate (isomers), dioctyl carbonate (isomers), dinonyl carbonate (isomers), didecyl carbonate (isomers), dicyclopentyl carbonate, dicyclohexyl carbonate, dicycloheptyl carbonate, dibenzyl carbonate, diphenethyl carbonate (isomers), di(phenylpropyl) carbonate (isomers), di(phenylbutyl) carbonate (isomers), di(chlorobenzyl) carbonate (isomers), di(methoxybenzyl) carbonate (isomers), di(methoxymethyl) carbonate, di (methoxyethyl) carbonate (isomers), di(chloroethyl) carbonate (isomers) and di(cyanoethyl) carbonate (isomers).

Of these dialkyl carbonates, ones preferably used in the present invention are dialkyl carbonates in which $R^2$ is an alkyl group having not more than four carbon atoms and not containing a halogen atom. A particularly preferable one is dimethyl carbonate. Moreover, of preferable dialkyl carbonates, particularly preferable ones are dialkyl carbonates produced in a state substantially not containing a halogen, for example ones produced from an alkylene carbonate substantially not containing a halogen and an alcohol substantially not containing a halogen.

The phenol used in step (II) is one represented by the following general formula, being a compound in which a hydroxyl group is directly bonded to a phenyl group (Ph). In some cases, a substituted phenol in which the phenyl group is substituted with lower alkyl group(s) or lower alkoxy group(s) may be used:

PhOH

The phenol used in the present invention preferably substantially does not contain a halogen. The diphenyl carbonate referred to in the present invention is thus one represented by the following formula:

PhOCOOPh

A molar ratio of the dialkyl carbonate to the phenol used in the starting material in step (II) is preferably in a range of from 0.1 to 10. Outside this range, the amount of unreacted starting material remaining based on a predetermined amount of the desired diphenyl carbonate produced becomes high, which is not efficient, and moreover much energy is required to recover the starting material. For such reasons, the above molar ratio is more preferably in a range of from 0.5 to 5, yet more preferably from 0.8 to 3, still more preferably from 1 to 2.

In the present invention, not less than 1 ton / hr of high-purity diphenyl carbonate must be continuously produced.

In step (II), the minimum amount of the phenol fed in continuously is thus generally 15 Q ton / hr, preferably 13 Q ton / hr, more preferably 10 Q ton / hr, based on the amount of the high-purity diphenyl carbonate (Q ton / hr) to be produced. In a more preferable case, this amount can be made to be less than 8 Q ton / hr.

The dialkyl carbonate and the phenol used in the starting material in step (II) may each be of high purity, or may contain other compounds, for example may contain compounds or reaction by-products produced in the first continuous multi-stage distillation column and / or a second continuous multi-stage distillation column, described below. In the case of industrial implementation, for the starting material, besides fresh dialkyl carbonate and phenol newly introduced into the reaction system, it is also preferable to use dialkyl carbonate and / or phenol recovered from the first continuous multi-stage distillation column and / or the second continuous multi-stage distillation column. In the process according to the present invention, column top components constituting a low boiling point reaction mixture from the second continuous multi-stage distillation column are fed into the first continuous multi-stage distillation column. Here, the second column low boiling point reaction mixture may be fed into the first continuous multi-stage distillation column as is, or after some of the components thereof have been separated out.

Accordingly, in the present invention, which is implemented industrially, it is preferable for the starting material fed into the first continuous multi-stage distillation column to contain any of an alcohol, an alkyl phenyl carbonate, the diphenyl carbonate, an alkyl phenyl ether, and so on. A starting material further containing small amounts of high boiling point by-products such as Fries rearrangement products of the alkyl phenyl carbonate and diphenyl carbonate which are the products, derivatives thereof, and so on can also be preferably used. In the present invention, in the case, for example, of producing methyl phenyl carbonate and diphenyl carbonate using a starting material containing dimethyl carbonate as the dialkyl carbonate and unsubstituted phenol as the phenol, it is preferable for this starting material to contain methanol, and methyl phenyl carbonate and diphenyl carbonate, which are the reaction products, and the starting material may further contain small amounts of anisole, phenyl salicylate and methyl salicylate, which are reaction by-products, and high boiling point by-products derived therefrom.

The high-purity diphenyl carbonate of the present invention is preferably used for producing an aromatic polycarbonate through polymerization with an aromatic dihydroxy compound, and in this polymerization step a large amount of the phenol is by-produced and recovered. This by-produced phenol is preferably used as starting material in step (II) of the present invention.

The diphenyl carbonate produced in step (II) is obtained through transesterification between the dialkyl carbonate and the phenol. Included under this transesterification are a reaction in which one or both of the alkoxy groups of the dialkyl carbonate is/are exchanged with the phenoxy group of the phenol and an alcohol is eliminated, and a

reaction in which two molecules of the alkyl phenyl carbonate produced are converted into the diphenyl carbonate and the dialkyl carbonate through a transesterification reaction therebetween, i.e. a disproportionation reaction. In step (II), in the first continuous multi-stage distillation column, the alkyl phenyl carbonate is mainly obtained, and in the second continuous multi-stage distillation column, the diphenyl carbonate and the dialkyl carbonate are mainly obtained through the disproportionation reaction of the alkyl phenyl carbonate. The diphenyl carbonate obtained in step (II) does not contain a halogen at all, and hence is important as a starting material when industrially producing the aromatic polycarbonate in the present invention. The reason for this is that if a halogen is present in the starting material for the polymerization even in a small amount of less than 1 ppm, then this may impede the polymerization reaction, thus impeding stable production of the aromatic polycarbonate, or cause a deterioration of the properties of, or discoloration of, the aromatic polycarbonate produced.

As a catalyst used in the first continuous multi-stage distillation column and/or the second continuous multi-stage distillation column in step (II), for example, a metal-containing compound selected from the following compounds can be used:

<lead compounds>
lead oxides such as $PbO$, $PbO_2$ and $Pb_3O_4$;
lead sulfides such as $PbS$ and $Pb_2S$;
lead hydroxides such as $Pb(OH)_2$ and $Pb_2O_2(OH)_2$;
plumbites such as $Na_2PbO_2$ $K_2PbO_2$, $NaHPbO_2$ and $KHPbO_2$
plumbates such as $Na_2PbO_3$, $Na_2H_2PbO_4$, $K_2PbO_3$, $K_2[Pb(OH)_6]$, $K_4PbO_4$, $Ca_2PbO_4$ and $CaPbO_3$;
lead carbonates and basic salts thereof such as $PbCO_3$ and $2PbCO_3$ $Pb(OH)_2$;
lead salts of organic acids, and carbonates and basic salts thereof, such as $Pb(OCOCH_3)_2$, $Pb(OCOCH_3)_4$ and $Pb(OCOCH_3)_2 \cdot PbO \cdot 3H_2O$,
organolead compounds such as $Bu_4Pb$, $Ph_4Pb$, $Bu_3PbCl$, $Ph_3PbBr$, $Ph_3Pb$ (or $Ph_6Pb_2$), $BU_3PbOH$ and $Ph_3PbO$ (wherein Bu represents a butyl group, and Ph represents a phenyl group);
alkoxylead compounds and aryloxylead compounds such as $Pb(OCH_3)_2$, $(CH_3O)Pb(OPh)$ and $Pb(OPh)_2$;
lead alloys such as Pb-Na, Pb-Ca, Pb-Ba, Pb-Sn and Pb-Sb; lead minerals such as galena and zinc blende; and hydrates of such lead compounds;
<copper family metal compounds>
salts and complexes of copper family metals such as $CuCl$, $CuCl_2$, $CuBr$, $CuBr_2$, $CuI$, $CuI_2$, $Cu(OAc)_2$, $Cu(acac)_2$, copper oleate, $Bu_2Cu$, $(CH_3O)_2Cu$, $AgNO_3$, $AgBr$, silver picrate, $AgC_6H_6ClO_4$, $[AuC{=}C{-}C(CH_3)_3]_n$ and $[Cu(C_7H_8)Cl]_4$ (wherein acac represents an acetylacetone chelate ligand);
<alkali metal complexes>
alkali metal complexes such as $Li(acac)$ and $LiN(C_4H_9)_2$;
<zinc complexes>
zinc complexes such as $Zn(acac)_2$;
<cadmium complexes>
cadmium complexes such as $Cd(acac)_2$;
<iron family metal compounds>
complexes of iron family metals such as $Fe(C_{10}H_8)(CO)_5$, $Fe(CO)_5$, $Fe(C_4H_6)(CO)_3$, $Co(mesitylene)_2$, $(PEt_2Ph)_2$, $CoC_5F_5(CO)_7$, $Ni{-}\pi{-}C_5H_5NO$ and ferrocene;
<zirconium complexes>
zirconium complexes such as $Zr(acac)_4$ and zirconocene;
<Lewis acid type compounds>
Lewis acids and Lewis acid-forming transition metal compounds such as $AlX_3$, $TiX_3$, $TiX_4$, $VOX_3$, $VX_5$, $ZnX_2$, $FeX_3$ and $SnX_4$ (wherein X represents a halogen atom, an acetoxy group, an alkoxy group or an aryloxy group); and
<organo-tin compounds>
organo-tin compounds such as $(CH_3)_3SnOCOCH_3$, $(C_2H_5)_3SnOCOC_6H_5$, $BU_3SnOCOCH_3$, $Ph_3SnOCOCH_3$, $Bu_2Sn(OCOCH_3)_2$, $BU_2Sn(OCOC_{11}H_{23})_2$, $Ph_3SnOCH_3$, $(C_2H_5)_3SnOPh$, $Bu_2Sn(OCH_3)_2$, $Bu_2Sn(OC_2H_5)_2$, $Bu_2Sn(OPh)_2$, $Ph_2Sn(OCH_3)_2$, $(C_2Hs)_3SnOH$, $Ph_3SnOH$, $Bu_2SnO$, $(C_8H_{17})_2SnO$, $Bu_2SnCl_2$ and $BuSnO(OH)$.

A catalyst used in the present invention is a homogeneous catalyst that dissolves in the reaction system, and preferably contains a metal such as Pb, Cu, Zn, Fe, Co, Ni, Al, Ti, V or Sn. A catalyst in which such a metallic component is bonded to organic groups can thus be preferably used. The catalyst component may of course have been reacted with an organic compound present in the reaction system such as an aliphatic alcohol, the phenol, the alkyl phenyl carbonate, the diphenyl carbonate or the dialkyl carbonate, or may have been subjected to heating treatment with the starting material or products prior to the reaction. The catalyst used in the present invention is preferably one that has a high solubility in the reaction liquid under the reaction conditions. Examples of preferable catalysts in this sense include

PbO, $Pb(OH)_2$ and $Pb(OPh)_2$; $TiCl_4$, $Ti(OMe)_4$, $(MeO)Ti(OPh)_3$, $(MeO)_2Ti(OPh)_2$, $(MeO)_3Ti(OPh)$ and $Ti(OPh)_4$; $SnCl_4$, $Sn(OPh)_4$, $Bu_2SnO$ and $Bu_2Sn(OPh)_2$; $FeCl_3$, $Fe(OH)_3$ and $Fe(OPh)_3$; and such catalysts that have been treated with phenol, the reaction liquid or the like. The catalyst used in the first continuous multi-stage distillation column and the catalyst used in the second continuous multi-stage distillation column may be the same as one another, or different.

In the present invention, it is particularly preferable to use a starting material and catalyst not containing a halogen. In this case, the diphenyl carbonate produced does not contain a halogen at all, and hence is important as a starting material when industrially producing a polycarbonate by means of a transesterification method. The reason for this is that if a halogen is present in the starting material for the polymerization in even an amount less than, for example, 1 ppm, then this may impede the polymerization reaction, or cause a deterioration of the properties of, or discoloration of, the polycarbonate produced.

The first continuous multi-stage distillation column used in step (II) comprises a structure having a cylindrical trunk portion having a length $L_1$ (cm) and an inside diameter $D_1$ (cm), and having internals with a number of stages $n_1$ thereinside, and further having a gas outlet having an inside diameter $d_{11}$ (cm) at a top of the column or in an upper portion of the column near to the top, a liquid outlet having an inside diameter $d_{12}$ (cm) at a bottom of the column or in a lower portion of the column near to the bottom, at least one third inlet provided in the upper portion and / or a middle portion of the column below the gas outlet, and at least one fourth inlet provided in the middle portion and / or the lower portion of the column above the liquid outlet, wherein $L_1$, $D_1$, $L_1$ / $D_1$, $n_1$, $D_1$ / $d_{11}$, and $D_1$ / $d_{12}$ must satisfy the following formulae (7) to (12) respectively;

$$1500 \leq L_1 \leq 8000 \qquad (7)$$

$$100 \leq D_1 \leq 2000 \qquad (8)$$

$$2 \leq L_1 / D_1 \leq 40 \qquad (9)$$

$$20 \leq n_1 \leq 120 \qquad (10)$$

$$5 \leq D_1 / d_{11} \leq 30 \qquad (11)$$

and

$$3 \leq D_1 / d_{12} \leq 20 \qquad (12).$$

Moreover, the second continuous multi-stage distillation column used in step (II) comprises a structure having a cylindrical trunk portion having a length $L_2$ (cm) and an inside diameter $D_2$ (cm), and having internals with a number of stages $n_2$ thereinside, and has a gas outlet having an inside diameter $d_{21}$ (cm) at a top of the column or in an upper portion of the column near to the top, a liquid outlet having an inside diameter $d_{22}$ (cm) at a bottom of the column or in a lower portion of the column near to the bottom, at least one fifth inlet provided in the upper portion and/or a middle portion of the column below the gas outlet, and at least one sixth inlet provided in the middle portion and / or the lower portion of the column above the liquid outlet, wherein $L_2$, $D_2$, $L_2$ / $D_2$, $n_2$, $D_2$ / $d_{21}$, and $D_2$ / $d_{22}$ must satisfy the following formulae (13) to (18) respectively;

$$1500 \leq L_2 \leq 8000 \qquad (13)$$

$$100 \leq D_2 \leq 2000 \qquad (14)$$

$$2 \leq L_2 / D_2 \leq 40 \qquad (15)$$

$$10 \leq n_2 \leq 80 \qquad (16)$$

$$2 \leq D_2 / d_{21} \leq 15 \qquad (17)$$

and

$$5 \leq D_2 / d_{22} \leq 30 \qquad (18).$$

It has been discovered that by using the first continuous multi-stage distillation column and the second continuous multi-stage distillation column that simultaneously satisfy the formulae (7) to (18), the diphenyl carbonate can be produced from the dialkyl carbonate and the phenol on an industrial scale of not less than approximately 0.85 ton / hr, preferably not less than 1 ton / hr, with high selectivity and high productivity stably for a prolonged period of time, for example not less than 2000 hours, preferably not less than 3000 hours, more preferably not less than 5000 hours. The reason why it has become possible to produce such an aromatic carbonate on an industrial scale with such excellent effects by implementing the process according to the present invention is not clear, but this is supposed to be due to a composite effect brought about when the conditions of the formulae (7) to (18) are combined. Preferable ranges for the respective factors for the continuous multi-stage distillation columns used in step (II) are described below.

If $L_1$ (cm) and $L_2$ (cm) are less than 1500, then the conversion decreases and hence it is not possible to attain the desired production amount. Moreover, to keep down the equipment cost while securing the conversion enabling the desired production amount to be attained, $L_1$ and $L_2$ must each be made to be not more than 8000. More preferable ranges for $L_1$ (cm) and $L_2$ (cm) are respectively $2000 \leq L_1 \leq 6000$ and $2000 \leq L_2 \leq 6000$, with $2500 \leq L_1 \leq 5000$ and $2500 \leq L_2 \leq 5000$ being yet more preferable.

If $D_1$ (cm) and $D_2$ (cm) are less than 100, then it is not possible to attain the desired production amount. Moreover, to keep down the equipment cost while attaining the desired production amount, $D_1$ and $D_2$ must each be made to be not more than 2000. More preferable ranges for $D_1$ (cm) and $D_2$ (cm) are respectively $150 \leq D_1 \leq 1000$ and $150 \leq D_2 < 1000$, with $200 \leq D_1 \leq 800$ and $200 \leq D_2 \leq 800$ being yet more preferable.

For the first continuous multi-stage distillation column and the second continuous multi-stage distillation column, so long as $D_1$ and $D_2$ are within the above ranges, each of the columns may have the same inside diameter from the upper portion thereof to the lower portion thereof, or the inside diameter may differ for different portions. For example, for each of the continuous multi-stage distillation columns, the inside diameter of the upper portion of the column may be smaller than, or larger than, the inside diameter of the lower portion of the column.

If $L_1 / D_1$ and $L_2 / D_2$ are less than 2 or greater than 40, then stable operation becomes difficult. In particular, if $L_1 / D_1$ and $L_2 / D_2$ are greater than 40, then the pressure difference between the top and bottom of the column becomes too great, and hence prolonged stable operation becomes difficult. Moreover, it becomes necessary to increase the temperature in the lower portion of the column, and hence side reactions become liable to occur, bringing about a decrease in the selectivity. More preferable ranges for $L_1 / D_1$ and $L_2 / D_2$ are respectively $3 \leq L_1 / D_1 \leq 30$ and $3 \leq L_2 / D_2 < 30$, with $5 \leq L_1 / D_1 \leq 15$ and $5 \leq L_2 / D_2 \leq 15$ being yet more preferable.

If $n_1$ is less than 20, then the conversion decreases and it is not possible to attain the desired production amount for the first continuous multi-stage distillation column. Moreover, to keep down the equipment cost while securing the conversion enabling the desired production amount to be attained, $n_1$ must be made to be not more than 120. Furthermore, if $n_1$ is greater than 120, then the pressure difference between the top and bottom of the column becomes too great, and hence prolonged stable operation of the first continuous multi-stage distillation column becomes difficult. Moreover, it becomes necessary to increase the temperature in the lower portion of the column, and hence side reactions become liable to occur, bringing about a decrease in the selectivity. A more preferable range for $n_1$ is $30 \leq n_1 \leq 100$, with $40 \leq$

$n_1 \leq 90$ being yet more preferable.

Moreover, if $n_2$ is less than 10, then the conversion decreases and it is not possible to attain the desired production amount for the second continuous multi-stage distillation column. Moreover, to keep down the equipment cost while securing the conversion enabling the desired production amount to be attained, $n_2$ must be made to be not more than 80. Furthermore, if $n_2$ is greater than 80, then the pressure difference between the top and bottom of the column becomes too great, and hence prolonged stable operation of the second continuous multi-stage distillation column becomes difficult. Moreover, it becomes necessary to increase the temperature in the lower portion of the column, and hence side reactions become liable to occur, bringing about a decrease in the selectivity. A more preferable range for $n_2$ is $15 \leq n_2 \leq 60$, with $20 \leq n_2 \leq 50$ being yet more preferable.

If $D_1 / d_{11}$ is less than 5, then the equipment cost for the first continuous multi-stage distillation column becomes high. Moreover, large amounts of gaseous components are readily released to the outside of the system, and hence stable operation of the first continuous multi-stage distillation column becomes difficult. If $D_1 / d_{11}$ is greater than 30, then the gaseous component withdrawal amount becomes relatively low, and hence stable operation becomes difficult, and moreover a decrease in the conversion is brought about. A more preferable range for $D_1 / d_{11}$ is $8 \leq D_1 / d_{11} \leq 25$, with $10 \leq D_1 / d_{11} \leq 20$ being yet more preferable. Furthermore, if $D_2 / d_{21}$ is less than 2, then the equipment cost for the second continuous multi-stage distillation column becomes high. Moreover, large amounts of gaseous components are readily released to the outside of the system, and hence stable operation of the second continuous multi-stage distillation column becomes difficult. If $D_2 / d_{21}$ is greater than 15, then the gaseous component withdrawal amount becomes relatively low, and hence stable operation becomes difficult, and moreover a decrease in the conversion is brought about. A more preferable range for $D_2 / d_{21}$ is $5 < D_2 / d_{21} \leq 12$, with $3 \leq D_2 / d_{21} \leq 10$ being yet more preferable.

If $D_1 / d_{12}$ is less than 3, then the equipment cost for the first continuous multi-stage distillation column becomes high. Moreover, the liquid withdrawal amount becomes relatively high, and hence stable operation of the first continuous multi-stage distillation column becomes difficult. If $D_1 / d_{12}$ is greater than 20, then the flow rate through the liquid outlet and piping becomes excessively fast, and hence erosion becomes liable to occur, bringing about corrosion of the apparatus. A more preferable range for $D_1 / d_{12}$ is $5 \leq D_1 / d_{12} \leq 18$, with $7 \leq D_1 / d_{12} \leq 15$ being yet more preferable. Furthermore, if $D_2 / d_{22}$ is less than 5, then the equipment cost for the second continuous multi-stage distillation column becomes high. Moreover, the liquid withdrawal amount becomes relatively high, and hence stable operation of the second continuous multi-stage distillation column becomes difficult. If $D_2 / d_{22}$ is greater than 30, then the flow rate through the liquid outlet and piping becomes excessively fast, and hence erosion becomes liable to occur, bringing about corrosion of the apparatus. A more preferable range for $D_2 / d_{22}$ is $7 \leq D_2 / d_{22} < 25$, with $9 \leq D_2 / d_{22} \leq 20$ being yet more preferable.

Furthermore, it has been found that in step (II) it is further preferable for the $d_{11}$ and the $d_{12}$ to satisfy the following formula (29), and for the $d_{21}$ and the $d_{22}$ to satisfy the following formula (30);

$$1 \leq d_{12} / d_{11} \leq 5 \qquad (29)$$

and

$$1 \leq d_{21} / d_{22} \leq 6 \qquad (30).$$

The term "prolonged stable operation" for step (II) means that operation can be carried out continuously in a steady state based on the operating conditions with no flooding, weeping, clogging of piping, erosion or the like for not less than 1000 hours, preferably not less than 3000 hours, more preferably not less than 5000 hours, and a predetermined amount of the diphenyl carbonate can be produced while maintaining high selectivity.

A characteristic feature for step (II) is that the diphenyl carbonate can be produced stably for a prolonged period of time with high selectivity and with a high productivity of not less than 1 ton / hr, preferably not less than 2 ton / hr, more preferably not less than 3 ton / hr. Moreover, another characteristic feature for step (II) is that in the case that $L_1$, $D_1$, $L_1 / D_1$, $n_1$, $D_1 / d_{11}$, and $D_1 / d_{12}$ for the first continuous multi-stage distillation column satisfy respectively $2000 \leq L_1 \leq 6000$, $150 \leq D_1 \leq 1000$, $3 \leq L_1 / D_1 \leq 30$, $30 \leq n_1 \leq 100$, $8 \leq D_1 / d_{11} \leq 25$, and $5 \leq D_1 / d_{12} \leq 18$, and $L_2$, $D_2$, $L_2 / D_2$, $n_2$, $D_2 / d_{21}$, and $D_2 / d_{22}$ for the second continuous multi-stage distillation column satisfy respectively $2000 \leq L_2 \leq 6000$, $150 \leq D_2 \leq 1000$, $3 \leq L_2 / D_2 \leq 30$, $15 \leq n_2 \leq 60$, $2.5 \leq D_2 / d_{21} \leq 12$, and $7 \leq D_2 / d_{22} \leq 25$, not less than 2 ton / hr, preferably not less than 2.5 ton / hr, more preferably not less than 3 ton / hr of the diphenyl carbonate can be produced.

Furthermore, another characteristic feature for step (II) is that in the case that $L_1$, $D_1$, $L_1 / D_1$, $n_1$, $D_1 / d_{11}$, and $D_1 / d_{12}$ for the first continuous multi-stage distillation column satisfy respectively $2500 \leq L_1 \leq 5000$, $200 \leq D_1 \leq 800$, 5

$\leq L_1 / D_1 \leq 15$, $40 \leq n_1 \leq 90$, $10 \leq D_1 / d_{11} \leq 25$, and $7 \leq D_1 / d_{12} \leq 15$, and $L_2$, $D_2$, $L_2 / D_2$, $n_2$, $D_2 / d_{21}$, and $D_2 / d_{22}$ for the second continuous multi-stage distillation column satisfy respectively $2500 \leq L_2 \leq 5000$, $200 \leq D_2 \leq 800$, $5 \leq L_2 / D_2 \leq 10$, $20 \leq n_2 \leq 50$, $3 \leq D_2 / d_{21} \leq 10$, and $9 \leq D_2 / d_{22} \leq 20$, not less than 3 ton / hr, preferably not less than 3.5 ton / hr, more preferably not less than 4 ton / hr of the diphenyl carbonate can be produced.

The "selectivity" for the diphenyl carbonate in step (II) is based on the phenol reacted. In step (II), a high selectivity of not less than 95% can generally be attained, preferably not less than 97%, more preferably not less than 98%.

Each of the first continuous multi-stage distillation column and the second continuous multi-stage distillation column used in step (II) is preferably a distillation column having trays and/or packings as the internal. The term "internal" used in the present invention means the part in the distillation column where gas and liquid are actually brought into contact with one another. As trays, ones as described in the section on step (I) are preferable. Moreover, the term "number of stages of the internals" has the same meaning as above.

In step (11), in the first continuous multi-stage distillation column, a reaction in which the alkyl phenyl carbonate is produced from the dialkyl carbonate and the phenol mainly occurs. This reaction has an extremely low equilibrium constant, and the reaction rate is slow, and hence it has been discovered that a plate type distillation column having trays as the internal is particularly preferable as the first continuous multi-stage distillation column used in the reactive distillation. Moreover, in the second continuous multi-stage distillation column, it is mainly a disproportionation reaction of the alkyl phenyl carbonate that occurs. This reaction also has a low equilibrium constant, and a slow reaction rate. Even so, it has been discovered that a distillation column having both the packings and the trays as the internal is preferable as the second continuous multi-stage distillation column used in the reactive distillation. Furthermore, it has been discovered that the distillation column having the packings installed in the upper portion thereof and the trays installed in the lower portion thereof is preferable as the second continuous multi-stage distillation column. It has been discovered that the packings in the second continuous multi-stage distillation column are preferably structured packings, and that of structured packings, Mellapak is particularly preferable.

Furthermore, it has been discovered that, as each of the trays installed in the first continuous multi-stage distillation column and the second continuous multi-stage distillation column, a sieve tray having a sieve portion and a downcomer portion is particularly good in terms of the relationship between performance and equipment cost. It has also been discovered that each sieve tray preferably has 100 to 1000 holes / $m^2$ in the sieve portion. A more preferable number of holes is from 120 to 900 holes / $m^2$, yet more preferably from 150 to 800 holes / $m^2$.

Moreover, it has been discovered that the cross-sectional area per hole of each sieve tray is preferably in a range of from 0.5 to 5 $cm^2$. A more preferable cross-sectional area per hole is from 0.7 to 4 $cm^2$, yet more preferably from 0.9 to 3 $cm^2$. Furthermore, it has been discovered that it is particularly preferable if each sieve tray has 100 to 1000 holes / $m^2$ in the sieve portion, and the cross-sectional area per hole is in a range of from 0.5 to 5 $cm^2$. It has been shown that by adding the above conditions to the continuous multi-stage distillation columns, the object of the present invention can be attained more easily.

When carrying out step (II), the diphenyl carbonate is continuously produced by continuously feeding the dialkyl carbonate and the phenol constituting the starting material into the first continuous multi-stage distillation column in which the catalyst is present, carrying out reaction and distillation simultaneously in the first column, continuously withdrawing a first column low boiling point reaction mixture containing a produced alcohol from the upper portion of the first column in a gaseous form, continuously withdrawing a first column high boiling point reaction mixture containing a produced alkyl phenyl carbonate from the lower portion of the first column in a liquid form, continuously feeding the first column high boiling point reaction mixture into the second continuous multi-stage distillation column in which the catalyst is present, carrying out reaction and distillation simultaneously in the second column, continuously withdrawing a second column low boiling point reaction mixture containing a produced dialkyl carbonate from the upper portion of the second column in a gaseous form, continuously withdrawing a second column high boiling point reaction mixture containing a produced diphenyl carbonate from the lower portion of the second column in a liquid form, and continuously feeding the second column low boiling point reaction mixture containing the dialkyl carbonate into the first continuous multi-stage distillation column.

As mentioned earlier, the starting material may contain the alcohol, the alkyl phenyl carbonate and the diphenyl carbonate that are reaction products, and reaction by-products such as an alkyl phenyl ether and high boiling point compounds. Considering the equipment and cost required for separation and purification in other processes, when actually implementing the present invention industrially, it is preferable for the starting material to contain small amounts of such compounds.

In addition to the alcohol produced in the reaction, the column top component from the first continuous multi-stage distillation column in step (II) often also contains the dialkyl carbonate, the phenol, an alkyl aryl ether and so on, and may also contain small amounts of an alkyl aryl carbonate, the diphenyl carbonate and so on. The column top component from the first continuous multi-stage distillation column can be used as aliphatic monohydric alcohol in step (I) as is, but is preferably used as starting material in step (I) after the content of material therein having a higher boiling point than that of the produced alcohol has been reduced through distillation or the like. It is particularly preferable for

a mixture of the alcohol and the dialkyl carbonate to be used as the aliphatic monohydric alcohol in step (I).

In step (II), when continuously feeding the dialkyl carbonate and the phenol constituting the starting material into the first continuous multi-stage distillation column, this starting material may be fed into the first distillation column in a liquid form and / or a gaseous form from inlet(s) provided in one or a plurality of positions in the upper portion or the middle portion of the first distillation column below the gas outlet in the upper portion of the first distillation column. It is also preferable to feed the starting material containing a large proportion of the phenol into the first distillation column in a liquid form from an inlet provided in the upper portion of the first distillation column, and feed the starting material containing a large proportion of the dialkyl carbonate into the first distillation column in a gaseous form from an inlet provided in the lower portion of the first distillation column above the liquid outlet in the lower portion of the first distillation column.

Moreover, in step (II), the first column high boiling point reaction mixture containing the alkyl phenyl carbonate continuously withdrawn from the lower portion of the first continuous multi-stage distillation column is continuously fed into the second continuous multi-stage distillation column. Here, the first column high boiling point reaction mixture is preferably fed into the second distillation column in a liquid form and / or a gaseous form from inlet(s) provided in one or a plurality of positions in the upper portion or the middle portion of the second distillation column below the gas outlet in the upper portion of the second distillation column. Moreover, in the case of using, as the second distillation column, a distillation column having a packing portion in the upper portion thereof and a tray portion in the lower portion thereof, which is a preferable embodiment of the present invention, it is preferable for at least one position where an inlet is provided to be between the packing portion and the tray portion. Moreover, in the case that the packings comprise a plurality of sets of structured packings, it is preferable for an inlet to be installed in a space between the sets of structured packings.

Moreover, in step (II), it is also preferable to carry out a reflux operation of condensing the gaseous component withdrawn from the top of each of the first continuous multi-stage distillation column and the second continuous multi-stage distillation column, and then returning some of this component into the upper portion of that distillation column. In this case, the reflux ratio for the first continuous multi-stage distillation column is in a range of from 0 to 10, and the reflux ratio for the second continuous multi-stage distillation column is in a range of from 0.01 to 10, preferably from 0.08 to 5, more preferably from 0.1 to 2. For the first continuous multi-stage distillation column, not carrying out such a reflux operation (i.e. reflux ratio = 0) is also a preferable embodiment.

In step (II), the method of making the homogeneous catalyst be present in the first continuous multi-stage distillation column may be any method, but it is preferable to feed the catalyst into the first distillation column from a position above the middle portion of the first distillation column. Moreover, it is preferable for catalyst to be fed so that the reaction solution can be brought into contact with the catalyst in a region of at least 7 stages of the first distillation column, preferably at 10 stages, more preferably 15 stages. In this case, a catalyst solution obtained by dissolving the catalyst in the starting material or reaction liquid may be introduced into the column together with the starting material, or may be introduced into the column from a different inlet to the starting material. The amount of the catalyst used in the first continuous multi-stage distillation column in the present invention varies depending on the type of catalyst used, the types and proportions of the starting material compounds, and reaction conditions such as the reaction temperature and the reaction pressure. The amount of the catalyst is generally in a range of from 0.0001 to 30 % by weight, preferably from 0.0005 to 10 % by weight, more preferably from 0.001 to 1 % by weight, based on the total weight of the starting material.

Moreover, in step (II), the method of making the homogeneous catalyst be present in the second continuous multi-stage distillation column may be any method, but it is preferable to feed the catalyst into the second distillation column from a position above the middle portion of the second distillation column. In this case, a catalyst solution obtained by dissolving the catalyst in the starting material or reaction liquid may be introduced into the column together with the starting material, or may be introduced into the column from a different inlet to the starting material. The amount of the catalyst used in the second continuous multi-stage distillation column in the present invention varies depending on the type of catalyst used, the types and proportions of the starting material compounds, and reaction conditions such as the reaction temperature and the reaction pressure. The amount of the catalyst is generally in a range of from 0.0001 to 30 % by weight, preferably from 0.0005 to 10 % by weight, more preferably from 0.001 to 1 % by weight, based on the total weight of the starting material.

In step (II), the catalyst used in the first continuous multi-stage distillation column and the catalyst used in the second continuous multi-stage distillation column may be the same or different, but are preferably the same. More preferably, the same catalyst is used in both columns, this catalyst being one that dissolves in the reaction liquid in both columns. In this case, the catalyst dissolved in the high boiling point reaction mixture in the first continuous multi-stage distillation column is generally withdrawn from the lower portion of the first distillation column together with the alkyl phenyl carbonate and so on, and fed into the second continuous multi-stage distillation column as is; this is a preferable embodiment, If necessary, more of the catalyst can be newly added into the second continuous multi-stage distillation column.

The reaction times for the transesterification reactions carried out in step (II) are considered to equate to the average residence times of the reaction liquids in the first continuous multi-stage distillation column and the second continuous multi-stage distillation column. Each of these reaction times varies depending on the form of the internals in that distillation column and the number of stages, the amount of the starting material fed into the column, the type and amount of the catalyst, the reaction conditions, and so on. The reaction time in each of the first continuous multi-stage distillation column and the second continuous multi-stage distillation column is generally in a range of from 0.01 to 10 hours, preferably from 0.05 to 5 hours, more preferably from 0.1 to 3 hours.

The reaction temperature in the first continuous multi-stage distillation column varies depending on the type of the starting material compounds used, and the type and amount of the catalyst. This reaction temperature is generally in a range of from 100 to 350°C. It is preferable to increase the reaction temperature so as to increase the reaction rate. However, if the reaction temperature is too high, then side reactions become liable to occur, for example production of by-products such as an alkyl phenyl ether increases, which is undesirable. For this reason, the reaction temperature in the first continuous multi-stage distillation column is preferably in a range of from 130 to 280°C, more preferably from 150 to 260°C, yet more preferably from 180 to 250°C.

The reaction temperature in the second continuous multi-stage distillation column varies depending on the type of the starting material compounds used, and the type and amount of the catalyst. This reaction temperature is generally in a range of from 100 to 350°C. It is preferable to increase the reaction temperature so as to increase the reaction rate. However, if the reaction temperature is too high, then side reactions become liable to occur, for example production of by-products such as an alkyl phenyl ether, and Fries rearrangement products of the starting material compounds and the produced alkyl phenyl carbonate and diphenyl carbonate, and derivatives thereof increases, which is undesirable. For this reason, the reaction temperature in the second continuous multi-stage distillation column is preferably in a range of from 130 to 280°C, more preferably from 150 to 260°C, yet more preferably from 180 to 250°C.

Moreover, the reaction pressure in the first continuous multi-stage distillation column varies depending on the type of the starting material compounds used and the composition of the starting material, the reaction temperature, and so on. The first continuous multi-stage distillation column may be at any of a reduced pressure, normal pressure, or an applied pressure. The pressure at the top of the column is generally in a range of from 0.1 to $2\times10^7$ Pa, preferably from $10^5$ to $10^7$ Pa, more preferably from $2\times10^5$ to $5\times10^6$ Pa.

The reaction pressure in the second continuous multi-stage distillation column varies depending on the type of the starting material compounds used and the composition of the starting material, the reaction temperature, and so on. The second continuous multi-stage distillation column may be at any of a reduced pressure, normal pressure, or an applied pressure. The pressure at the top of the column is generally in a range of from 0.1 to $2\times10^7$ Pa, preferably from $10^3$ to $10^6$ Pa, more preferably from $5\times10^3$ to $10^5$ Pa.

Furthermore, as the first continuous multi-stage distillation column in step (II), a plurality of distillation columns may be used. In this case, the distillation columns may be linked together in series, in parallel, or in a combination of series and parallel. Moreover, as the second continuous multi-stage distillation column in step (II), a plurality of distillation columns may be used. In this case, the distillation columns may be linked together in series, in parallel, or in a combination of series and parallel.

The material constituting each of the first continuous multi-stage distillation column and the second continuous multi-stage distillation column used in step (II) is generally a metallic material such as carbon steel or stainless steel. In terms of the quality of the aromatic carbonates to be produced, stainless steel is preferable.

The second column high boiling point reaction mixture continuously withdrawn from the lower portion of the second continuous multi-stage distillation column in a liquid form in step (II) has the diphenyl carbonate as a main component thereof, but generally also contains unreacted alkyl phenyl carbonate, a small amount of unreacted starting material, a small amount of high boiling point by-products and so on, and moreover in the case that a homogeneous catalyst is used, also contains this catalyst component. It is thus necessary to carry out a purification step (III) for obtaining a high-purity diphenyl carbonate from the second column high boiling point reaction mixture. In step (III), two distillation columns (a high boiling point material separating column A, and a diphenyl carbonate purifying column B having a side cut outlet) are used, separation is carried out continuously in the high boiling point material separating column A into a column top component $A_T$ having as a main component thereof the diphenyl carbonate, unreacted alkyl phenyl carbonate, and a small amount of unreacted starting material, and a column bottom component $A_B$ having as a main component thereof a small amount of high boiling point by-products and so on and / or a catalyst component, the column top component $A_T$ from the high boiling point material separating column is continuously fed into the diphenyl carbonate purifying column B, and separation is carried out continuously in the diphenyl carbonate purifying column B into three components, i.e. a column top component $B_T$, a side cut component $B_s$, and a column bottom component $B_B$, the high-purity diphenyl carbonate being obtained as the side cut component $B_s$.

In step (III), the high boiling point material separating column A must be a continuous multi-stage distillation column having a length $L_A$ (cm) and an inside diameter $D_A$ (cm), and having internals with a number of stages $n_A$ thereinside, wherein $L_A$, $D_A$, and $n_A$ satisfy the following formulae (19) to (21);

$$800 \leq L_A \leq 3000 \quad (19)$$

$$100 \leq D_A \leq 1000 \quad (20)$$

and

$$20 \leq n_A \leq 100 \quad (21).$$

The diphenyl carbonate purifying column B must be a continuous multi-stage distillation column having a length $L_B$ (cm) and an inside diameter $D_B$ (cm), having internals thereinside, having an inlet B1 at a middle portion of the column, and a side cut outlet B2 between the inlet B1 and the column bottom, and further having a number of stages $n_{B1}$ of the internals above the inlet B1, a number of stages $n_{B2}$ of the internals between the inlet B1 and the side cut outlet B2, a number of stages $n_{B3}$ of the internals below the side cut outlet B2, and a total number of stages ins (= $n_{B1} + n_{B2} + n_{B3}$), wherein $L_B$, $D_B$, $n_{B1}$, $n_{B2}$, $n_{B3}$, and $n_B$ satisfy the following formulae (22) to (27);

$$1000 \leq L_B \leq 5000 \quad (22)$$

$$100 \leq D_B \leq 1000 \quad (23)$$

$$5 \leq n_{B1} \leq 20 \quad (24)$$

$$12 \leq n_{B2} \leq 40 \quad (25)$$

$$3 \leq n_{B3} \leq 15 \quad (26)$$

and

$$20 \leq n_B \leq 70 \quad (27).$$

It has been discovered that by simultaneously satisfying all of these conditions, a high-purity diphenyl carbonate can be produced on an industrial scale of not less than 1 ton / hr stably for a prolonged period of time, for example not less than 2000 hours, preferably not less than 3000 hours, more preferably not less than 5000 hours, from the diphenyl carbonate-containing second column high boiling point reaction mixture that has been obtained in step (II). The reason why it has become possible to produce a high-purity diphenyl carbonate on an industrial scale with such excellent effects by implementing the process according to the present invention is not clear, but this is supposed to be due to an effect brought about through the combination of the high boiling point material separating column A and the diphenyl carbonate purifying column B satisfying the conditions (19) to (27). Preferable ranges for the respective factors are described below.

It is undesirable for $L_A$ (cm) to be less than 800, since then the height over which internals can be installed in the high boiling point material separating column A becomes limited and hence the separation efficiency decreases. Moreover, to keep down the equipment cost while attaining the desired separation efficiency, $L_A$ must be made to be

not more than 3000. A more preferable range for $L_A$ (cm) is $1000 \leq L_A \leq 2500$, with $1200 \leq L_A \leq 2000$ being yet more preferable.

If $D_A$ (cm) is less than 100, then it is not possible to attain the desired production amount. Moreover, to keep down the equipment cost while attaining the desired production amount, $D_A$ must be made to be not more than 1000. A more preferable range for $D_A$ (cm) is $200 \leq D_A \leq 600$, with $250 \leq D_A \leq 450$ being yet more preferable.

If $n_A$ is less than 20, then the separation efficiency decreases and hence the desired high purity cannot be attained. Moreover, to keep down the equipment cost while attaining the desired separation efficiency, $n_A$ must be made to be not more than 100. Furthermore, if $n_A$ is greater than 100, then the pressure difference between the top and bottom of the column becomes too great, and hence prolonged stable operation of the high boiling point material separating column A becomes difficult. Moreover, it becomes necessary to increase the temperature in the lower portion of the column, and hence side reactions become liable to occur, which is undesirable. A more preferable range for $n_A$ is $30 \leq n_A \leq 70$, with $35 \leq n_A \leq 60$ being yet more preferable.

It is undesirable for $L_B$ (cm) to be less than 1000, since then the height over which internals can be installed in the diphenyl carbonate purifying column B becomes limited and hence the separation efficiency decreases. Moreover, to keep down the equipment cost while attaining the desired separation efficiency, $L_B$ must be made to be not more than 5000. A more preferable range for $L_B$ (cm) is $1500 \leq L_B \leq 3000$, with $1700 \leq L_B \leq 2500$ being yet more preferable.

If $D_B$ (cm) is less than 100, then it is not possible to attain the desired production amount. Moreover, to keep down the equipment cost while attaining the desired production amount, $D_B$ must be made to be not more than 1000. A more preferable range for $D_B$ (cm) is $150 \leq D_B \leq 500$, with $200 \leq D_B \leq 400$ being yet more preferable.

If $n_B$ is less than 20, then the separation efficiency for the column as a whole decreases and hence the desired high purity cannot be attained. Moreover, to keep down the equipment cost while attaining the desired separation efficiency, $n_B$ must be made to be not more than 70. Furthermore, if $n_B$ is greater than 70, then the pressure difference between the top and bottom of the column becomes too great, and hence prolonged stable operation of the diphenyl carbonate purifying column B becomes difficult. Moreover, it becomes necessary to increase the temperature in the lower portion of the column, and hence side reactions become liable to occur, which is undesirable. A more preferable range for $n_B$ is $25 \leq n_B \leq 55$, with $30 \leq n_B \leq 50$ being yet more preferable. Furthermore, it has been ascertained that to obtain the desired high-purity diphenyl carbonate stably for a prolonged period of time, $n_{B1}$, $n_{B2}$, and $n_{B3}$ must be in the ranges $5 \leq n_{B1} \leq 20$, $12 \leq n_{B2} \leq 40$, and $3 \leq n_{B3} \leq 15$ respectively. More preferable ranges are $7 \leq n_{B1} \leq 15$, $12 \leq n_{B2} \leq 30$, and $3 \leq n_{B3} \leq 10$.

Moreover, when carrying out the present invention, it is preferable for the high boiling point material separating column A to be operated at a column bottom temperature $T_A$ in a range of from 185 to 280°C, and a column top pressure $P_A$ in a range of from 1000 to 20000 Pa, and for the diphenyl carbonate purifying column B to be operated at a column bottom temperature $T_B$ in a range of from 185 to 280°C, and a column top pressure $P_B$ in a range of from 1000 to 20000 Pa.

It is undesirable for $T_A$ to be less than 185°C, since then the column top pressure must be reduced, and hence equipment for maintaining a high vacuum must be used, and moreover the equipment increases in size. Moreover, it is undesirable for $T_A$ to be greater than 280°C, since then high boiling point by-products are produced during the distillation. A more preferable range for $T_A$ is from 190 to 240°C, with from 195 to 230°C being yet more preferable.

It is undesirable for $P_A$ to be less than 1000 Pa, since then large equipment enabling a high vacuum to be maintained must be used. Moreover, it is undesirable for $P_A$ to be greater than 20000 Pa, since then the distillation temperature must be increased and hence production of by-products increases. A more preferable range for $P_A$ is from 2000 to 15000 Pa, with from 3000 to 13000 Pa being yet more preferable.

It is undesirable for $T_B$ to be less than 185°C, since then the column top pressure must be reduced, and hence equipment for maintaining a high vacuum must be used, and moreover the equipment increases in size. Moreover, it is undesirable for $T_B$ to be greater than 280°C, since then high boiling point by-products are produced during the distillation. A more preferable range for $T_B$ is from 190 to 240°C, with from 195 to 230°C being yet more preferable.

It is undesirable for $P_B$ to be less than 1000 Pa, since then large equipment enabling a high vacuum to be maintained must be used. Moreover, it is undesirable for $P_B$ to be greater than 20000 Pa, since then the distillation temperature must be increased and hence production of by-products increases. A more preferable range for $P_B$ is from 2000 to 15000 Pa, with from 3000 to 13000 Pa being yet more preferable.

For the high boiling point material separating column A and the diphenyl carbonate purifying column B, so long as $D_A$ and $D_B$ are within the above ranges, each of the columns may have the same inside diameter from the upper portion thereof to the lower portion thereof, or the inside diameter may differ in different portions. For example, for each of the continuous multi-stage distillation columns, the inside diameter of the upper portion of the column may be smaller than, or larger than, the inside diameter of the lower portion of the column.

Each of the high boiling point material separating column A and the diphenyl carbonate purifying column B used in step (III) is a distillation column having trays and/or packings as the internal. The term "internal" used in the present invention means the part in the distillation column where gas and liquid are actually brought into contact with one another. As trays, ones as described in the section on step (I) are preferable. Moreover, the term "number of stages

of the internals" has the same meaning as above.

It has been ascertained that for step (III), the high boiling point material separating column A preferably has packings as the internal, and furthermore structured packings are preferable as these packings: Moreover, it has been discovered that the diphenyl carbonate purifying column B preferably has packings as the internal, particularly preferably one set or a plurality of sets of the structured packings.

The high boiling point reaction mixture continuously withdrawn from the bottom of the second reactive distillation column of step (II) generally contains 0.05 to 2 % by weight of the dialkyl carbonate, 1 to 20 % by weight of the phenol, 0.05 to 2 % by weight of an alkyl phenyl ether, 10 to 45 % by weight of the alkyl phenyl carbonate, 50 to 80 % by weight of the diphenyl carbonate, 0.1 to 5 % by weight of high boiling point by-products, and 0.001 to 5 % by weight of the catalyst, and hence this continuously withdrawn column bottom liquid is preferably continuously fed as is into the high boiling point material separating column A of step (III).

The composition of the reaction mixture varies depending on the conditions of the transesterification between the dialkyl carbonate and the phenol, the type and amount of the catalyst, and so on, but so long as the transesterification is carried out under fixed conditions, a reaction mixture of approximately fixed composition can be produced, and hence the composition of the reaction mixture fed into the high boiling point material separating column A is approximately fixed. Nevertheless, in step (III), so long as the composition of the reaction mixture is within the above range, then even if this composition fluctuates somewhat, the separation can still be carried out with approximately the same separation efficiency. This is one of the characteristic features of step (III) of the present invention.

In step (III), when continuously feeding the column bottom liquid from the second reactive distillation column of step (II) into the high boiling point material separating column A, this column bottom liquid may be fed in as a liquid from inlet(s) provided in one or a plurality of positions below a middle portion of the separating column A, or it is also preferable to feed the column bottom liquid from the second reactive distillation column into the separating column A via a reboiler of the separating column A from piping provided at a lower portion of the reboiler. The amount of the column bottom liquid from the second reactive distillation column fed into the high boiling point material separating column A varies depending on the amount of the high-purity diphenyl carbonate to be produced, the concentration of the diphenyl carbonate in the reaction mixture, the separation conditions for the separating column A, and so on, but is generally not less than approximately 2 ton / hr, preferably not less than approximately 6 ton / hr, more preferably not less than approximately 10 ton / hr.

The high boiling point reaction mixture from the second reactive distillation column fed continuously into the high boiling point material separating column A is separated into a column top component (AT) containing most of the diphenyl carbonate and most of compounds having a lower boiling point than that of the diphenyl carbonate such as unreacted starting material, an alkyl phenyl ether and the alkyl phenyl carbonate, and a column bottom component ($A_B$) containing the catalyst, high boiling point by-products and a small amount of the diphenyl carbonate. The column bottom component ($A_B$) may contain a small amount of the alkyl phenyl carbonate. Such organic material in the column bottom component plays a useful role in dissolving the catalyst component and thus maintaining a liquid state. All or some of the column bottom component ($A_B$) is generally reused by being circulated back into the first reactive distillation column and / or the second reactive distillation column of step (II) as is as a transesterification reaction catalyst component, but in some cases the catalyst may be recycled after being separated from the organic material in a catalyst recovery process, and then reused by being circulated back into the first reactive distillation column and / or the second reactive distillation column of step (II).

It is a characteristic feature of step (III) that in step (III) the catalyst component and by-products having a higher boiling point than that of the diphenyl carbonate such as phenyl salicylate, xanthone, phenyl methoxybenzoate and 1-phenoxycarbonyl-2-phenoxycarboxy-phenylene are almost completely separated off as the column bottom component ($A_B$) in the high boiling point material separating column A, it being easy to make the content thereof in the column top component (AT) be generally not more than 200 ppm, preferably not more than 100 ppm, more preferably not more than 50 ppm. It is another characteristic feature of step (III) that despite making the column top component (AT) hardly contain any such high boiling point by-products, most of the diphenyl carbonate in the reaction mixture introduced into the high boiling point material separating column A can be withdrawn from the top of the column. In step (III), not less than 95%, preferably not less than 96%, more preferably not less than 98%, of the diphenyl carbonate in the reaction mixture continuously fed into the high boiling point material separating column A can be withdrawn from the top of the column. Moreover, in step (III), although dependent on the composition of the high boiling point reaction mixture from the second reactive distillation column fed into the separating column A, in general 90 to 97 % by weight of the liquid continuously fed in is continuously withdrawn from the top of the column as the column top component (AT), with 10 to 3 % by weight being continuously withdrawn from the bottom of the column as the column bottom component ($A_B$). The composition of the column top component (AT) is generally from 0.05 to 1 % by weight of the dialkyl carbonate, 1 to 10 % by weight of the phenol, 0.05 to 0.5 % by weight of an alkyl phenyl ether, 20 to 40 % by weight of the alkyl phenyl carbonate, and 50 to 80 % by weight of the diphenyl carbonate; the content of high boiling point by-products is generally not more than 200 ppm, preferably not more than 100 ppm, more preferably not more than 50 ppm.

In step (III), a reflux ratio for the high boiling point material separating column A is in a range of from 0.01 to 10, preferably from 0.08 to 5, more preferably from 0.1 to 3.

As stated above, the amount of the column top component (AT) continuously withdrawn from the top of the high boiling point material separating column A is generally approximately from 90 to 97% of the high boiling point reaction mixture from the second reactive distillation column fed into the separating column A. This column top component (AT) is continuously fed as is into the diphenyl carbonate purifying column B from the inlet B1 provided at a middle portion of the purifying column B, and is continuously separated into three components, i.e. a column top component ($B_T$), a side cut component ($B_s$), and a column bottom component ($B_B$). All of components having a lower boiling point than that of the diphenyl carbonate contained in the column top component (AT) from the separating column A fed into the purifying column B are continuously withdrawn from the top of the purifying column B as the column top component ($B_T$), and a small amount of liquid is continuously withdrawn from the bottom of the purifying column B. A small amount of the diphenyl carbonate is contained in the column top component ($B_T$), this amount generally being from 1 to 9%, preferably from 3 to 8%, of the diphenyl carbonate fed in. The diphenyl carbonate in the column top component ($B_T$) is separated out and thus recovered using another distillation column for separating the column top component ($B_T$). Alternatively, a method in which this diphenyl carbonate is separated off as the column bottom component from this other distillation column, and is then recovered by being returned into the high boiling point material separating column A and / or the diphenyl carbonate purifying column B is also preferable.

The column bottom component ($B_B$) contains the diphenyl carbonate, and a small amount of high boiling point by-products concentrated to approximately a few percent. Another characteristic feature of the present invention is that the amount of the diphenyl carbonate in the column bottom component ($B_B$) withdrawn from the bottom of the purifying column B can be kept very low. This amount is generally from 0.05 to 0.5% of the diphenyl carbonate fed in.

The high-purity diphenyl carbonate is continuously withdrawn from the side cut outlet B2 at a flow rate of generally not less than 1 ton / hr, preferably not less than 3 ton / hr, more preferably not less than 5 ton / hr; this amount generally corresponds to approximately 90 to 96% of the diphenyl carbonate fed into the purifying column B.

The purity of the diphenyl carbonate obtained as the side cut component (Bs) in step (III) is generally not less than 99.9%, preferably not less than 99.99%, more preferably not less than 99.999%. The contents of high boiling point impurities in the high-purity diphenyl carbonate obtained when carrying out step (II) and step (III) with dimethyl carbonate and phenol as the starting material are not more than 30 ppm, preferably not more than 10 ppm, more preferably not more than 1 ppm for phenyl salicylate, not more than 30 ppm, preferably not more than 10 ppm, more preferably not more than 1 ppm for xanthone, not more than 30 ppm, preferably not more than 10 ppm, more preferably not more than 1 ppm for phenyl methoxybenzoate, and not more than 30 ppm, preferably not more than 10 ppm, more preferably not more than 5 ppm for 1-phenoxycarbonyl-2-phenoxycarboxy-phenylene. Moreover, the total content of these high boiling point by-products is not more than 100 ppm, preferably not more than 50 ppm, more preferably not more than 10 ppm.

Moreover, in the present invention, starting material and catalyst not containing a halogen are generally used, and hence the halogen content of the diphenyl carbonate obtained is not more than 0.1 ppm, preferably not more than 10 ppb, more preferably not more than 1 ppb (outside the detection limit for the ion chromatography).

In step (III), the reflux ratio for the diphenyl carbonate purifying column B is in a range of from 0.01 to 10, preferably from 0.1 to 8, more preferably from 0.5 to 5.

The material constituting the high boiling point material separating column A, the diphenyl carbonate purifying column B, and other liquid-contacting parts used in the present invention is generally a metallic material such as carbon steel or stainless steel. In terms of the quality of the diphenyl carbonate to be produced, stainless steel is preferable.

EXAMPLES

Following is a more detailed description of the present invention through Examples. However, the present invention is not limited to the following Examples.

The halogen content was measured using an ion chromatography method.

Example 1:

(1) Step (I) of continuously producing dimethyl carbonate and ethylene glycol

<Continuous multi-stage distillation column $T_0$>

A continuous multi-stage distillation column as shown in FIG. 1 having $L_0$ = 3300 cm, $D_0$ = 300 cm, $L_0$ / $D_0$ = 11, $n_0$ = 60, $D_0$ / $d_{01}$ = 7.5, and $D_0$ / $d_{02}$ = 12 was used. In this example, as the internal, sieve trays each having a cross-sectional area per hole in the sieve portion thereof of approximately 1.3 $cm^2$ and a number of holes of approximately 180 to 320 / $m^2$ were used.

<Reactive distillation>

3.27 Ton / hr of ethylene carbonate in a liquid form was continuously introduced into the distillation column To from an inlet (3-a) provided at the $55^{th}$ stage from the bottom. 3.238 Ton / hr of methanol in a gaseous form (containing 8.96 % by weight of dimethyl carbonate) and 7.489 ton / hr of methanol in a liquid form (containing 6.66 % by weight of dimethyl carbonate) were respectively continuously introduced into the distillation column To from inlets (3-b and 3-c) provided at the $31^{st}$ stage from the bottom. The molar ratio of the starting materials introduced into the distillation column To was methanol / ethylene carbonate = 8.36.

The catalyst used was obtained by adding 4.8 ton of ethylene glycol to 2.5 ton of KOH (48 % by weight aqueous solution), heating to approximately 130 °C, gradually reducing the pressure, and carrying out heat treatment for approximately 3 hours at approximately 1300 Pa, so as to produce a homogeneous solution. This catalyst solution was continuously introduced into the distillation column To from an inlet (3-e) provided at the $54^{th}$ stage from the bottom (K concentration: 0.1 % by weight based on ethylene carbonate fed in). Reactive distillation was carried out continuously under conditions of a column bottom temperature of 98 °C, a column top pressure of approximately $1.118 \times 10^5$ Pa, and a reflux ratio of 0.42.

It was possible to attain stable steady state operation after 24 hours. A low boiling point reaction mixture withdrawn from the top 1 of the column in a gaseous form was cooled using a heat exchanger and thus turned into a liquid. The liquid low boiling point reaction mixture, which was continuously withdrawn from the distillation column at 10.678 ton / hr, contained 4.129 ton / hr of dimethyl carbonate, and 6.549 ton / hr of methanol. A liquid continuously withdrawn from the bottom 2 of the column at 3.382 ton / hr contained 2.356 ton / hr of ethylene glycol, 1.014 ton / hr of methanol, and 4 kg / hr of unreacted ethylene carbonate. Excluding the dimethyl carbonate contained in the starting material, the actual produced amount of dimethyl carbonate was 3.340 ton / hr, and excluding the ethylene glycol contained in the catalyst solution, the actual produced amount of ethylene glycol was 2.301 ton / hr. The ethylene carbonate conversion was 99.88%, the dimethyl carbonate selectivity was not less than 99.99%, and the ethylene glycol selectivity was not less than 99.99%.

Prolonged continuous operation was carried out under these conditions. After 500 hours, 2000 hours, 4000 hours, 5000 hours, and 6000 hours, the actual produced amounts per hour were 3.340 ton, 3.340 ton, 3.340 ton, 3.340 ton, and 3.340 ton respectively for dimethyl carbonate, and 2.301 ton, 2.301 ton, 2.301 ton, 2.301 ton, and 2.301 ton respectively for ethylene glycol, the ethylene carbonate conversions were respectively 99.90%, 99.89%, 99.89%, 99.88%, and 99.88%, the dimethyl carbonate selectivities were respectively not less than 99.99%, not less than 99.99%, not less than 99.99%, not less than 99.99%, and not less than 99.99%, and the ethylene glycol selectivities were respectively not less than 99.99%, not less than 99.99%, not less than 99.99%, not less than 99.99%, and not less than 99.99%.

(2) Step (II) of continuously producing diphenyl carbonate

<First continuous multi-stage distillation column 101 >

A continuous multi-stage distillation column as shown in FIG. 2 having $L_1$ = 3300 cm, $D_1$ = 500 cm, $L_1 / D_1$ = 6.6, $n_1$ = 80, $D_1 / d_{11}$ = 17, and $D_1 / d_{12}$ = 9 was used. In this Example, sieve trays each having a cross-sectional area per hole of approximately 1.5 $cm^2$ and a number of holes of approximately 250 / $m^2$ were used as the internal.

<Second continuous multi-stage distillation column 201 >

A continuous multi-stage distillation column as shown in FIG. 3 having $L_2$ = 3100 cm, $D_2$ = 500 cm, $L_2 / D_2$ = 6.2, $n_2$ = 30, $D_2 / d_{21}$ = 3.85, and $D_2 / d_{22}$ = 11.1 was used. In this Example, as the internal, two sets of Mellapak (11 theoretical stages in total) were installed in the upper portion, and sieve trays each having a cross-sectional area per hole of approximately 1.3 $cm^2$ and a number of holes of approximately 250 / $m^2$ were used in the lower portion.

<Reactive distillation>

Diphenyl carbonate was produced by carrying out reactive distillation using an apparatus in which the first continuous multi-stage distillation column 101 and the second continuous multi-stage distillation column 201 were connected together as shown in FIG. 4.

A starting material 1 containing phenol and dimethyl carbonate in a weight ratio of phenol / dimethyl carbonate = 1.9 was introduced continuously in a liquid form at a flow rate of 50 ton / hr from an upper inlet 11 of the first continuous multi-stage distillation column 101. On the other hand, a starting material 2 containing dimethyl carbonate and phenol in a weight ratio of dimethyl carbonate / phenol = 3.6 was introduced continuously in a gaseous form at a flow rate of 50 ton / hr from a lower inlet 12 of the first continuous multi-stage distillation column 101. The molar ratio for the starting

materials introduced into the first continuous multi-stage distillation column 101 was dimethyl carbonate / phenol = 1.35. The starting materials substantially did not contain halogens (outside the detection limit of the ion chromatography, i.e. not more than 1 ppb). $Pb(OPh)_2$ as a catalyst was introduced from the upper inlet 11 of the first continuous multi-stage distillation column 101 such that a concentration thereof in the reaction liquid would be approximately 100 ppm. Reactive distillation was carried out continuously under conditions of a temperature at the bottom of the first continuous multi-stage distillation column 101 of 225 °C, a pressure at the top of the column of $7x10^5$ Pa and a reflux ratio of 0. A first column low boiling point reaction mixture containing methanol, dimethyl carbonate, phenol and so on was continuously withdrawn in a gaseous form from the top 13 of the first column, was passed through a heat exchanger 14, and was withdrawn at a flow rate of 34 ton / hr from an outlet 16. On the other hand, a first column high boiling point reaction mixture containing methyl phenyl carbonate, dimethyl carbonate, phenol, diphenyl carbonate, the catalyst and so on was continuously withdrawn in a liquid form from 21 via the bottom 17 of the first column.

A stable steady state was attained after 24 hours. The first column high boiling point reaction mixture was thus then fed continuously as is into the second continuous multi-stage distillation column 201 at a flow rate of 66 ton / hr from a starting material inlet 21 installed between the Mellapak and the sieve trays. The liquid fed into the second continuous multi-stage distillation column 201 contained 18.2 % by weight of methyl phenyl carbonate and 0.8 % by weight of diphenyl carbonate. Reactive distillation was carried out continuously in the second continuous multi-stage distillation column 201 under conditions of a temperature at the bottom of the column of 210°C, a pressure at the top of the column of $3x10^4$ Pa, and a reflux ratio of 0.3. It was possible to attain stable steady state operation after 24 hours. A second column low boiling point reaction mixture containing 35 % by weight of dimethyl carbonate and 56 % by weight of phenol was continuously withdrawn from the top 23 of the second column, the flow rate at an outlet 26 being 55.6 ton / hr, and a second column high boiling point reaction mixture containing 38.4 % by weight of methyl phenyl carbonate and 55.6 % by weight of diphenyl carbonate was continuously withdrawn from the bottom 27 of the second column. The second column low boiling point reaction mixture was continuously fed into the first continuous multi-stage distillation column 101 from the inlet 11. At this time, the amounts of fresh dimethyl carbonate and phenol newly fed into the first continuous multi-stage distillation column 101 were adjusted so as to maintain the above compositions and amounts of the starting material 1 and the starting material 2, taking into consideration the composition and amount of the second column low boiling point reaction mixture. It was found that the produced amount of diphenyl carbonate per hour was 5.74 ton. The selectivity for the diphenyl carbonate based on the phenol reacted was 98%.

Prolonged continuous operation was carried out under these conditions. The produced amounts of diphenyl carbonate per hour after 500 hours, 2000 hours, 4000 hours, 5000 hours, and 6000 hours (excluding the diphenyl carbonate contained in the starting material) were 5.74 ton, 5.75 ton, 5.74 ton, 5.74 ton, and 5.75 ton respectively, and the selectivities were 98%, 98%, 98%, 98%, and 98% respectively, and hence the operation was very stable. Moreover, the aromatic carbonates produced substantially did not contain halogens (not more than 1 ppb).

(3) Step (III) of obtaining high-purity diphenyl carbonate

<High boiling point material separating column A>

A continuous multi-stage distillation column as shown in FIG. 5 having $L_A$ = 1700 cm and $D_A$ = 340 cm, and having Mellapak with $n_A$ = 30-installed therein as the internal was used as the separating column A.

<Diphenyl carbonate purifying column B>

A continuous multi-stage distillation column as shown in FIG. 5 having $L_B$ = 2200 cm and $D_B$ = 280 cm, and having three sets of Mellapak with $n_{B1}$ = 12, $n_{B2}$ = 18, and $n_{B3}$ = 5 installed therein as the internal was used as the purifying column B.

Using an apparatus comprising the high boiling point material separating column A and the diphenyl carbonate purifying column B as shown in FIG. 5, the high boiling point reaction mixture from the second reactive distillation column obtained in step (II) above was continuously introduced at 13.1 ton / hr into the separating column A from an inlet A1. The column bottom temperature ($T_A$) was made to be 206°C and the column top pressure ($P_A$) was made to be 3800 Pa in the separating column A, distillation was carried out continuously with a reflux ratio of 0.6, a column top component (AT) was continuously withdrawn at 12.5 ton / hr via a conduit 16, and a column bottom component ($A_B$) was continuously withdrawn at 0.6 ton / hr via a conduit 11. The column top component ($A_T$) was continuously introduced as is into the purifying column B from the inlet B1. The column bottom temperature ($T_B$) was made to be 213°C and the column top pressure ($P_B$) was made to be 5000 Pa in the purifying column B, distillation was carried out continuously with a reflux ratio of 1.5, a column top component ($B_T$) was continuously withdrawn at 5.3 ton / hr via a conduit 26, a column bottom component ($B_B$) was continuously withdrawn at 0.03 ton / hr via a conduit 31, and a side cut component (Bs) was continuously withdrawn at 7.17 ton / hr via a conduit 33.

The compositions of the components 24 hours after the system had become completely stable were as follows.
$A_T$: 6.8 % by weight of material having a lower boiling point than that of methyl phenyl carbonate (0.1 % by weight of dimethyl carbonate, 0.1 % by weight of anisole, 6.6 % by weight of phenol), 33.8 % by weight of methyl phenyl carbonate, 59.4 % by weight of diphenyl carbonate
$A_B$: 41.0 % by weight of diphenyl carbonate, 59.0 % by weight of high boiling point material including a catalyst component and by-products having a higher boiling point than that of diphenyl carbonate such as phenyl salicylate, xanthone, phenyl methoxybenzoate and 1-phenoxycarbonyl-2-phenoxycarboxy-phenylene
$B_T$: 0.25 % by weight of dimethyl carbonate, 0.25 % by weight of anisole, 15.6 % by weight of phenol, 79.6 % by weight of methyl phenyl) carbonate, 4.3 % by weight of diphenyl carbonate
$B_B$: 95.0 % by weight of diphenyl carbonate, 5.0 % by weight of high boiling point material
The content of each of phenyl salicylate, xanthone and phenyl methoxybenzoate in the side cut component was not more than 1 ppm, and the content of 1-phenoxycarbonyl-2-phenoxycarboxy-phenylene was 4 ppm. Moreover, the halogen content was not more than 1 ppb. It was thus found that the purity of the diphenyl carbonate obtained from the side cut was not less than 99.999%. Moreover, the produced amount of this high-purity diphenyl carbonate was 7.17 ton / hr.
Prolonged continuous operation was carried out under these conditions. The produced amount of diphenyl carbonate and the purity were substantially unchanged after 500 hours, 2000 hours, 4000 hours, 5000 hours, and 6000 hours.

Example 2:

(1) Step (I) of continuously producing dimethyl carbonate and ethylene glycol

Reactive distillation was carried out under the following conditions using the same continuous multi-stage distillation column as in Example 1.
2.61 Ton I hr of ethylene carbonate in a liquid form was continuously introduced into the distillation column from the inlet (3-a) provided at the 55th stage from the bottom. 4.233 Ton / hr of methanol in a gaseous form (containing 2.41 % by weight of dimethyl carbonate) and 4.227 ton / hr of methanol in a liquid form (containing 1.46 % by weight of dimethyl carbonate) were respectively continuously introduced into the distillation column from the inlets (3-b and 3-c) provided at the 31st stage from the bottom. The molar ratio of the starting materials introduced into the distillation column was methanol / ethylene carbonate = 8.73. The catalyst was made to be the same as in Example 1, and was continuously fed into the distillation column. Reactive distillation was carried out continuously under conditions of a column bottom temperature of 93 °C, a column top pressure of approximately $1.046 \times 10^5$ Pa, and a reflux ratio of 0.48.
It was possible to attain stable steady state operation after 24 hours. A low boiling point reaction mixture withdrawn from the top 1 of the column in a gaseous form was cooled using a heat exchanger and thus turned into a liquid. The liquid low boiling point reaction mixture, which was continuously withdrawn from the distillation column at 8.17 ton / hr, contained 2.84 ton / hr of dimethyl carbonate, and 5.33 ton / hr of methanol. A liquid continuously withdrawn from the bottom 2 of the column at 2.937 ton / hr contained 1.865 ton / hr of ethylene glycol, 1.062 ton / hr of methanol, and 0.2 kg / hr of unreacted ethylene carbonate. Excluding the dimethyl carbonate contained in the starting material, the actual produced amount of dimethyl carbonate was 2.669 ton / hr, and excluding the ethylene glycol contained in the catalyst solution, the actual amount produced of ethylene glycol was 1.839 ton / hr. The ethylene carbonate conversion was 99.99%, the dimethyl carbonate selectivity was not less than 99.99%, and the ethylene glycol selectivity was not less than 99.99%.
Prolonged continuous operation was carried out under these conditions. After 1000 hours, 2000 hours, 3000 hours, and 5000 hours, the actual produced amounts per hour were 2.669 ton, 2.669 ton, 2.669 ton, and 2.669 ton respectively for dimethyl carbonate, and 1.839 ton, 1.839 ton, 1.839 ton, and 1.839 ton respectively for ethylene glycol, the ethylene carbonate conversions were respectively 99.99%, 99.99%, 99.99%, and 99.99%, the dimethyl carbonate selectivities were respectively not less than 99.99%, not less than 99.99%, not less than 99.99%, and not less than 99.99%, and the ethylene glycol selectivities were respectively not less than 99.99%, not less than 99.99%, not less than 99.99%, and not less than 99.99%.

(2) Step (II) of continuously producing diphenyl carbonate

Reactive distillation was carried out under the following conditions using the same apparatus as in Example 1.
A starting material 1 containing phenol and dimethyl carbonate in a weight ratio of phenol / dimethyl carbonate = 1.1 was introduced continuously in a liquid form at a flow rate of 40 ton / hr from the upper inlet 11 of the first continuous multi-stage distillation column 101. On the other hand, a starting material 2 containing dimethyl carbonate and phenol in a weight ratio of dimethyl carbonate / phenol = 3.9 was introduced continuously in a gaseous form at a flow rate of

43 ton / hr from the lower inlet 12 of the first continuous multi-stage distillation column 101. The molar ratio for the starting materials introduced into the first continuous multi-stage distillation column 101 was dimethyl carbonate / phenol = 1.87. The starting materials substantially did not contain halogens (outside the detection limit of the ion chromatography, i.e. not more than 1 ppb). $Pb(OPh)_2$ as a catalyst was introduced from the upper inlet 11 of the first continuous multi-stage distillation column 101 such that a concentration thereof in the reaction liquid would be approximately 250 ppm. Reactive distillation was carried out continuously in the first continuous multi-stage distillation column 101 under conditions of a temperature at the bottom of the column of 235°C, a pressure at the top of the column of $9\times10^5$ Pa and a reflux ratio of 0. A first column low boiling point reaction mixture containing methanol, dimethyl carbonate, phenol and so on was continuously withdrawn in a gaseous form from the top 13 of the first column, was passed through the heat exchanger 14, and was withdrawn at a flow rate of 43 ton / hr from the outlet 16. On the other hand, a first column high boiling point reaction mixture containing methyl phenyl carbonate, dimethyl carbonate, phenol, diphenyl carbonate, the catalyst and so on was continuously withdrawn in a liquid form from 21 via the bottom 17 of the first column.

A stable steady state was attained after 24 hours. The first column high boiling point reaction mixture was thus then fed continuously as is into the second continuous multi-stage distillation column 201 at a flow rate of 40 ton / hr from the starting material inlet 21 installed between the Mellapak and the sieve trays. The liquid fed into the second continuous multi-stage distillation column 201 contained 20.7 % by weight of methyl phenyl carbonate and 1.0 % by weight of diphenyl carbonate. Reactive distillation was carried out continuously in the second continuous multi-stage distillation column 201 under conditions of a temperature at the bottom of the column of 205°C, a pressure at the top of the column of $2\times10^4$ Pa, and a reflux ratio of 0.5. It was possible to attain stable steady state operation after 24 hours. A second column low boiling point reaction mixture was continuously withdrawn from the top 23 of the second column, and a second column high boiling point reaction mixture containing 36.2 % by weight of methyl phenyl carbonate and 60.8 % by weight of diphenyl carbonate was continuously withdrawn from the bottom 27 of the second column. The second column low boiling point reaction mixture was continuously fed into the first continuous multi-stage distillation column 101 from the inlet 11. At this time, the amounts of fresh dimethyl carbonate and phenol newly fed into the first continuous multi-stage distillation column 101 were adjusted so as to maintain the above compositions and amounts of the starting material 1 and the starting material 2, taking into consideration the composition and amount of the second column low boiling point reaction mixture. It was found that the produced amount of diphenyl carbonate per hour was 4.03 ton. The selectivity for the diphenyl carbonate based on the phenol reacted was 97%.

Prolonged continuous operation was carried out under these conditions. The produced amounts of diphenyl carbonate per hour after 500 hours, 1000 hours, and 2000 hours were 4.03 ton, 4.03 ton, and 4.04 ton respectively, and the selectivities based on the reacted phenol were 97%, 97%, and 97% respectively, and hence the operation was very stable. Moreover, the diphenyl carbonate produced substantially did not contain halogens (not more than 1 ppb).

(3) Step (III) of obtaining high-purity diphenyl carbonate

This was carried out using the same process as in Example 1.

Example 3:

(1) Step (I) of continuously producing dimethyl carbonate and ethylene glycol

A continuous multi-stage distillation column as shown in FIG. 1 having $L_0$ = 3300 cm, $D_0$ = 300 cm, $L_0$ / $D_0$ = 11, $n_0$ = 60, $D_0$ / $d_{01}$ = 7.5, and $D_0$ / $d_{02}$ = 12 was used. In this example, as the internal, sieve trays each having a cross-sectional area per hole in the sieve portion thereof of approximately 1.3 $cm^2$ and a number of holes of approximately 220 to 340 / $m^2$ were used.

3.773 Ton / hr of ethylene carbonate in a liquid form was continuously introduced into the distillation column from the inlet (3-a) provided at the 55$^{th}$ stage from the bottom. 3.736 Ton / hr of methanol in a gaseous form (containing 8.97 % by weight of dimethyl carbonate) and 8.641 ton / hr of methanol in a liquid form (containing 6.65 % by weight of dimethyl carbonate) were respectively continuously introduced into the distillation column from the inlets (3-b and 3-c) provided at the 31$^{st}$ stage from the bottom. The molar ratio of the starting materials introduced into the distillation column was methanol / ethylene carbonate = 8.73. The catalyst was made to be the same as in Example 1, and was continuously fed into the distillation column. Reactive distillation was carried out continuously under conditions of a column bottom temperature of 98 °C, a column top pressure of approximately $1.118\times10^5$ Pa, and a reflux ratio of 0.42.

It was possible to attain stable steady state operation after 24 hours. A low boiling point reaction mixture withdrawn from the top of the column in a gaseous form was cooled using a heat exchanger and thus turned into a liquid. The liquid low boiling point reaction mixture, which was continuously withdrawn from the distillation column at 12.32 ton / hr, contained 4.764 ton / hr of dimethyl carbonate, and 7.556 ton / hr of methanol. A liquid continuously withdrawn from the bottom of the column at 3.902 ton / hr contained 2.718 ton / hr of ethylene glycol, 1.17 ton / hr of methanol, and 4.6

kg / hr of unreacted ethylene carbonate. Excluding the dimethyl carbonate contained in the starting material, the actual produced amount of dimethyl carbonate was 3.854 ton / hr, and excluding the ethylene glycol contained in the catalyst solution, the actual produced amount of ethylene glycol was 2.655 ton / hr. The ethylene carbonate conversion was 99.88%, the dimethyl carbonate selectivity was not less than 99.99%, and the ethylene glycol selectivity was not less than 99.99%.

Prolonged continuous operation was carried out under these conditions. After 1000 hours, 2000 hours, 3000 hours, and 5000 hours, the actual produced amounts per hour were 3.854 ton, 3.854 ton, 3.854 ton, and 3.854 ton respectively for dimethyl carbonate, and 2.655 ton, 2.655 ton, 2.655 ton, and 2.655 ton respectively for ethylene glycol, the ethylene carbonate conversions were respectively 99.99%, 99.99%, 99.99%, and 99.99%, the dimethyl carbonate selectivities were respectively not less than 99.99%, not less than 99.99%, not less than 99.99%, and not less than 99.99%, and the ethylene glycol selectivities were respectively not less than 99.99%, not less than 99.99%, not less than 99.99%, and not less than 99.99%.

(2) Step (II) of continuously producing diphenyl carbonate

Reactive distillation was carried out under the following conditions using the same apparatus as in Example 1 except that the cross-sectional area per hole of each of the sieve trays in the second continuous multi-stage distillation column 201 was made to be approximately 1.8 $cm^2$.

A starting material 1 containing phenol and dimethyl carbonate in a weight ratio of phenol / dimethyl carbonate = 1.7 was introduced continuously in a liquid form at a flow rate of 86 ton / hr from the upper inlet 11 of the first continuous multi-stage distillation column 101. On the other hand, a starting material 2 containing dimethyl carbonate and phenol in a weight ratio of dimethyl carbonate / phenol = 3.5 was introduced continuously in a gaseous form at a flow rate of 90 ton / hr from the lower inlet 12 of the first continuous multi-stage distillation column 101. The molar ratio for the starting materials introduced into the first continuous multi-stage distillation column 101 was dimethyl carbonate / phenol = 1.44. The starting materials substantially did not contain halogens (outside the detection limit of the ion chromatography, i.e. not more than 1 ppb). $Pb(OPh)_2$ as a catalyst was introduced from the upper inlet 11 of the first continuous multi-stage distillation column 101 such that a concentration thereof in the reaction liquid would be approximately 150 ppm. Reactive distillation was carried out continuously in the first continuous multi-stage distillation column 101 under conditions of a temperature at the bottom of the column of 220°C, a pressure at the top of the column of $8\times10^5$ Pa and a reflux ratio of 0. A first column low boiling point reaction mixture containing methanol, dimethyl carbonate, phenol and so on was continuously withdrawn in a gaseous form from the top 13 of the first column, was passed through the heat exchanger 14, and was withdrawn at a flow rate of 82 ton / hr from the outlet 16. On the other hand, a first column high boiling point reaction mixture containing methyl phenyl carbonate, dimethyl carbonate, phenol, diphenyl carbonate, the catalyst and so on was continuously withdrawn in a liquid form from 21 via the bottom 17 of the first column.

A stable steady state was attained after 24 hours. The first column high boiling point reaction mixture was thus then fed continuously as is into the second continuous multi-stage distillation column 201 at a flow rate of 94 ton / hr from the starting material inlet 21 installed between the Mellapak and the sieve trays. The liquid fed into the second continuous multi-stage distillation column 201 contained 16.0 % by weight of methyl phenyl carbonate and 0.5 % by weight of diphenyl carbonate. Reactive distillation was carried out continuously in the second continuous multi-stage distillation column 201 under conditions of a temperature at the bottom of the column of 215°C, a pressure at the top of the column of $2.5\times10^4$ Pa, and a reflux ratio of 0.4. It was possible to attain stable steady state operation after 24 hours. A second column low boiling point reaction mixture was continuously withdrawn from the top 23 of the second column, and a second column high boiling point reaction mixture containing 35.5 % by weight of methyl phenyl carbonate and 59.5 % by weight of diphenyl carbonate was continuously withdrawn from the bottom 27 of the second column. The second column low boiling point reaction mixture was continuously fed into the first continuous multi-stage distillation column 101 from the inlet 11. At this time, the amounts of fresh dimethyl carbonate and phenol newly fed into the first continuous multi-stage distillation column 101 were adjusted so as to maintain the above compositions and amounts of the starting material 1 and the starting material 2, taking into consideration the composition and amount of the second column low boiling point reaction mixture. It was found that the produced amount of diphenyl carbonate per hour was 7.28 ton. The selectivity for the diphenyl carbonate based on the phenol reacted was 98%.

Prolonged continuous operation was carried out under these conditions. The produced amounts of diphenyl carbonate per hour after 500 hours, 1000 hours, and 2000 hours were 7.28 ton, 7.29 ton, and 7.29 ton respectively, and the selectivities based on the reacted phenol were 98%, 98%, and 98% respectively, and hence the operation was very stable. Moreover, the aromatic carbonates produced substantially did not contain halogens (not more than 1 ppb).

(3) Step (III) of obtaining high-purity diphenyl carbonate

This was carried out using the same process as in Example 1.

Industrial Applicability

It has been discovered that by implementing the process according to the present invention, a high-purity diphenyl carbonate required for producing a high-quality high-performance aromatic polycarbonate can be produced on an industrial scale of not less than 1 ton / hr, preferably not less than 2 ton / hr, more preferably not less than 3 ton / hr, from a cyclic carbonate and a phenol. Moreover, it has been discovered that the high-purity diphenyl carbonate can be produced stably for a prolonged period of time of, for example, not less than 2000 hours, preferably not less than 3000 hours, more preferably not less than 5000 hours, with a halogen content of not more than 0.1 ppm, preferably not more than 10 ppb, more preferably not more than 1 ppb and an impurity having a high boiling point than that of the diphenyl carbonate, a content of which being not more than 100 ppm, preferably not more than 50 ppm, more preferably not more than 10 ppm. The present invention thus provides a process that achieves excellent effects as an industrial process for the production of the high-purity diphenyl carbonate.

## Claims

**1.** A process for producing a high-purity diphenyl carbonate on an industrial scale in which the high-purity diphenyl carbonate is continuously produced from a cyclic carbonate and a phenol, the process comprising the steps of:

(I) continuously producing a dialkyl carbonate and a diol through a reactive distillation system of continuously feeding the cyclic carbonate and an aliphatic monohydric alcohol into a continuous multi-stage distillation column To in which a catalyst is present, carrying out reaction and distillation simultaneously in said column, continuously withdrawing a low boiling point reaction mixture containing the produced dialkyl carbonate from an upper portion of the column in a gaseous form, and continuously withdrawing a high boiling point reaction mixture containing the diol from a lower portion of the column in a liquid form;
(II) continuously producing a diphenyl carbonate by taking the dialkyl carbonate and a phenol as a starting material, continuously feeding the starting material into a first continuous multi-stage distillation column in which a homogeneous catalyst is present, carrying out reaction and distillation simultaneously in said first column, continuously withdrawing a first column low boiling point reaction mixture containing a produced alcohol from an upper portion of said first column in a gaseous form, continuously withdrawing a first column high boiling point reaction mixture containing a produced alkyl phenyl carbonate from a lower portion of said first column in a liquid form, continuously feeding the first column high boiling point reaction mixture into a second continuous multi-stage distillation column in which a catalyst is present, carrying out reaction and distillation simultaneously in said second column, continuously withdrawing a second column low boiling point reaction mixture containing a produced dialkyl carbonate from an upper portion of said second column in a gaseous form, continuously withdrawing a second column high boiling point reaction mixture containing the produced diphenyl carbonate from a lower portion of said second column in a liquid form, and continuously feeding the second column low boiling point reaction mixture containing the dialkyl carbonate into the first continuous multi-stage distillation column; and
(III) purifying said diphenyl carbonate by continuously introducing the second column high boiling point reaction mixture containing said diphenyl carbonate into a high boiling point material separating column A, and continuously carrying out separation by distillation into a column top component $A_T$ containing the diphenyl carbonate and a column bottom component $A_B$ containing the catalyst, and then continuously introducing the column top component $A_T$ into a diphenyl carbonate purifying column B having a side cut outlet, and continuously carrying out separation by distillation into three components being a column top component $B_T$, a side cut component $B_S$ and a column bottom component $B_B$, so as to obtain the high-purity diphenyl carbonate as the side cut component;
wherein:

(a) said continuous multi-stage distillation column To comprises a structure having a cylindrical trunk portion having a length $L_0$ (cm) and an inside diameter $D_0$ (cm) and having an internal with a number of stages $n_0$ thereinside, and further having a gas outlet having an inside diameter $d_{01}$ (cm) at a top of the column or in an upper portion of the column near to the top, a liquid outlet having an inside diameter $d_{o2}$ (cm) at a bottom of the column or in a lower portion of the column near to the bottom, at least one first inlet provided in the upper portion and / or a middle portion of the column below the gas outlet, and at least one second inlet provided in the middle portion and / or the lower portion of the column above the liquid outlet, wherein $L_0$, $D_0$, $L_0$ / $D_0$, $n_0$, $D_0$ / $d_{01}$, and $D_0$ / $d_{02}$ respectively satisfy the following formulae (1) to (6);

$$2100 \leq L_0 \leq 8000 \quad (1),$$

$$180 \leq D_0 \leq 2000 \quad (2),$$

$$4 \leq L_0 / D_0 \leq 40 \quad (3),$$

$$10 \leq n_0 \leq 120 \quad (4),$$

$$3 \leq D_0 / d_{01} \leq 20 \quad (5),$$

and

$$5 \leq D_0 / d_{02} \leq 30 \quad (6);$$

(b) said first continuous multi-stage distillation column comprises a structure having a cylindrical trunk portion having a length $L_1$ (cm) and an inside diameter $D_1$ (cm), and having and internal with a number of stages $n_1$ thereinside, and further having a gas outlet having an inside diameter $d_{11}$ (cm) at a top of the column or in an upper portion of the column near to the top, a liquid outlet having an inside diameter $d_{12}$ (cm) at a bottom of the column or in a lower portion of the column near to the bottom, at least one third inlet provided in the upper portion and / or a middle portion of the column below the gas outlet, and at least one fourth inlet provided in the middle portion and / or the lower portion of the column above the liquid outlet, wherein $L_1$. $D_1$, $L_1$ / $D_1$, $n_1$, $D_1$ / $d_{11}$, and $D_1$ / $d_{12}$ respectively satisfy the following formulae (7) to (12);

$$1500 \leq L_1 \leq 8000 \quad (7),$$

$$100 \leq D_1 \leq 2000 \quad (8),$$

$$2 \leq L_1 / D_1 \leq 40 \quad (9),$$

$$20 \leq n_1 \leq 120 \quad (10),$$

$$5 \leq D_1 / d_{11} \leq 30 \quad (11),$$

and

$$3 \leq D_1 / d_{12} \leq 20 \qquad (12);$$

(c) said second continuous multi-stage distillation column comprises a structure having a cylindrical trunk portion having a length $L_2$ (cm) and an inside diameter $D_2$ (cm), and having an internal with a number of stages $n_2$ thereinside, and further having a gas outlet having an inside diameter $d_{21}$ (cm) at a top of the column or in an upper portion of the column near to the top, a liquid outlet having an inside diameter $d_{22}$ (cm) at a bottom of the column or in a lower portion of the column near to the bottom, at least one fifth inlet provided in the upper portion and / or a middle portion of the column below the gas outlet, and at least one sixth inlet provided in the middle portion and / or the lower portion of the column above the liquid outlet, wherein $L_2$, $D_2$, $L_2$ / $D_2$, $n_2$, $D_2$ / $d_{21}$, and $D_2$ / $d_{22}$ respectively satisfy the following formulae (13) to (18);

$$1500 \leq L_2 \leq 8000 \qquad (13),$$

$$100 \leq D_2 \leq 2000 \qquad (14),$$

$$2 \leq L_2 / D_2 \leq 40 \qquad (15),$$

$$10 \leq n_2 \leq 80 \qquad (16),$$

$$2 \leq D_2 / d_{21} \leq 15 \qquad (17),$$

and

$$5 \leq D_2 / d_{22} \leq 30 \qquad (18);$$

(d) said high boiling point material separating column A comprises a continuous multi-stage distillation column having a length $L_A$ (cm) and an inside diameter $D_A$ (cm), and having an internal with a number of stages $n_A$ thereinside, wherein $L_A$, $D_A$, and $n_A$ satisfy following the following formulae (19) to (21);

$$800 \leq L_A \leq 3000 \qquad (19),$$

$$100 \leq D_A \leq 1000 \qquad (20),$$

and

$$20 \leq n_A \leq 100 \qquad (21);$$

(e) said diphenyl carbonate purifying column B comprises a continuous multi-stage distillation column having

a length $L_B$ (cm) and an inside diameter $D_B$ (cm), having an internals thereinside, having an inlet B1 at a middle protion of the column, and a side cut outlet B2 between the inlet B1 and the column bottom, and having a number of stages $n_{B1}$ of the internal above the inlet B1, a number of stages $n_{B2}$ of the internal between the inlet B1 and the side cut outlet B2, a number of stages $n_{B3}$ of the internals below the side cut outlet B2, and a total number of stages $n_B$ (= $n_{B1}$ + $n_{B2}$ + $n_{B3}$), wherein $L_B$, $D_B$, $n_{B1}$, $n_{B2}$, $n_{B3}$, and $n_B$ satisfy following formulae (22) to (27);

$$1000 \leq L_B \leq 5000 \quad (22),$$

$$100 \leq D_B \leq 1000 \quad (23),$$

$$5 \leq n_{B1} \leq 20 \quad (24),$$

$$12 \leq n_{B2} \leq 40 \quad (25),$$

$$3 \leq n_{B3} \leq 15 \quad (26),$$

and

$$20 \leq n_B \leq 70 \quad (27).$$

2. The process according to Claim 1, wherein not less than 1 ton / hr of the high-purity diphenyl carbonate is produced.

3. The process according to Claim 1 or 2, wherein said $d_{01}$ and said $d_{o2}$ for said continuous multi-stage distillation column To used in step (I) satisfy the formula (28);

$$1 \leq d_{01} / d_{02} \leq 5 \quad (28).$$

4. The process according to any one of Claims 1 to 3, wherein $L_0$, $D_0$, $L_0$ / $D_0$, $n_0$, $D_0$ / $d_{01}$, and $D_0$ / $d_{02}$ for said continuous multi-stage distillation column To satisfy respectively 2300 ≤ $L_0$ ≤ 6000, 200 ≤ $D_0$ ≤ 1000, 5 ≤ $L_0$ / $D_0$ ≤ 30, 30 ≤ $n_0$ ≤ 100, 4 ≤ $D_0$ / $d_{01}$ ≤ 15, and 7 ≤ $D_o$ / $d_{o2}$ ≤ 25.

5. The process according to any one of Claims 1 to 4, wherein $L_0$, $D_0$, $L_0$ / $D_0$, $n_0$, $D_0$ / $d_{01}$, and $D_0$ / $d_{02}$ for said continuous multi-stage distillation column To satisfy respectively 2500 ≤ $L_0$ ≤ 5000, 210 ≤ $D_0$ ≤ 800, 7 ≤ $L_0$ / $D_0$ ≤ 20, 40 ≤ $n_0$ ≤ 90, 5 ≤ $D_0$ / $d_0$ ≤ 13, and 9 ≤ $D_0$ / $d_{02}$ ≤ 20.

6. The process according to any one of Claims 1 to 5, wherein said continuous multi-stage distillation column $T_0$ is a distillation column having a tray and / or a packing as said internal.

7. The process according to Claim 6, wherein said continuous multi-stage distillation column $T_0$ is a plate type distillation column having the tray as said internal.

8. The process according to Claim 6 or 7, wherein said tray in said continuous multi-stage distillation column $T_0$ is a sieve tray having a sieve portion and a downcomer portion.

**9.** The process according to Claim 8, wherein said sieve tray in said continuous multi-stage distillation column To has 100 to 1000 holes / $m^2$ in said sieve portion thereof.

**10.** The process according to Claim 8 or 9, wherein a cross-sectional area per hole of said sieve tray in said continuous multi-stage distillation column $T_0$ is in a range of from 0.5 to 5 $cm^2$.

**11.** The process according to any one of Claims 8 to 10, wherein an aperture ratio of said sieve tray in said continuous multi-stage distillation column $T_0$ is in a range of from 1.5 to 15%.

**12.** The process according to any one of Claims 1 to 11, wherein said $d_{11}$ and said $d_{12}$ for said first continuous multi-stage distillation column used in step (II) satisfy the following formula (29), and said $d_{21}$ and said $d_{22}$ for said second continuous multi-stage distillation column used in step (II) satisfy the following formula (30);

$$1 \leq d_{12} / d_{11} \leq 5 \qquad (29),$$

and

$$1 \leq d_{21} / d_{22} \leq 6 \qquad (30).$$

**13.** The process according to any one of Claims 1 to 12, wherein $L_1$, $D_1$, $L_1$ / $D_1$, $n_1$, $D_1$ $d_{11}$, and $D_1$ / $d_{12}$ for said first continuous multi-stage distillation column used in step (II) satisfy respectively $2000 \leq L_1 \leq 6000$, $150 \leq D_1 \leq 1000$, $3 \leq L_1 / D_1 \leq 30$, $30 \leq n_1 \leq 100$, $8 \leq D_1 / d_{11} \leq 25$, and $5 \leq D_1 / d_{12} \leq 18$, and $L_2$, $D_2$, $L_2$ / $D_2$, $n_2$, $D_2$ / $d_{21}$, and $D_2$ / $d_{22}$ for said second continuous multi-stage distillation column used in step (II) satisfy respectively $2000 \leq L_2 \leq 6000$, $150 \leq D_2 \leq 1000$, $3 \leq L_2 / D_2 \leq 30$, $15 \leq n_2 \leq 60$, $2.5 \leq D_2 / d_{21} \leq 12$, and $7 \leq D_2 / d_{22} \leq 25$.

**14.** The process according to any one of Claims 1 to 13, wherein $L_1$, $D_1$, $L_1$ / $D_1$, $n_1$, $D_1$ / $d_{11}$, and $D_1$ / $d_{12}$ for said first continuous multi-stage distillation column satisfy respectively $2500 \leq L_1 \leq 5000$, $200 \leq D_1 \leq 800$, $5 \leq L_1 / D_1 \leq 15$, $40 \leq n_1 \leq 90$, $10 \leq D_1 / d_{11} \leq 25$, and $7 \leq D_1 / d_{12} \leq 15$, and $L_2$, $D_2$, $L_2$ / $D_2$, $n_2$, $D_2$ / $d_{21}$, and $D_2$ / $d_{22}$ for said second continuous multi-stage distillation column satisfy respectively $2500 \leq L_2 \leq 5000$, $200 \leq D_2 \leq 800$, $5 \leq L_2 / D_2 \leq 15$, $20 \leq n_2 \leq 50$, $3 \leq D_2 / d_{21} \leq 10$, and $9 \leq D_2 / d_{22} \leq 20$.

**15.** The process according to any one of Claims 1 to 14, wherein each of said first continuous multi-stage distillation column and said second continuous multi-stage distillation column is a distillation column having a tray and / or a packing as said internal.

**16.** The process according to Claim 15, wherein said first continuous multi-stage distillation column is a plate type distillation column having the tray as said internal, and said second continuous multi-stage distillation column is a distillation column having both the packing and the tray as said internal.

**17.** The process according to Claim 15 or 16, wherein each of said trays in said first continuous multi-stage distillation column and said second continuous multi-stage distillation column is a sieve tray having a sieve portion and a downcomer portion.

**18.** The process according to Claim 17, wherein each of said sieve trays in the first continuous multi-stage distillation column and the second continuous multi-stage distillation column has 100 to 1000 holes / $m^2$ in said sieve portion.

**19.** The process according, to Claim 17 or 18, wherein the cross-sectional area per hole of each of said sieve trays in said first continuous multi-stage distillation column and said second continuous multi-stage distillation column is in a range of from 0.5 to 5 $cm^2$.

**20.** The process according to Claim 15 or 16, wherein said second continuous multi-stage distillation column is a distillation column having, as said internal, the packing in the upper portion of the column, and the tray in the lower portion of the column.

**21.** The process according to any one of Claims 15 to 20, wherein said packing of said internal in said second continuous multi-stage distillation column is one or a plurality of sets of structured packings.

**22.** The process according to Claim 21, wherein said structured packing in said second continuous multi-stage distillation column is of at least one type selected from the group consisting of Mellapak, Gempak, Techno-pack, Flexipac, a Sulzer packing, a Goodroll packing, and Glitschgrid.

**23.** The process according to any one of Claims 1 to 22, wherein each of said high boiling point material separating column A and said diphenyl carbonate purifying column B is a distillation column having a tray and / or a packing as said internal.

**24.** The process according to Claim 23, wherein each of said internals of said high boiling point material separating column A and said diphenyl carbonate purifying column B is the packing.

**25.** The process according to Claim 24, wherein said packing is a structured packing of at least one type selected fromt the group consisting of Mellapak, Gempak, Techno-pack, Flexipac, a Sulzer packing, a Goodroll packing, and Glitschgrid.

**26.** A high-purity diphenyl carbonate produced by the process according to any one of Claims 1 to 25 in an amount of not less than 1 ton / hr.

**27.** The high-purity diphenyl carbonate according to Claim 26, which has a halogen content of not more than 0.1 ppm.

**28.** The high-purity diphenyl carbonate according to Claim 26, which has a halogen content of not more than 1 ppb.

**29.** The high-purity diphenyl carbonate according to any one of Claims 26 to 28, which has a content of by-products having a higher boiling point than that of the diphenyl carbonate of not more than 100 ppm.

**30.** The high-purity diphenyl carbonate according to Claim 29, which has a halogen content of not more than 10 ppb, and a content of each of phenyl salicylate, xanthone, phenyl methoxybenzoate, and 1-phenoxycarbonyl-2-phenoxycarboxy-phenylene, which are the by-products having a higher boiling point than that of the diphenyl carbonate, of not more than 30 ppm.

**31.** The high-purity diphenyl carbonate according to Claim 30, which has a content of the by-products having a higher boiling point than that of the diphenyl carbonate of not more than 50 ppm.

**32.** The high-purity diphenyl carbonate according to Claim 31, which has a halogen content of not more than 1 ppb, and a content of the by-products having a higher boiling point than that of the diphenyl carbonate of not more than 10 ppm.

# FIG.1

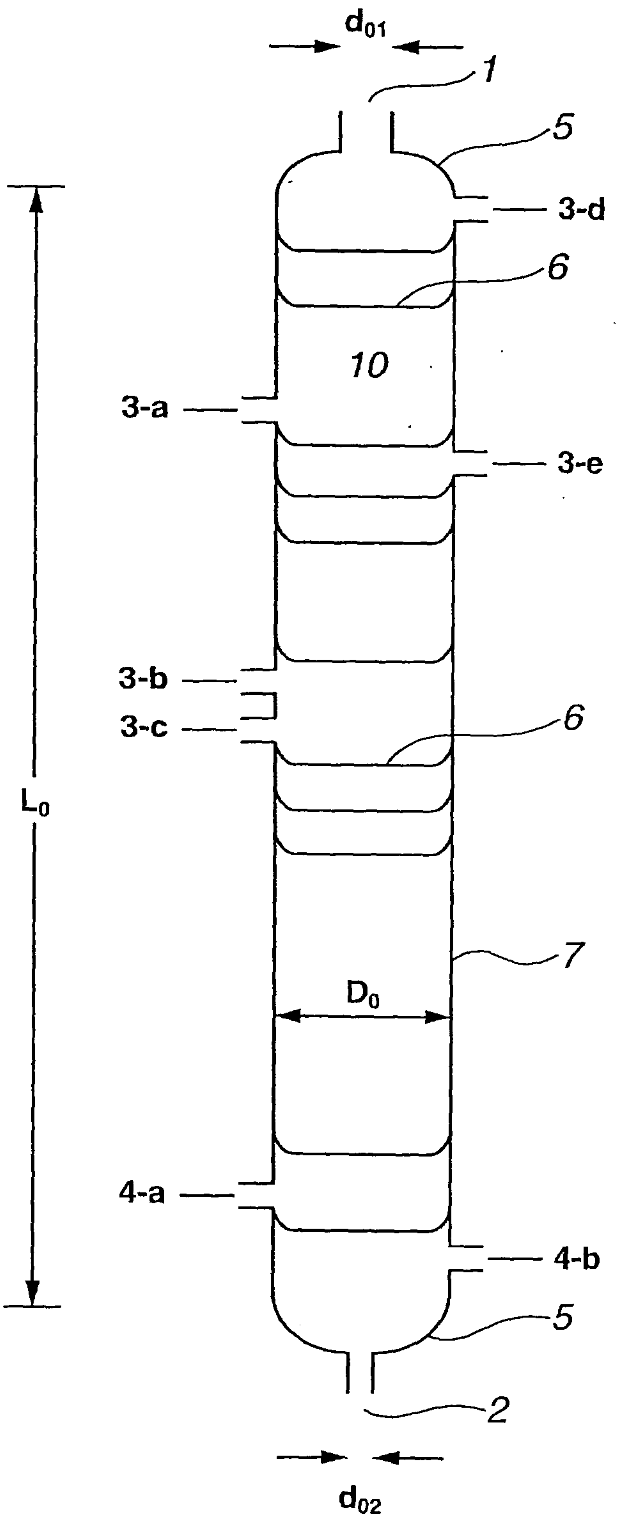

# FIG.2

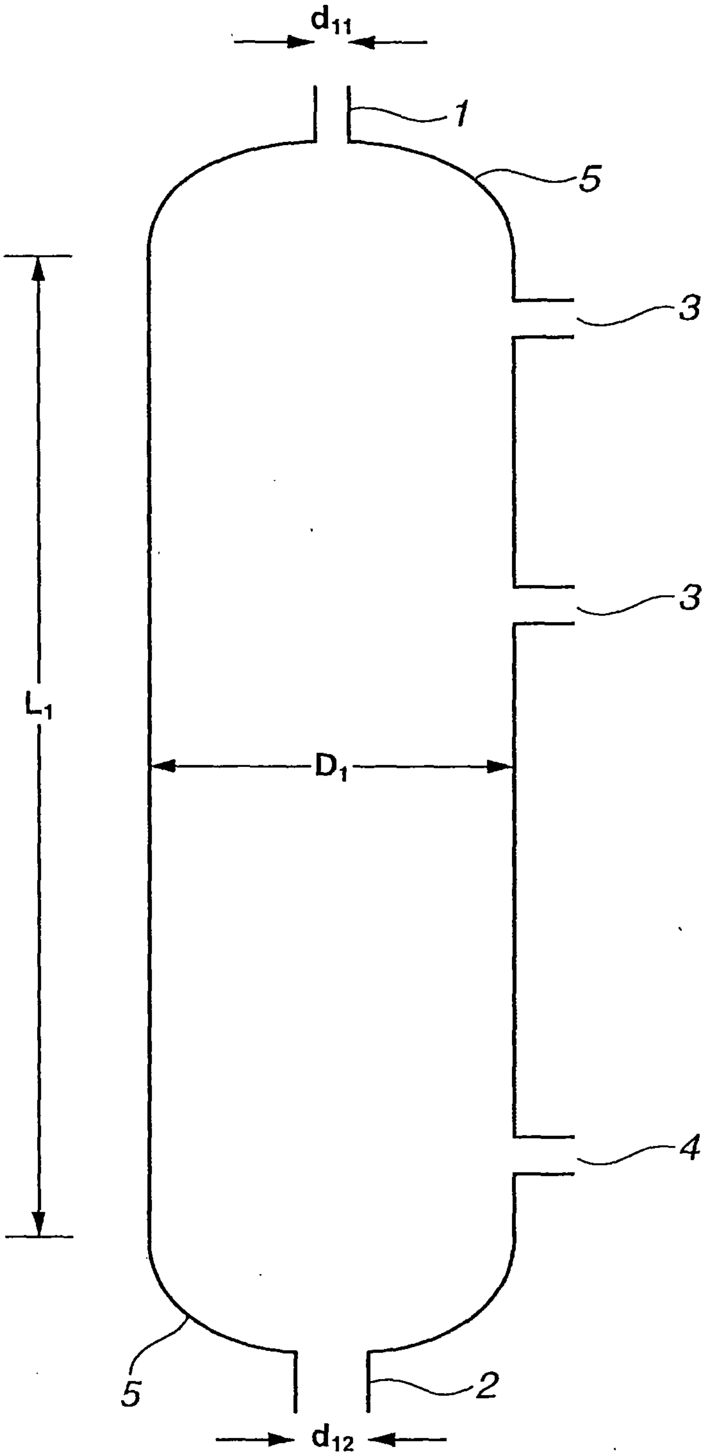

# FIG.3

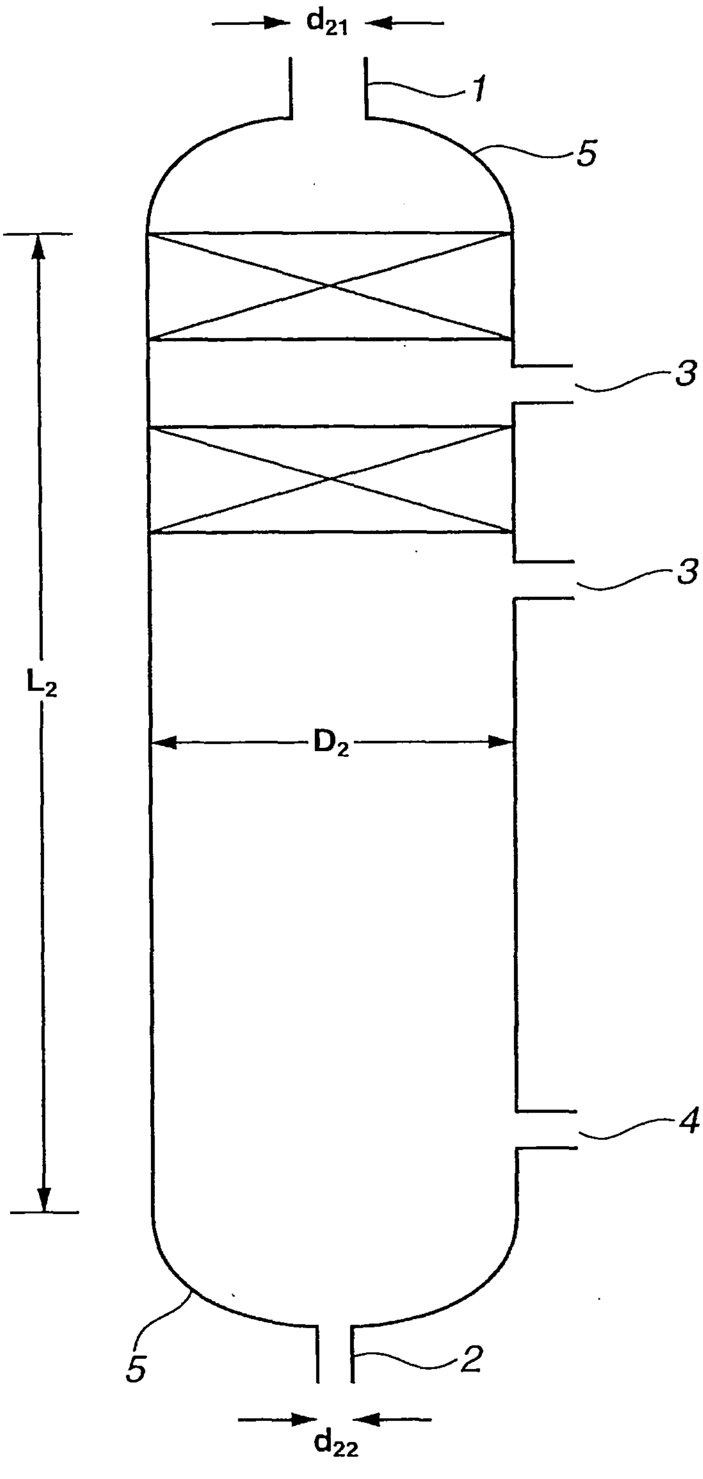

FIG.4

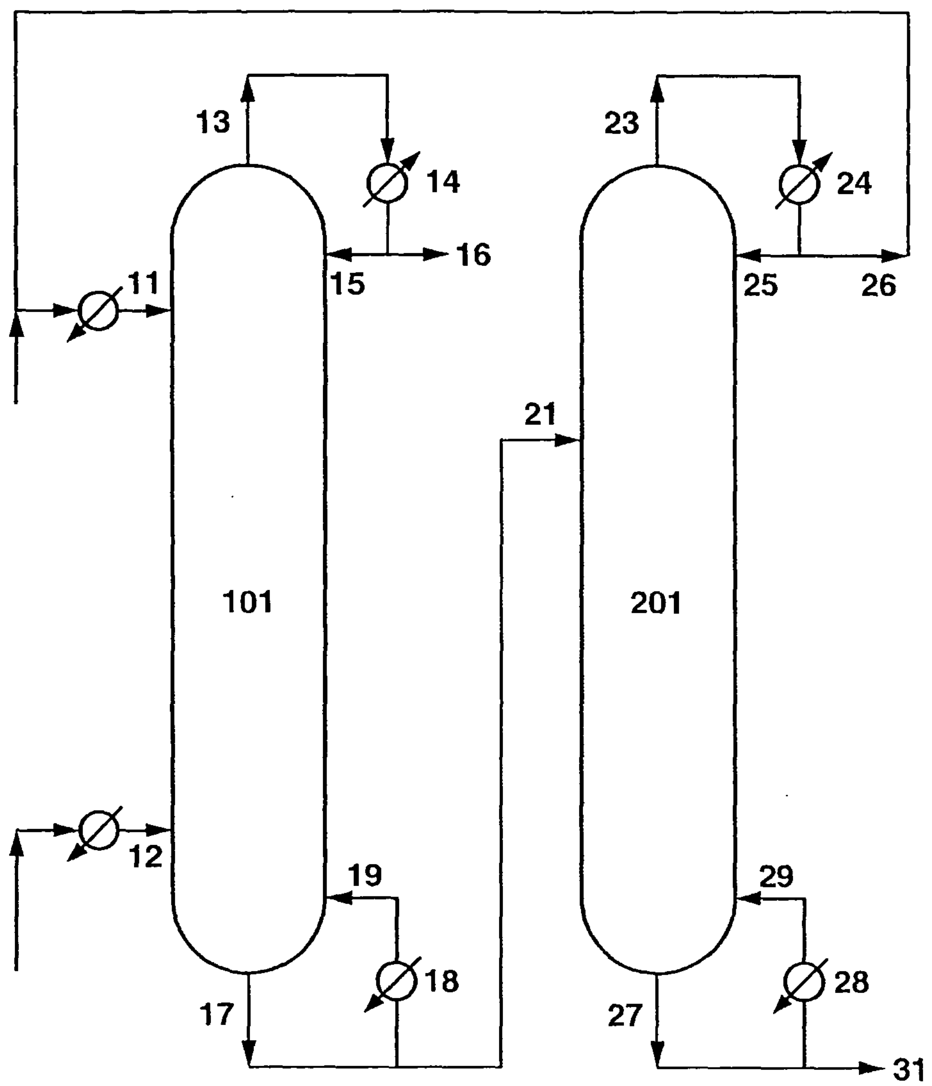

# FIG.5

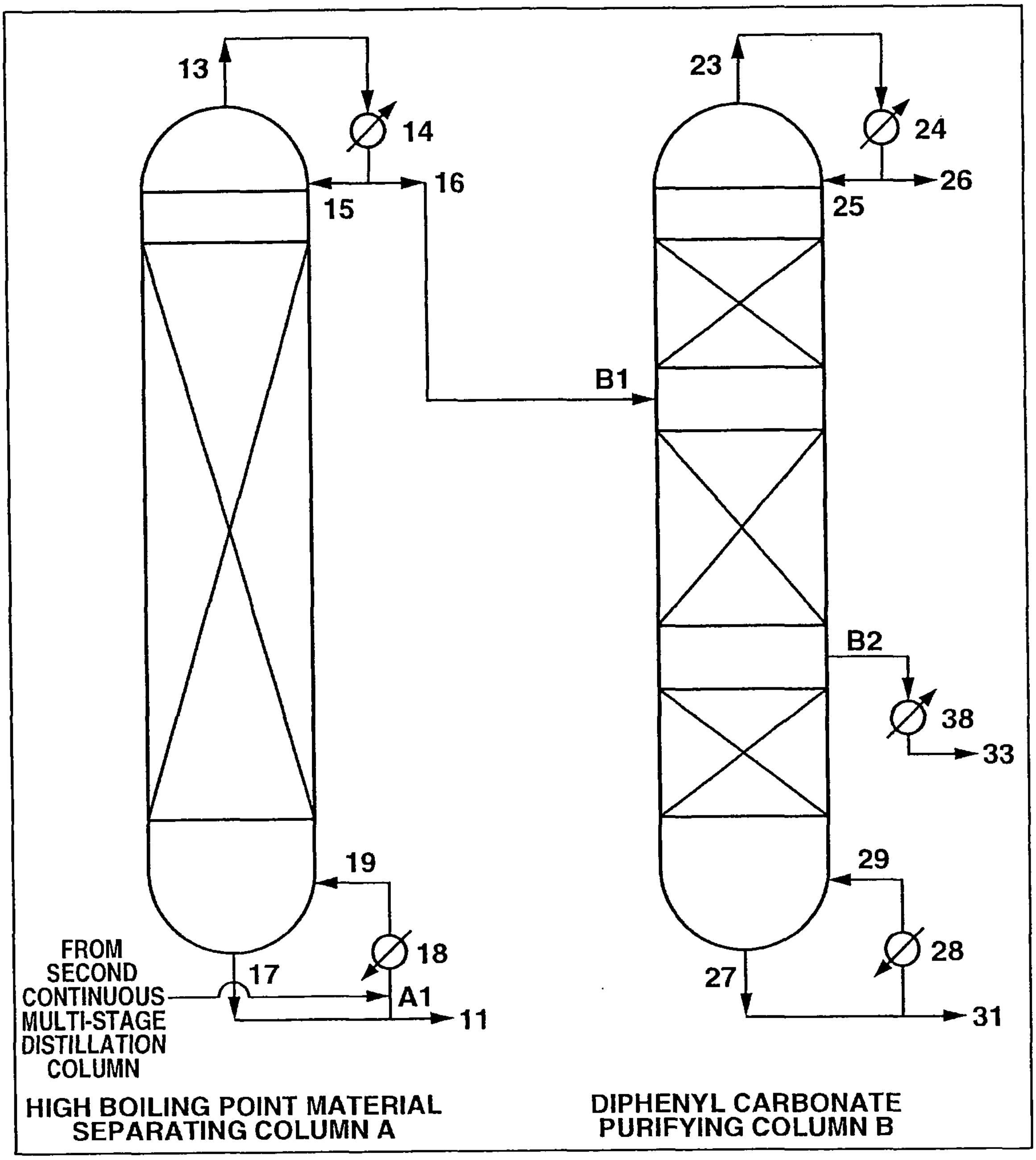
13
14
16
15
23
24
26
25
B1
B2
38
33
19
29
FROM SECOND CONTINUOUS MULTI-STAGE DISTILLATION COLUMN
18
17
A1
11
28
27
31
HIGH BOILING POINT MATERIAL SEPARATING COLUMN A
DIPHENYL CARBONATE PURIFYING COLUMN B

**INTERNATIONAL SEARCH REPORT**

International application No.
PCT/JP2006/324717

A. CLASSIFICATION OF SUBJECT MATTER
*C07C68/06*(2006.01)i, *C07C68/08*(2006.01)i, *C07C69/96*(2006.01)i, *C08G64/04* (2006.01)i

According to International Patent Classification (IPC) or to both national classification and IPC

B. FIELDS SEARCHED

Minimum documentation searched (classification system followed by classification symbols)
C07C68/06, C07C68/08, C07C69/96, C08G64/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| P,A | JP 2006-199643 A (Asahi Kasei Chemicals Corp.), 03 August, 2006 (03.08.06), Claims; Par. Nos. [0072] to [0077] (Family: none) | 1-32 |
| P,A | JP 2006-206497 A (Asahi Kasei Chemicals Corp.), 10 August, 2006 (10.08.06), Claims; Par. Nos. [0056] to [0060] (Family: none) | 1-32 |
| P,A | JP 2006-182683 A (Asahi Kasei Chemicals Corp.), 13 July, 2006 (13.07.06), Claims; Par. Nos. [0066] to [0068] (Family: none) | 1-32 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 January, 2007 (09.01.07) | 16 January, 2007 (16.01.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/324717

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 2005/123638 A1 (Asahi Kasei Chemicals Corp.), 29 December, 2005 (29.12.05), Claims (Family: none) | 1-32 |
| A | JP 2003-342209 A (Mitsubishi Chemical Corp.), 03 December, 2003 (03.12.03), Claims; Par. Nos. [0041] to [0053] (Family: none) | 1-32 |
| A | JP 2003-119168 A (Mitsubishi Chemical Corp.), 23 April, 2003 (23.04.03), Claims; Par. Nos. [0014] to [0020] & WO 2003/006418 A1 | 1-32 |
| A | JP 2003-300936 A (Mitsui Chemicals, Inc.), 21 October, 2003 (21.10.03), Claims; Par. Nos. [0060] to [0065] (Family: none) | 1-32 |
| X<br>A | JP 10-245366 A (Mitsubishi Chemical Corp.), 14 September, 1998 (14.09.98), Claims (Family: none) | 26-32<br>1-25 |
| X<br>A | JP 11-228504 A (Mitsubishi Chemical Corp.), 24 August, 1999 (24.08.99), Claims (Family: none) | 26-32<br>1-25 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 54048732 A
- DE 736063
- US 4252737 A
- JP 58185536 A
- US 410464 A
- JP 56123948 A
- US 4182726 A
- JP 3291257 A
- JP 4009358 A
- JP 4211038 A
- WO 9109832 A
- EP 0461274 A
- US 5210268 A
- JP 4235951 A
- JP 6157424 A
- EP 0582931 A
- US 5334742 A
- JP 6184058 A
- EP 0582930 A
- US 5344954 A
- JP 9040616 A
- JP 9059225 A
- JP 9176094 A
- WO 00118720 A
- US 6093842 A
- JP 2001064235 A
- WO 9711049 A
- EP 0855384 A
- US 5872275 A
- JP 11092429 A
- EP 1016648 A
- US 6262210 B
- JP 9255772 A
- EP 0892001 A
- US 5747609 A
- WO 03016257 A
- JP 4198141 A
- JP 9194435 A
- WO 9964382 A
- EP 1086940 A
- US 6346638 B
- WO 0051954 A
- EP 1174406 A
- US 6479689 B
- JP 5213830 A
- EP 0530615 A
- US 5231212 A
- JP 6009507 A
- EP 0569812 A
- US 5359118 A
- JP 2003119168 A
- WO 03006418 A
- JP 2003300936 A
- JP 2003342209 A
- JP 6009506 A
- EP 0560159 A
- US 5282965 A
- JP 4100824 A
- JP 9169704 A
- JP 9110805 A
- JP 2001064234 A
- WO 9218458 A
- US 5426207 A
- JP 11049727 A
- JP 9194437 A
- EP 0784048 A